# EUROPEAN PATENT SPECIFICATION

(11) **EP 2 123 681 B1**
(45) Date of publication and mention of the grant of the patent: **16.01.2013**
(21) Application number: 08721386.4
(22) Date of filing: 05.03.2008
(51) Int. Cl.: C08G 83/00, A61Q 19/00, C08B 37/00

(54) **POLYROTAXANES AND MATERIAL HAVING POLYROTAXANE, CROSSLINKED POLYROTAXANES AND MATERIAL HAVING THE CROSSLINKED POLYROTAXANE, AND PROCESSES FOR PRODUCING THESE**
POLYROTAXANE UND MATERIAL MIT POLYROTAXANEN, VERNETZTE POLYROTAXANE UND MATERIAL MIT DEN VERNETZTEN POLYROTAXANEN SOWIE VERFAHREN ZU IHRER HERSTELLUNG
POLYROTAXANES ET MATÉRIAU COMPRENANT DU POLYROTAXANE, POLYROTAXANES RÉTICULÉS ET MATÉRIAU COMPRENANT LE POLYROTAXANE RÉTICULÉ ET PROCÉDÉS DE PRODUCTION CORRESPONDANTS

(30) Priority: 06.03.2007 JP 2007056019
(43) Date of publication of application: 25.11.2009
(73) Proprietor: Advanced Softmaterials Inc., Tokyo 113-0033 (JP)
(72) Inventor: RUSLIM, Christian, Tokyo 113-0033 (JP); SUZUKI, Mariko, Tokyo 113-0033 (JP); ZHAO, Changming, Tokyo 113-0033 (JP)
(74) Representative: Reitstötter - Kinzebach
(86) International application number: PCT/JP2008/053966
(87) International publication number: WO 2008/108411

(56) References cited:
- EP-A1- 1 707 587
- EP-A1- 1 710 269
- WO-A1-2005/080469
- WO-A1-2005/080470
- WO-A1-2006/088200
- ARAKI J ET AL: "Polyrotaxane derivatives. I. Preparation of modified polyrotaxanes with nonionic functional groups and their solubility in organic solvents", JOURNAL OF POLYMER SCIENCE. PART A, POLYMER CHEMISTRY, JOHN WILEY & SONS, INC, US, vol. 44, no. 21, 1 November 2006 (2006-11-01), pages 6312-6323, XP008101850, ISSN: 0887-624X, DOI: DOI:10.1002/POLA.21717
- ARAKI J. AND ITO K.: 'Polyrotaxane derivatives. I. Preparation of modified polyrotaxanes with nonionic functional groups and their solubility in organic solvents' JOURNAL OF POLYMER SCIENCE, PART A: POLYMER CHEMISTRY vol. 44, no. 21, 2006, pages 6312 - 6323, XP008101850

## Description

### Technical Field

The present invention relates to polyrotaxane and a material comprising the polyrotaxane, crosslinked polyrotaxane and a material comprising the crosslinked polyrotaxane, and a method for producing thereof.

In particular, the present invention relates to 1) a polyrotaxane having enhanced solubility and being soluble in various solvents, 2) a polyrotaxane having an ability to respond reversibly to an external stimulus, 3) a chemically crosslinked polyrotaxane having a high Young's modulus and a high extension ratio and having a high transmittance, and/or 4) a chemically crosslinked polyrotaxane having an ability to respond reversibly to an external stimulus; and/or a material comprising any of them; and/or a process for producing them.

### Background Art

Conventionally, Patent Document 1 discloses a polyrotaxane which is comprised of a pseudopolyrotaxane, which comprises a linear molecule and cyclic molecules in which the linear molecule is included in cavities of cyclic molecules in a skewered manner, and capping groups, each of which locates at each end of the pseudopolyrotaxane in order to prevent the dissociation of the cyclic molecules, as well as a crosslinked polyrotaxane in which the polyrotaxanes are crosslinked.

Patent Document 1 discloses, for example, a polyrotaxane which is comprised of α-cyclodextrin as the cyclic molecule, polyethylene glycol as the linear molecule. However, the polyrotaxane is soluble only in strong aqueous alkalis or dimethylsulfoxide (DMSO).

Patent document 2 discloses a polyrotaxane obtained by partially substituting hydroxyl groups of cyclodextrin with hydroxypropyl groups or acetyl groups to improve the solubility of the polyrotaxane.

Further, N,N'-diisopropyl acrylamide based polymers and polypropylene based polymers have been known as typical hydrogel that exhibits the temperature characteristics opposite to a thermoplastic agent, that is, a sol state at a lower temperature and a gel state at a higher temperature. Patent document 3 discloses that the temperature-responsive characteristics same as those mentioned above are obtained by oxyalkylating or oxyalkyl-carbamoylating hydroxyl groups of cyclodextrin contained in a polyrotaxane. More, Patent document 3 discloses that, owing to the temperature characteristics, applications in medical and biotechnological fields such as cell cultivation media, wound coatings, bioadhesives and the like are expected.
Patent Document 1: Japanese Patent No. 3475252.
Patent Document 2: WO2005/080469.
Patent Document 3: WO2005/080470A1.
The non-patent document "Polyrotaxane derivatives, I. Preparation of modified polyrotaxanes with nonionic functional groups and their solubility in organic solvents" by Araki describes the preparation of modified polyrotaxanes comprising alfa-cyclodextrins which have been methylated, hydroxypropylated, acetylated in order to increase their solubility in organic solvents.

### Disclosure of the Invention

### Problem to be solved by the Invention

However, an improvement in the solubility in patent document 2 is insufficient and the solvents able to solve the polyrotaxane are limited. Polyrotaxane soluble in general industrial solvents such as acetone, dichloromethane, alcohols and the like are in demand, and thus, applications of the polyrotaxane are expected in various fields.

Further, a crosslinked body of hydroxypropylated polyrotaxane or acetylated polyrotaxane disclosed in patent document 2 has the transparency in a visible region. However, viscoelasticity and/or an extension ratio thereof, in particular, an extension ratio is inferior to that of unsubstituted crosslinked body of polyrotaxane. As a product, a crosslinked polyrotaxane simultaneously satisfying three requirements: high viscoelasticity, high extension ratio and transparency in the visible region is in demand. However, the polyrotaxane of patent document 2 is not able to sufficiently satisfy the requirements.

Further, the material of Patent document 3, obtained via oxyalkylation or oxyalkyl-carbamoylation, is necessary to be raised in the oxyalkylation rate or oxyalkyl-carbamoylation rate to exhibit the temperature characteristics. There are problems in production to realize such high substitution rates. While there is a demand of controlling the sol-gel transition point, the material of Patent document 3 is not able to control the transition point or is able only to attain a limited range of the transition point.

More, a chemically crosslinked polyrotaxane having high Young' s modulus, high extension ratio and/or high transparency is desired to develop as a material having an ability to respond to an external stimulus.

An object of the present invention is to solve the problem described above.

First object of the present invention is to provide a polyrotaxane having enhanced solubility and being soluble in various solvents, and a material comprising the polyrotaxane, as well as producing methods thereof.

Second object of the present invention is to provide a polyrotaxane having an ability to respond reversibly to an external stimulus, and a material comprising the polyrotaxane, as well as a producing methods thereof.

Third object of the present invention is to provide a chemically crosslinked polyrotaxane having a high Young's modulus and a high extension ratio and having a high transmittance, and a material comprising the chemically crosslinked polyrotaxane, as well as producing methods thereof.

Fourth object of the present invention is to provide a chemically crosslinked polyrotaxane having an ability to respond reversibly to an external stimulus, and a material comprising the chemically crosslinked polyrotaxane, as well as producing methods thereof.

### Means for solving the problems

The present inventors have found following inventions.

<1> A polyrotaxane comprising a pseudopolyrotaxane, which has a linear molecule and a cyclic molecule (s) in which the linear molecule is included in a cavity (cavities) of the cyclic molecule(s) in a skewered manner, and capping groups, each of which locates at each end of the pseudopolyrotaxane in order to prevent the dissociation of the cyclic molecule(s),
wherein the cyclic molecule(s) comprises a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom; and
R₆ represents a photoreactive group):

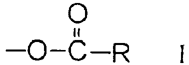

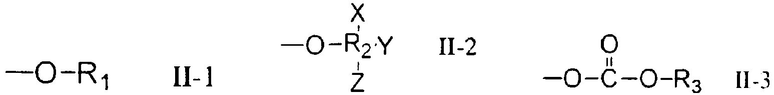

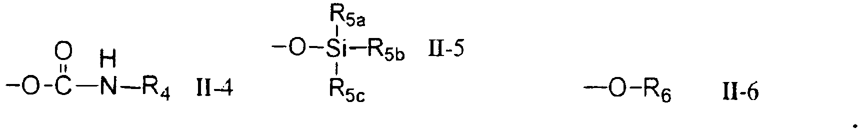

<2> In the above item <1>, the cyclic molecule is a cyclic molecule having a hydroxy group(s), and a part of the hydroxy groups may be substituted with the functional group represented by formula I and the at least one functional group selected from the group consisting of following formulae II-1 to II-6.

<3> In the above item <1> or <2>, the polyrotaxane may have an ability to respond reversibly to an external stimulus, which reversibly transforms the polyrotaxane from an uncrosslinked state to a crosslinked state, or from a crosslinked state to an uncrosslinked state, depending on the presence or the absence of the external stimulus. Furthermore, an uncrosslinked state means a state in which two or more molecules of the polyrotaxane are not crosslinked, and a crosslinked state means that at least two molecules of the polyrotaxane are crosslinked.

<4> In the above item <3>, the external stimulus may be heat.

<5> In the above item <3> or <4>, the external stimulus may be heat, and a temperature range in which the crosslinked polyrotaxane may transform from an uncrosslinked state to a crosslinked state, or from a crosslinked state to an uncrosslinked state, may be 5 to 90°C, preferably 10 to 80°C, more preferably 20 to 60°C.

<6> In any one of the above items <2> to <5>, the cyclic molecule having the hydroxy group(s) may be selected from the group consisting of α-cyclodextrin, β-cyclodextrin and γ-cyclodextrin.

<7> In any one of the above items <2> to <6>, the number of the functional group represented by formula I may be 0.05 to 0.9, preferably 0.2 to 0.7, more preferably 0.3 to 0.5, and the number of the functional group represented by formula II may be 0.05 to 0.9, preferably 0.2 to 0.7, more preferably 0.3 to 0.5, where the number of the hydroxy groups of the cyclic molecule is normalized to be 1.

<8> In any one of the above items <1> to <7>, the linear molecule may be selected from the group consisting of polyvinyl alcohol, polyvinylpyrrolidone, poly(meth)acrylic acid, cellulose-based resins (carboxymethylcellulose, hydroxyethylcellulose, hydroxypropylcellulose and the like), polyacrylamide, polyethylene oxide, polyethylene glycol, polypropylene glycol, polyvinyl acetal-based resins, polyvinyl methyl ether, polyamine, polyethyleneimine, casein, gelatin, starch and the like and/or copolymers thereof, polyolef in-based resins such as polyethylene, polypropylene, and copolymer resins with other olefinic monomers, polyester resins, polyvinyl chloride resins, polystyrene-based resins such as polystyrene, acrylonitrile-styrene copolymer resin and the like, acrylic resins such as polymethyl methacrylate, copolymer of (meth) acrylate, acrylonitrile-methyl acrylate copolymer resin and the like, polycarbonate resins, polyurethane resins, vinyl chloride-vinyl acetate copolymer resin, polyvinylbutyral resin and the like; and derivatives and modifications thereof, polyisobutylene, polytetrahydrofuran, polyaniline, acrylonitrile-butadiene-styrene copolymer (ABS resin), polyamides such as nylon and the like, polyimides, polydienes such as polyisoprene, polybutadiene and the like, polysiloxanes such as polydimethylsiloxane and the like, polysulfones, polyimines, polyacetic anhydrides, polyureas, polysulfides, polyphosphazenes, polyketones, polyphenylenes, polyhaloolefins, and derivatives thereof. For example, the linear molecule may be selected from the group consisting of polyethylene glycol, polyisoprene, polyisobutylene, polybutadiene, polypropylene glycol, polytetrahydrofuran, polydimethylsiloxane, polyethylene and polypropylene. And more specifically, the linear molecule may be selected from the group consisting of polyethylene glycol, polypropylene glycol, polytetrahydrofuran, polydimethylsiloxane, polyethylene and polypropylene, and preferably polyethylene glycol.

<9> In any one of the above items <1> to <8>, the linear molecule may have a molecular weight of 10, 000 or more, preferably 20,000 or more, more preferably 35,000 or more.

<10> In any one of the above items <1> to <9>, the capping group may be selected from the group consisting of dinitrophenyl groups; cyclodextrins; adamantane groups; trityl groups; fluoresceins; pyrenes; substituted benzenes (examples of the substituent may include, but are not limited to, alkyl, alkyloxy, hydroxy, halogen, cyano, sulfonyl, carboxyl, amino, phenyl and the like. The substituent may be single or plural.) ; polycyclic aromatics which may be substituted (examples of the substituent may include, but are not limited to, those described above. The substituent maybe single or plural.) ; and steroids. Preferably, the capping group may be selected from the group consisting of dinitrophenyl groups; cyclodextrins; adamantane groups; trityl groups; fluoresceins; and pyrenes, more preferably adamantane groups; or trityl groups.

<11> In any one of the above items <1> to <10>, the cyclic molecule may be derived from α-cyclodextrin, and the linear molecule may be polyethylene glycol.

<12> In any one of the above items <1> to <11>, the linear molecule may have the cyclic molecule included in a skewered manner at an amount of 0.001 to 0.6, preferably 0.01 to 0.5, more preferably 0.05 to 0.4 of a maximum inclusion amount, which is defined as an amount at which the cyclic molecules can be included at maximum when the linear molecule has the cyclic molecules included in a skeweredmanner, and the amount at maximum is normalized to be 1.

<13> A material comprising the polyrotaxane described in any one of the above items <1> to <12>.

<14> A method for producing a polyrotaxane comprising a pseudopolyrotaxane, which has a linear molecule and a cyclic molecule (s) in which the linear molecule is included in a cavity (cavities) of the cyclic molecule (s) in a skewered manner, and capping groups, each of which locates at each end of the pseudopolyrotaxane in order to prevent the dissociation of the cyclic molecule(s),
wherein a part of hydroxy groups of the cyclic molecule(s) is substituted with a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):
the method comprising the steps of:
   1) preparing the pseudopolyrotaxane;
   2) capping both ends of the resulting pseudopolyrotaxane with a capping group to prepare a polyrotaxane; and
   3) substituting a part of hydroxy groups of the cyclic molecule with the functional group;
wherein the step of substituting with the functional group is carried out
   A) before 1) the step of preparing the pseudopolyrotaxane,
      and/or
   B) after 2) the step of capping to prepare the polyrotaxane:

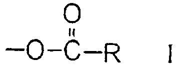

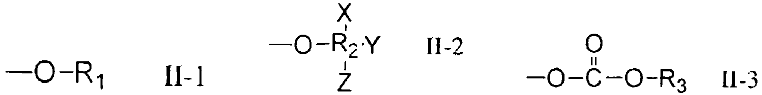

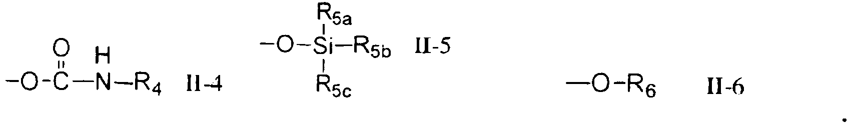

<15> In the above item <14>, the step of substituting with the functional group may be carried out after 2) the step of capping to prepare the polyrotaxane.

<16> In the step of substituting with the functional group according to the above item <14> or <15>, a step of introducing the functional group represented by formula I may be carried out
X) before a step of introducing any one of functional groups represented by formula II;
Y) after a step of introducing any one of functional groups represented by formula II; or
Z) simultaneously with the step of introducing any one of functional groups represented by formula II.

<17> In the step of substituting with the functional group according to any one of the above items <14> to <16>, a step of introducing the functional group represented by formula I may be carried out Y) after a step of introducing any one of functional groups represented by formula II.

<18> A crosslinked polyrotaxane comprising at least two molecules of polyrotaxane, wherein the at least two molecules of polyrotaxane are crosslinked via physical bond,
wherein the polyrotaxane comprises a pseudopolyrotaxane, which has a linear molecule and a cyclic molecule(s) in which the linear molecule is included in a cavity (cavities) of the cyclic molecule(s) in a skewered manner, and capping groups, each of which locates at each end of the pseudopolyrotaxane in order to prevent the dissociation of the cyclic molecule(s),
wherein the cyclic molecule(s) comprises a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):

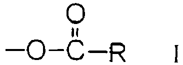

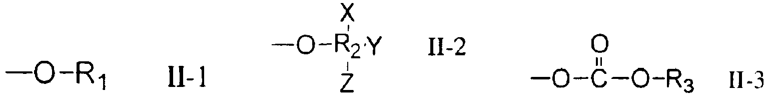

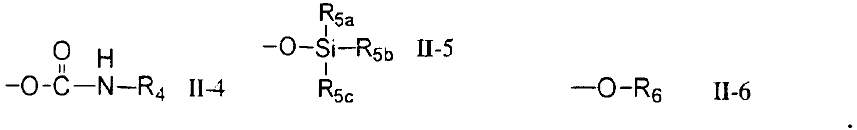

<19> In the above item <18>, the cyclic molecule may be a cyclic molecule having a hydroxy group(s), and a part of the hydroxy groups may be substituted with the functional group represented by formula I and the at least one functional group selected from the group consisting of the formulae II-1 to II-6.

<20> In the above item <18> or <19>, the polyrotaxane may have an ability to respond reversibly to an external stimulus, which reversibly transforms the polyrotaxane from an uncrosslinked state to a crosslinked state, or from a crosslinked state to an uncrosslinked state, depending on the presence or the absence of the external stimulus. Furthermore, an uncrosslinked state means a state in which two or more molecules of the polyrotaxane are not crosslinked, and a crosslinked state means that at least two molecules of the polyrotaxane are crosslinked.

<21> In the above item <20>, the external stimulus may be heat.

<22> In the above item <20> or <21>, the external stimulus may be heat, and a temperature range in which the crosslinked polyrotaxane may transform from an uncrosslinked state to a crosslinked state, or from a crosslinked state to an uncrosslinked state, may be 5 to 90°C, preferably 10 to 80°C, more preferably 20 to 60°C.

<23> In any one of the above items <19> to <22>, the cyclic molecule having the hydroxy group(s) may be selected from the group consisting of α-cyclodextrin, β-cyclodextrin and γ-cyclodextrin.

<24> In any one of the above items <19> to <23>, the number of the functional group represented by formula I may be 0.05 to 0.9, preferably 0.2 to 0.7, more preferably 0.3 to 0.5, and the number of the functional group represented by formula II may be 0.05 to 0.9, preferably 0.2 to 0.7, more preferably 0.3 to 0.5, where the number of the hydroxy groups of the cyclic molecule is normalized to be 1.

<25> In any one of the above items <18> to <24>, the linear molecule may be selected from the group consisting of polyvinyl alcohol, polyvinylpyrrolidone, poly(meth)acrylic acid, cellulose-based resins (carboxymethylcellulose, hydroxyethylcellulose, hydroxypropylcellulose and the like), polyacrylamide, polyethylene oxide, polyethylene glycol, polypropylene glycol, polyvinyl acetal-based resins, polyvinyl methyl ether, polyamine, polyethyleneimine, casein, gelatin, starch and the like and/or copolymers thereof, polyolefin-based resins such as polyethylene, polypropylene, and copolymer resins with other olefinic monomers, polyester resins, polyvinyl chloride resins, polystyrene-based resins such as polystyrene, acrylonitrile-styrene copolymer resin and the like, acrylic resins such as polymethyl methacrylate, copolymer of (meth) acrylate, acrylonitrile-methyl acrylate copolymer resin and the like, polycarbonate resins, polyurethane resins, vinyl chloride-vinyl acetate copolymer resin, polyvinylbutyral resin and the like; and derivatives and modifications thereof, polyisobutylene, polytetrahydrofuran, polyaniline, acrylonitrile-butadiene-styrene copolymer (ABS resin), polyamides such as nylon and the like, polyimides, polydienes such as polyisoprene, polybutadiene and the like, polysiloxanes such as polydimethylsiloxane and the like, polysulfones, polyimines, polyacetic anhydrides, polyureas, polysulfides, polyphosphazenes, polyketones, polyphenylenes, polyhaloolefins, and derivatives thereof. For example, the linear molecule may be selected from the group consisting of polyethylene glycol, polyisoprene, polyisobutylene, polybutadiene, polypropylene glycol, polytetrahydrofuran, polydimethylsiloxane, polyethylene and polypropylene. And more specifically, the linear molecule may be selected from the group consisting of polyethylene glycol, polypropylene glycol, polytetrahydrofuran, polydimethylsiloxane, polyethylene and polypropylene, and preferably polyethylene glycol.

<26> In any one of the above items <18> to <25>, the linear molecule may have a molecular weight of 10, 000 or more, preferably 20,000 or more, more preferably 35,000 or more.

<27> In any one of the above items <18> to <26>, the capping group may be selected from the group consisting of dinitrophenyl groups; cyclodextrins; adamantane groups; trityl groups; fluoresceins; pyrenes; substituted benzenes (examples of the substituent may include, but are not limited to, alkyl, alkyloxy, hydroxy, halogen, cyano, sulfonyl, carboxyl, amino, phenyl and the like. The substituent may be single or plural.); polycyclic aromatics which may be substituted (examples of the substituent may include, but are not limited to, those described above. The substituent may be single or plural.) ; and steroids. Preferably, the capping group may be selected from the group consisting of dinitrophenyl groups; cyclodextrins; adamantane groups; trityl groups; fluoresceins; and pyrenes, more preferably adamantane groups; or trityl groups.

<28> In any one of the above items <18> to <27>, the cyclic molecule may be derived from α-cyclodextrin, and the linear molecule may be polyethylene glycol.

<29> In any one of the above items <18> to <28>, the linear molecule may have the cyclic molecule included in a skewered manner at an amount of 0.001 to 0.6, preferably 0.01 to 0.5, more preferably 0.05 to 0.4 of a maximum inclusion amount, which is defined as an amount at which the cyclic molecules can be included at maximum when the linear molecule has the cyclic molecules included in a skewered manner, and the amount at maximum is normalized to be 1.

<30> A material comprising the crosslinked polyrotaxane described in any one of the above items <18> to <29>.

<31> Amethod for producing a crosslinked polyrotaxane, which comprises at least two molecules of polyrotaxane, wherein the at least two molecules of polyrotaxane are crosslinked via physical bond,
wherein the polyrotaxane comprises a pseudopolyrotaxane, which has a linear molecule and a cyclic molecule (s) in which the linear molecule is included in a cavity (cavities) of the cyclic molecule(s) in a skewered manner, and capping groups, each of which locates at each end of the pseudopolyrotaxane in order to prevent the dissociation of the cyclic molecule(s),
wherein a part of hydroxy groups of the cyclic molecule (s) is substituted with a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):
the method comprising the steps of:
1) preparing the pseudopolyrotaxane;
2) capping both ends of the resulting pseudopolyrotaxane with a capping group to prepare a polyrotaxane; and
3) substituting a part of hydroxy groups of the cyclic molecule with the functional group represented by formula I, and at least one functional group selected from the group consisting of formulae II-1 to II-6;
thereby to obtain the polyrotaxane;
4) dissolving at least two molecules of the resulting polyrotaxane in a solvent; and
5) imparting an external stimulus to the at least two molecules of polyrotaxane in the solvent to physically crosslink the at least two molecules of polyrotaxane via physical bond, wherein 3) the step of substituting is carried out
   A) before 1) the step of preparing the pseudopolyrotaxane,
      and/or
   B) after 2) the step of capping to prepare a polyrotaxane.

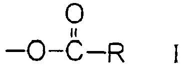

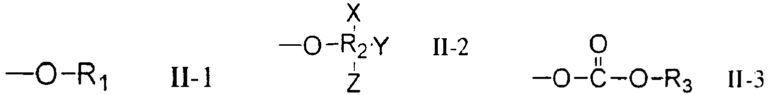

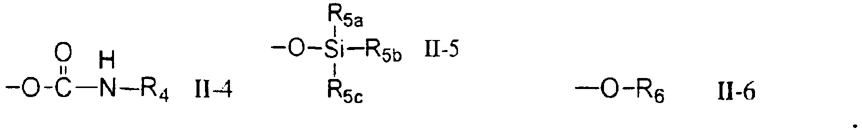

<32> In the above item <31>, the step of substituting with the functional group may be carried out after 2) the step of capping to prepare the polyrotaxane.

<33> In the step of substituting with the functional group according to the above item <31> or <32>, a step of introducing the functional group represented by formula I may be carried out
X) before a step of introducing any one of functional groups represented by formula II;
Y) after a step of introducing any one of functional groups represented by formula II; or
Z) simultaneously with the step of introducing any one of functional groups represented by formula II.

<34> In the step of substituting with the functional group according to any one of the above items <31> to <33>, a step of introducing the functional group represented by formula I may be carried out Y) after a step of introducing any one of functional groups represented by formula II.

<35> In any one of the above items <31> to <34>, the solvent may be a hydrophilic solvent.

<36> A crosslinked polyrotaxane comprising a first polymer and a first polyrotaxane, wherein all or a part of the first polymer and all or a part of the first polyrotaxane are crosslinked,
wherein the first polyrotaxane comprises a first pseudopolyrotaxane, which has a first linear molecule and a first cyclic molecule (s) in which the first linear molecule is included in a cavity (cavities) of the first cyclic molecule(s) in a skewered manner, and first capping groups, each of which locates at each end of the first pseudopolyrotaxane in order to prevent the dissociation of the first cyclic molecule(s),
wherein the first cyclic molecule(s) of the first polyrotaxane comprises a functional group represented by following formula I, and at least one functional group selected from the group consisting of following formulae II-1 to II-6,

wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group) :

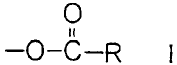

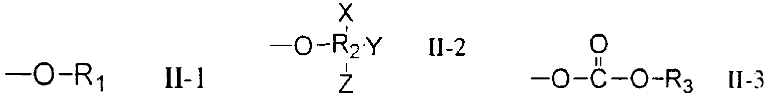

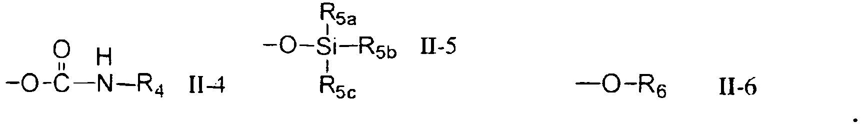

<37> In the above item <36>, the first polymer may be a secondpolyrotaxane, wherein the secondpolyrotaxane comprises a second pseudopolyrotaxane, which has a second linear molecule and a second cyclic molecule(s) in which the second linear molecule is included in a cavity (cavities) of the second cyclic molecule(s) in a skewered manner, and second capping groups, each of which locates at each end of the second pseudopolyrotaxane in order to prevent the dissociation of the second cyclic molecule(s),
wherein the second cyclic molecule(s) of the second polyrotaxane comprises a functional group represented by following formula I', and at least one functional group selected from the group consisting of following formulae II' -1 to II' -6,
wherein R' , R₁₁, R₁₃ and R₁₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₁₅ₐ, R_{15b} and R_{15c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₁₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₁₆ represents a photoreactive group):

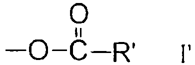

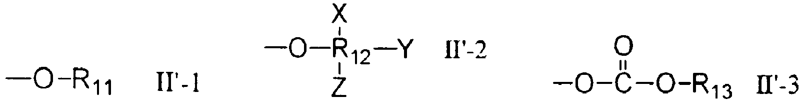

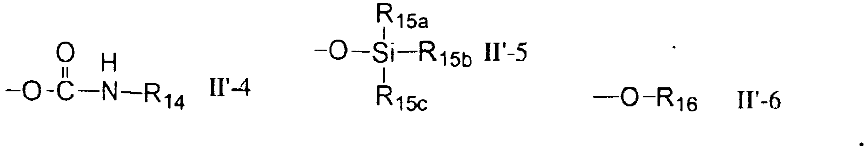

<38> In the above item <36> or <37>, the cyclic molecule may be a cyclic molecule having a hydroxy group (s), and a part of the hydroxy groups may be substituted with the functional group represented by formula I and the at least one functional group selected from the group consisting of the formulae II-1 to II-6.

<39> In any one of the above items <36> to <38>, the crosslinked polyrotaxane may have the transmittance of 90%/mm or more, preferably 95%/mm or more and more preferably 98%/mm or more in a wavelength from 400 to 800 nm.

<40> In any one of the above items <36> to <39>, the crosslinkedpolyrotaxane may have an extension ratio in the range of 100 to 1500%, preferably 200% or more, more preferably 300% or more, still more preferably 600% or more, most preferably 1000% or more.

<41> In any one of the above items <36> to <40>, the crosslinked polyrotaxane may have the transmittance of 90%/mm, or more, preferably 95%/mm or more, more preferably 98%/mm or more in a wavelength from 400 to 800 nm, and an extension ratio of 300% or more, preferably 600% or more, more preferably 1000% or more.

<42> In any one of the above items <36> to <41>, the crosslinked polyrotaxane may reversibly vary in an optical property and/or swelling-contraction characteristics, depending on the presence or the absence of the external stimulus.

<43> In the above item <42>, the external stimulus may be heat, and the crosslinked polyrotaxane may reversibly vary in the optical property and/or the swelling-contraction characteristics in a temperature range of from 5 to 90°C, preferably from 10 to 80°C, more preferably from 20 to 60°C.

<44> In the above item <43>, the optical property may be transparence of the crosslinked polyrotaxane. The term "transparence" used herein means that the transmittance of the crosslinked polyrotaxane is 90%/mm or more, preferably 95%/mm or more, more preferably 98%/mm or more in a wavelength from 400 to 800 nm.

<45> In the above item <43> or <44>, the swelling-contraction characteristics maybe a change of an amount of a contained solvent caused by absorption and/or release of the solvent by the crosslinked polyrotaxane.

<46> In any one of the above items <38> to <45>, the first and/or second cyclic molecule having the hydroxy group(s) may be selected from the group consisting of α-cyclodextrin, β-cyclodextrin and γ-cyclodextrin.

<47> In any one of the above items <38> to <46>, the number of the functional group represented by formula I may be 0.05 to 0. 9, preferably 0.2 to 0.7, more preferably 0.3 to 0.5, and the number of the functional group represented by formula II may be 0.05 to 0.9, preferably 0.2 to 0.7, more preferably 0.3 to 0.5, where the number of the hydroxy groups of the first and/or second cyclic molecule is normalized to be 1.

<48> In any one of the above items <36> to <47>, the first and/or second linear molecule may be selected from the group consisting of polyvinyl alcohol, polyvinylpyrrolidone, poly(meth)acrylic acid, cellulose-based resins (carboxymethylcellulose, hydroxyethylcellulose, hydroxypropylcellulose and the like), polyacrylamide, polyethylene oxide, polyethylene glycol, polypropylene glycol, polyvinyl acetal-based resins, polyvinyl methyl ether, polyamine, polyethyleneimine, casein, gelatin, starch and the like and/or copolymers thereof, polyolefin-based resins such as polyethylene, polypropylene, and copolymer resins with other olefinic monomers, polyester resins, polyvinyl chloride resins, polystyrene-based resins such as polystyrene, acrylonitrile-styrene copolymer resin and the like, acrylic resins such as polymethyl methacrylate, copolymer of (meth) acrylate, acrylonitrile-methyl acrylate copolymer resin and the like, polycarbonate resins, polyurethane resins, vinyl chloride-vinyl acetate copolymer resin, polyvinylbutyral resin and the like; and derivatives and modifications thereof, polyisobutylene, polytetrahydrofuran, polyaniline, acrylonitrile-butadiene-styrene copolymer (ABS resin), polyamides such as nylon and the like, polyimides, polydienes such as polyisoprene, polybutadiene and the like, polysiloxanes such as polydimethylsiloxane and the like, polysulfones, polyimines, polyacetic anhydrides, polyureas, polysulfides, polyphosphazenes, polyketones, polyphenylenes, polyhaloolefins, and derivatives thereof. For example, the linear molecule may be selected from the group consisting of polyethylene glycol, polyisoprene, polyisobutylene, polybutadiene, polypropylene glycol, polytetrahydrofuran, polydimethylsiloxane, polyethylene and polypropylene. And more specifically, the linear molecule may be selected from the group consisting of polyethylene glycol, polypropylene glycol, polytetrahydrofuran, polydimethylsiloxane, polyethylene and polypropylene, and preferably polyethylene glycol.

<49> In any one of the above items <36> to <48>, the first and/or second linear molecule may have a molecular weight of 10,000 or more, preferably 20,000 or more, more preferably 35,000 or more.

<50> In any one of the above items <36> to <49>, the first and/or second capping group may be selected from the group consisting of dinitrophenyl groups; cyclodextrins; adamantane groups; trityl groups; fluoresceins; pyrenes; substituted benzenes (examples of the substituent may include, but are not limited to, alkyl, alkyloxy, hydroxy, halogen, cyano, sulfonyl, carboxyl, amino, phenyl and the like. The substituent may be single or plural.); polycyclic aromatics which may be substituted (examples of the substituent may include, but are not limited to, those described above. The substituent may be single or plural.); and steroids. Preferably, the first and/or second capping group may be selected from the group consisting of dinitrophenyl groups; cyclodextrins; adamantane groups; trityl groups; fluoresceins; and pyrenes, more preferably adamantane groups; or trityl groups.

<51> In any one of the above items <36> to <50>, the first and/or second cyclic molecule may be derived from α-cyclodextrin, and the first and/or second linear molecule may be polyethylene glycol.

<52> In any one of the above items <36> to <51>, the first and/or second linear molecule may have the first and/or second cyclic molecule included in a skewered manner at an amount of 0.001 to 0.6, preferably 0.01 to 0.5, more preferably 0.05 to 0.4 of a maximum inclusion amount, which is defined as an amount at which the first and/or second cyclic molecules can be included at maximum when the first and/or second linear molecule has the first and/or second cyclic molecules included in a skewered manner, and the amount at maximum is normalized to be 1.

<53> A material comprising the crosslinked polyrotaxane described in any one of the above items <36> to <52>. In particular, a material comprising the crosslinked polyrotaxane described in any one of the above items <37> to <52>, in more particular <38> to <52>.

<54> A method for producing a crosslinked polyrotaxane comprising a first polymer and a first polyrotaxane, wherein all or a part of the first polymer and all or a part of the first polyrotaxane are crosslinked,
wherein the first polyrotaxane comprises a first pseudopolyrotaxane, which has a first linear molecule and a first cyclic molecule (s) in which the first linear molecule is included in a cavity (cavities) of the first cyclic molecule(s) in a skewered manner, and first capping groups, each of which locates at each end of the first pseudopolyrotaxane in order to prevent the dissociation of the first cyclic molecule(s),
wherein a part of hydroxy groups of the first cyclic molecule(s) is substituted with a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):
the method comprising the steps of:
   1) preparing the first pseudopolyrotaxane;
   2) capping both ends of the resulting first pseudopolyrotaxane with a first capping group to prepare a first polyrotaxane; and
   3) substituting a part of hydroxy groups of the first cyclic molecule with the functional group represented by formula I, and at least one functional group selected from the group consisting of formulae II-1 to II-6;
      thereby to obtain the first polyrotaxane; and
   4) chemically crosslinking the resulting first polyrotaxane and a first polymer;
      wherein 3) the step of substituting is carried out
      A) before 1) the step of preparing the first pseudopolyrotaxane, and/or
      B) after 2) the step of capping to prepare a first polyrotaxane; and
   4) the step of chemically crosslinking is carried out by
      G) a crosslinking reaction through addition of a crosslinking agent, or
      H) a photo-crosslinking reaction where a.photo-reactive group contained in the first polyrotaxane is irradiated with light:

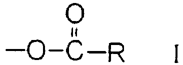

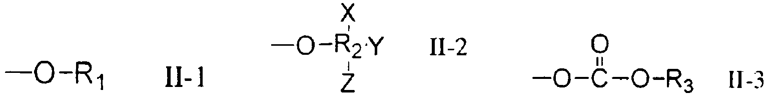

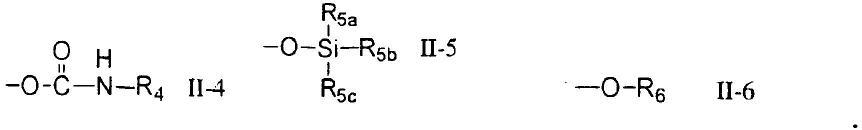

<55> In the above item <54>, the first polymer may be a secondpolyrotaxane, wherein the secondpolyrotaxane comprises a second pseudopolyrotaxane, which has a second linear molecule and a second cyclic molecule(s) in which the second linear molecule is included in a cavity (cavities) of the second cyclic molecule(s) in a skewered manner, and second capping groups, each of which locates at each end of the second pseudopolyrotaxane in order to prevent the dissociation of the second cyclic molecule(s),
wherein a part of hydroxy groups of the second cyclic molecule(s) is substituted with a functional group represented by following formula I', and at least one functional group selected from the group consisting of following formulae II'-1 to II' -6,
wherein R', R₁₁, R₁₃ and R₁₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₁₅ₐ, R_{15b} and R_{15c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₁₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₁₆ represents a photoreactive group); and
the second polyrotaxane may be obtained by
1') preparing the second pseudopolyrotaxane;
2') capping both ends of the resulting second pseudopolyrotaxane with a second capping group to prepare a second polyrotaxane; and
3') substituting a part of hydroxy groups of the second cyclic molecule with the functional group represented by formula I', and at least one functional group selected from the group consisting of formulae II'-1 to II'-6:

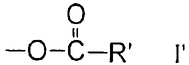

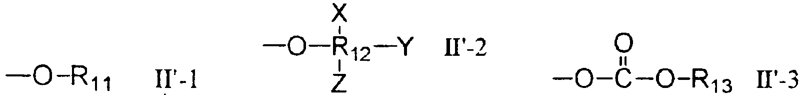

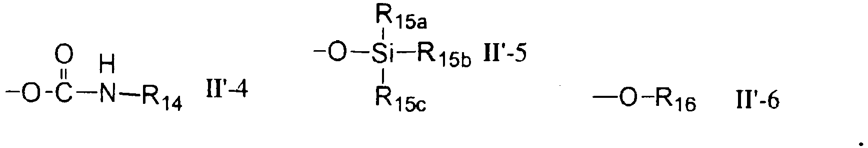

<56> In the above item <54> or <55>, 4) the step of chemically crosslinking may be carried out by G) the crosslinking reaction through addition of a crosslinking agent, and the crosslinking agent may be selected from the group consisting of cyanuric chloride, trimesoyl chloride, terephthaloyl chloride, epichlorohydrin, dibromobenzene, glutaraldehyde, aliphatic polyfunctional isocyanate, aromatic polyfunctional isocyanate, tolylene diisocyanate, hexamethylene diisocyanate, divinyl sulfone, 1,1'-carbonyldiimidazole, alkoxysilanes and derivatives thereof, and photo-crosslinking reaction initiators. Furthermore, the photo-crosslinking reaction initiator may be at least one selected from the group consisting of quinones, aromatic ketones, benzoin, benzoin ethers, biimidazole compounds and derivatives thereof, N-phenylglycines, combinations of thioxanthones and alkylaminobenzoic acid, combinations of biimidazole compounds and derivatives thereof and Michler's ketones, acridines, and oxime esters. Specifically, the reaction initiator may be quinones such as 2-ethylanthraquinone, octaethylanthraquinone, 1,2-benzanthraquinone, 2,3-benzanthraquinone, 2-phenylanthraquinone, 2,3-diphenylanthraquinone, 1-chloroanthraquinone, 1,4-naphthoquinone, 9,10-phenanthraquinone, 2-methyl-1,4-naphthoquinone, 2,3-dimethylanthraquinone, 3-chloro-2-methylanthraquinone and the like; aromatic ketones such as benzophenone, Michler's ketone[4,4'-bis(dimethylamino)benzophenone], 4,4'-bis(diethylamino)benzophenone and the like; benzoin ethers such as benzoin, benzoinethyl ether, benzoinphenylether, methylbenzoin, ethylbenzoin and the like; biimidazole compounds and derivatives thereof such as benzyldimethyl ketal, benzyldiethyl ketal, triarylimidazolyl dimers and the like; N-phenylglycines such as N-phenylglycine, N-methyl-N-phenylglycine, N-ethyl-N-phenylglycine and the like; combinations of thioxanthones and alkylamine benzoic acid such as a combination of ethylthioxanthone and ethyl dimethylaminobenzoate, a combination of 2-chlorothioxanthone and ethyl dimethylaminobenzoate, a combination of isopropylthioxanthone and ethyl dimethylbenzoate, and the like; combinations of biimidazole compounds such as triarylimidazolyl dimers and derivatives thereof and Michler' s ketone; acridines such as 9-phenylacridine and the like; and oxime esters such as 1-phenyl-1,2-propanedione-2-o-benzoineoxime, 1-phenyl-1,2-propanedione-2-(o-ethoxycarbonyl)oxime and the like. Preferably, the reaction initiator may be thioxanthones such as diethylthioxanthone, chlorothioxanthone and the like; dialkylaminobenzoate esters such as ethyl dimethylaminobenzoate and the like; benzophenone, 4,4'-bis(dimethylamino)benzophenone, 4,4'-bis(diethylamino)benzophenone; biimidazole compounds such as triarylimidazolyl dimers and derivatives thereof; 9-phenylacridine, N-phenylglycines; and combinations thereof. Furthermore, the biimidazole compounds such as triarylimidazolyl dimers and derivatives thereof may include, for example,2-(o-chlorophenyl)-4,5-diphenylimidazolyl dimers, 2,2',5-tris-(o-chlorophenyl)-4-(3,4-dimethoxyphenyl)-4',5'-diphenylimidazolyl dimers, 2,4-bis-(o-chlorophenyl)-5-(3,4-dimethoxyphenyl)-diphenylim idazolyl dimers, 2,4,5-tris-(o-chlorophenyl)-diphenylimidazolyl dimers, 2-(o-chlorophenyl)-bis-4,5-(3,4-dimethoxyphenyl)-imidazolyl dimers, 2,2'-bis-(2-fluorophenyl)-4,4',5,5'-tetrakis-(3-methoxyphen yl)-imidazolyl dimers, 2,2'-bis-(2,3-difluoromethylphenyl)-4,4',5,5'-tetrakis-(3-m ethoxyphenyl)-imidazolyl dimers, 2,2'-bis-(2,4-difluorophenyl)-4,4',5,5'-tetrakis-(3-methoxy phenyl)-imidazolyl dimers, 2,2'-bis-(2,5-difluorophenyl)-4,4',5,5'-tetrakis-(3-methoxy phenyl)-imidazolyl dimers, 2,2'-bis-(2,6-difluorophenyl)-4,4',5,5'-tetrakis-(3-methoxy phenyl)-imidazolyl dimers, 2,2'-bis-(2,3,4-trifluorophenyl)-4,4',5,5'-tetrakis-(3-meth oxyphenyl)-imidazolyl dimers, 2,2'-bis-(2,3,5-trifluorophenyl)-4,4',5,5'-tetrakis-(3-meth oxyphenyl)-imidazolyl dimers, 2,2'-bis-(2,3,6-trifluorophenyl)-4,4',5,5'-tetrakis-(3-meth oxyphenyl)-imidazolyl dimers, 2,2'-bis-(2,4,5-trifluorophenyl)-4,4',5,5'-tetrakis-(3-meth oxyphenyl)-imidazolyl dimers, 2,2'-bis-(2,4,6-trifluorophenyl)-4,4',5,5'-tetrakis-(3-meth oxyphenyl)-imidazolyl dimers, 2,2'-bis-(2,3,4,5-tetrafluorophenyl)-4,4',5,5'-tetrakis-(3-methoxyphenyl)-imidazolyl dimers, 2,2'-bis-(2,3,4,6-tetrafluorophenyl)-4,4',5,5'-tetrakis-(3-methoxyphenyl)-imidazolyl dimers, 2,2'-bis-(2,3,4,5,6-pentafluorophenyl)-4,4',5,5'-tetrakis-( '3-methoxyphenyl)-imidazolyl dimers, and the like.

<57> In any one of the above items <54> to <56>, the step of substituting with the functional group may be carried out after 2) and/or 2') the step of capping to prepare the first and/or second polyrotaxane.

<58> In the step of substituting with the functional group according to any one of the above items <54> to <57>, a step of introducing the functional group represented by formula I or I' may be carried out
X) before a step of introducing any one of functional groups represented by formula II or II';
Y) after a step of introducing any one of functional groups represented by formula II or II'; or
Z) simultaneously with the step of introducing any one of functional groups represented by formula II or II'.

<59> In the step of substituting with the functional group according to any one of the above items <54> to <58>, a step of introducing the functional group represented by formula I or I' may be carried out Y) after a step of introducing any one of functional groups represented by formula II or II'.

### Effects of the invention

The present invention can provide a material having a polyrotaxane having enhanced solubility and being soluble in various solvents, and a material comprising the polyrotaxane, as well as producing methods thereof.

Further, other than or in addition to the above-described effect, the present invention can provide a polyrotaxane having an ability to respond reversibly to an external stimulus, and a material comprising the polyrotaxane, as well as producing methods thereof.

More, other than or in addition to the above-described effects, the present invention can provide a chemically crosslinked polyrotaxane having a high Young's modulus and a high extension ratio and having a high transmittance, and a material comprising the chemically crosslinked polyrotaxane, as well as producing methods thereof.

Further, other than or in addition to the above-described effects, the present invention can provide a chemically crosslinkedpolyrotaxane having an ability to respond reversibly to an external stimulus, and a material comprising the chemically crosslinked polyrotaxane, as well as producing methods thereof.

### Brief Description of the Drawings

Fig. 1 shows a graph comparing sol-gel transition temperatures of Examples 12 to 16.
Fig. 2 shows a graph comparing sol-gel transition temperatures of Examples 17 to 20.
Fig. 3 shows a graph comparing sol-gel transition temperatures of Comparative Examples 6 to 11.
Fig. 4 is a diagram showing a stress-strain curve of the chemically crosslinked body of Example 21;
Fig. 5 is a diagram showing the transmittance of the chemically crosslinked body of Example 22;
Fig. 6 is a diagram showing the temperature dependence of the transmittance and water content of the chemically crosslinked body of Example 27;
Fig. 7 is a diagram showing the temperature dependence of the transmittance and water content of the chemically crosslinked body of Example 28;
Fig. 8 is a diagram showing the temperature dependence of the transmittance and water content of the chemically crosslinked body of Example 29;
Fig. 9 is a diagram showing the temperature dependence of the transmittance and water content of the chemically crosslinked body of Example 30; and
Fig. 10 is a diagram showing the temperature dependence of the transmittance and water content of the chemically crosslinked body of Comparative Example 14.

### Preferred Embodiments for Carrying Out the Present Invention

The present invention will be described in detail hereinafter.

The present invention provides 1) a polyrotaxane having enhanced solubility and being soluble in various solvents, 2) a polyrotaxane having an ability to respond reversibly to an external stimulus, 3) a chemically crosslinked polyrotaxane having a high Young's modulus and a high extension ratio and having a high transmittance, and/or 4) a chemically crosslinked polyrotaxane having an ability to respond reversibly to an external stimulus; and/or a material comprising them; and/or methods for producing them. Hereinafter, these will be described in order.

### <Polyrotaxane having enhanced solubility>

The present invention provides polyrotaxanes having enhanced solubility, or materials comprising them. The term "enhanced solubility" used herein means that the kinds of solvents capable of dissolving polyrotaxanes are increased or an amount of dissolution thereof in the same solvent is increased, or both of which are realized.

A polyrotaxane according to the present invention is capable of having enhanced solubility owing to possession of both of a functional group represented by formula I and a functional group represented by formula II (specifically formulae II-1 to II-6) . When the polyrotaxane having enhanced solubility is used, the choice of the solvent is expanded. Expansion of the choice of the solvent can lead to various advantages such that in a case where a mixed, crosslinked body with other polymer is produced as described later, a polymer, that had not been able to be selected conventionally, can be used, thereby to afford a mixed, crosslinked body having desired performance.

A polyrotaxane according the present invention is comprised of a pseudopolyrotaxane, which has a linear molecule and a cyclic molecule (s) in which the linear molecule is included in a cavity (cavities) of the cyclic molecule (s) in a skewered manner, and capping groups, each of which locates at each end of the pseudopolyrotaxane in order to prevent the dissociation of the cyclic molecule(s). The cyclic molecule comprises the functional group represented by the above formula I and at least one functional group selected from the above formulae II-1 to II-6.

### <<Cyclic Molecule and Functional Groups represented by Formulae I and II>>

The cyclic molecule of the polyrotaxane according to the present invention is not particularly restricted as long as a linear molecule is included in cavities of the cyclic molecules in a skewered manner.

The cyclic molecule has a functional group represented by formula I and a functional group represented by formula II (specifically, at least anyone of formulae II-1 to II-6), thereby to provide foregoing advantages. The functional group represented by formula II is at least one selected from formulae II-1 to II-6, that is, a plurality thereof, for instance, two or three thereof may be present.

The cyclic molecule may be a cyclic molecule having hydroxyl groups and may be selected from the group consisting of α-cyclodextrin, β-cyclodextrin and γ-cyclodextrin. When the cyclic molecule has hydroxyl groups as mentioned above, a part of the hydroxyl groups may be substituted with the functional groups represented by formulae I and II.

The number of the functional group represented by formula I may be 0.05 to 0.9, preferably 0.2 to 0.7, more preferably 0.3 to 0.5, and the number of the functional group represented by formula II may be 0.05 to 0.9, preferably 0.2 to 0.7, more preferably 0.3 to 0.5, where the number of the hydroxy groups of the cyclic molecule is normalized to be 1.

The number of functional groups of formula I and the number of functional groups of formula II can be measured as follows. The situation will be described with a case where α-cyclodextrin is used for the cyclic molecule and polyethylene glycol is used for the linear molecule as an example.

In a case where α-cyclodextrin is used as the cyclic molecule and polyethylene glycol is used as the linear molecule, the number of hydroxyl groups in one molecule of α-cyclodextrin being 18 is taken as a standard. In ¹H-NMR (DMSO-d6), a ratio of an integrated value from 4.0 to 6. 0 ppm (derived from hydroxyl groups of α-cyclodextrin and protons of C1 of sugar units of α-cyclodextrin) and an integrated value from 3.0 to 4.0 ppm (derived from protons of polyethylene glycol) is constant (such as 1: 2.2). In the case where the functional group of the formula II is a hydroxypropyl group, owing to additional protons of the hydroxypropyl group, a ratio of integrated values from 4.0 to 6.0 ppm and from 3.0 to 4.0 ppm varies. Further, protons of CH₃ of the hydroxypropyl appear newly at 1.0 ppm; accordingly, the number of substituted hydroxypropyl groups is calculated from a ratio of a value of 1 ppm and an integrated value from 4.0 to 6.0 ppm or a ratio of a value of 1 ppm and an integrated value from 3.0 to 4.0 ppm to the number of original hydroxyl groups (hydroxyl groups of α-cyclodextrin of an unsubstituted polyrotaxane). For instance, in the case where a ratio of a value of 1 ppm and an integrated value from 4.0 to 6.0 is 1.1: 1.0, substantially 48% of the hydroxyl groups are substituted.

More, in the case where the resulting hydroxypropylated polyrotaxane is substituted with an acetyl group represented by formula I, a signal of protons of CH₃ derived from the acetyl group appears newly at 2.0 ppm. Since the acetyl groups partially substitute as well the hydroxyl groups of the hydroxypropyl groups, a signal at 1.0 ppm derived from CH₃ of the hydroxypropyl group is splitted into 1. 0 and 1.1 ppm. An amount of hydroxypropyl groups is determined to the initial α-cyclodextrin (herein, 48%); accordingly, a relative amount of the acetyl groups and the hydroxypropyl groups is calculated from a ratio of a value at 2.0 ppm and an integrated value from 1.0 to 1.1 ppm. As the result, an amount of the acetyl groups to the initial α-cyclodextrin as well is calculated. In the case where a ratio of a value at 2.0 ppm and an integrated value from 1.0 to 1.1 ppm is, for instance, 0.97: 1, a substitution amount of the acetyl groups to the initial α-cyclodextrin is 97% × 48% = 47%.

In the functional groups represented by formula I or I', R or R' may represent a linear or branched alkyl group having 1 to 12 carbon atoms, preferably 1 to 9 carbon atoms and more preferably 1 to 5 carbon atoms. Examples of the functional groups represented by formula I or I' may include, but are not limited to, -O-CO-CH₃, -O-CO-CH₂CH₃, -O-CO-CH₂CH₂CH (CH₃) CH₃ and the like.

In the functional groups represented by formula II-1 or II'-1, R₁ or R₁₁ may represent a linear or branched alkyl group having 1 to 12 carbon atoms, preferably 1 to 8 carbon atoms and more preferably 1 to 4 carbon atoms. Examples of the functional group represented by formula II-1 or II' -1 may include, but are not limited to, -OCH₃, -OCH₂CH₃, -OCH₂CH(CH₃)CH₃ and the like.

In the functional groups represented by formula II-2 or II' -2, R₂ or R₁₂ may have 1 to 12 carbon atoms, preferably 1 to 8 carbon atoms, more preferably 1 to 4 carbon atoms. Examples of the functional group represented by formula II-2 or II'-2 may include, but are not limited to, -OCH₂CH(OH)CH₃, -OCH₂CH(OH)CH₂OH, -OCH₂CH(NH₂)CH₃ and the like.

In the functional groups represented by formula II-3 or II'-3, R₃ or R₁₃ may represent a linear or branched alkyl group having 1 to 12 carbon atoms, preferably 1 to 9 carbon atoms, more preferably 1 to 5 carbon atoms. Examples of the functional group represented by formula II-3 or II'-3 may include, but are not limited to, -O-CO-OCH₃, -O-CO-OCH₂CH₃, -O-CO-OCH₂CH (CH₃) CH) and the like.

In the functional groups represented by formula II-4 or II'-4, R₄ or R₁₄ may represent a linear or branched alkyl group having 1 to 12 carbon atoms, preferably 1 to 9 carbon atoms, more preferably 1 to 5 carbon atoms. Examples of the functional groups represented by formula II-4 or II'-4 may include, but are not limited to, -O-CO-NH-CH₃, -O-CO-NH-CH₂CH₃, -O-CO-NH-(CH₂)₅CH₃ and the like.

In the functional groups represented by formula II-5 or II' -5, R₅ₐ to R_{5c} or R₁₅ₐ to R_{15c} may represent a linear or branched alkyl group having 1 to 12 carbon atoms, preferably 1 to 8 carbon atoms, more preferably 1 to 4 carbon atoms. Examples of the functional groups represented by formula II-5 may include, but are not limited to, -O-Si- (CH₃) ₃, -O-Si- (CH₂CH₃) -O-Si-(CH(CH₃)CH3)₃ and the like.

In the functional groups represented by formula II-6 or II'-6, R₆ is a photoreactive group. Examples of the photoreactive group may include a group having an unsaturated bond group (such as unsaturated double bond group) or a photosensitive group such as an organic residue.

Examples of the unsaturated bond group, for example, the unsaturated double bond group, may include, but are not limited to, olefinyl groups such as an acryl group, a methacryl group, a vinyl ether group, a styryl group and the like.

Examples of the photosensitive group may include, but are not limited to, a cinnamoyl group, a cinnamylidene group, a chalcone residue, an isocoumarin residue, a 2,5-dimethoxystylbene residue, a thymine residue, a styrylpyridinium residue, an α-phenylmaleimide residue, an anthracene residue, a 2-pyrone residue and the like.

Among the functional groups represented by formulae II-1 to II-6, functional groups represented by formulae II-1 to II-3 are preferable, and functional groups represented by formula II-1 and/or II-2 are more preferable.

### <<Linear molecule>>

The linear molecule of a polyrotaxane according to the present invention is not limited as long as the linear molecule may be included in a cavity (cavities) of the cyclic molecule (s) in a skewered manner.

For example, the linear molecule of polyrotaxane in a material according to the present invention may include polyvinyl alcohol, polyvinylpyrrolidone, poly(meth)acrylic acid, cellulose-based resins (carboxymethylcellulose, hydroxyethylcellulose, hydroxypropylcellulose and the like), polyacrylamide, polyethylene oxide, polyethylene glycol, polypropylene glycol, polyvinyl acetal-based resins, polyvinyl methyl ether, polyamine, polyethyleneimine, casein, gelatin, starch and the like and/or copolymers thereof, polyolefin-based resins such as polyethylene, polypropylene, and copolymer resins with other olefinic monomers, polyester resins, polyvinyl chloride resins, polystyrene-based resins such as polystyrene, acrylonitrile-styrene copolymer resin and the like, acrylic resins such as polymethyl methacrylate, copolymer of (meth) acrylate, acrylonitrile-methyl acrylate copolymer resin and the like, polycarbonate resins, polyurethane resins, vinyl chloride-vinyl acetate copolymer resin, polyvinylbutyral resin and the like; and derivatives and modifications thereof, polyisobutylene, polytetrahydrofuran, polyaniline, acrylonitrile-butadiene-styrene copolymer (ABS resin), polyamides such as nylon and the like, polyimides, polydienes such as polyisoprene, polybutadiene and the like, polysiloxanes such as polydimethylsiloxane and the like, polysulfones, polyimines, polyacetic anhydrides, polyureas, polysulfides, polyphosphazenes, polyketones, polyphenylenes, polyhaloolefins, and derivatives thereof. For example, the linear molecule may be selected from the group consisting of polyethylene glycol, polyisoprene, polyisobutylene, polybutadiene, polypropylene glycol, polytetrahydrofuran, polydimethylsiloxane, polyethylene and polypropylene. More specifically, the linear molecule may be selected from the group consisting of polyethylene glycol, polypropylene glycol, polytetrahydrofuran, polydimethylsiloxane, polyethylene and polypropylene, and preferably polyethylene glycol.

A molecular weight of the linear molecule may be 10,000 or more, preferably 20, 000 or more, more preferably 35,000 or more.

In the polyrotaxane according to the present invention, the cyclic molecule may be derived from α-cyclodextrin, and the linear molecule may be polyethylene glycol.

The linear molecule may have the cyclic molecules included in a skewered manner at an amount of 0.001 to 0.6, preferably 0.01 to 0.5, and more preferably 0.05 to 0.4 of a maximum inclusion amount, which is defined as an amount at which the cyclic molecules can be included at maximum when the linear molecule has the cyclic molecules included in a skeweredmanner, and the amount at maximum is normalized to be 1.

The maximum inclusion amount of a cyclic molecule can be determined depending on the length of a linear molecule and the thickness of a cyclic molecule. For example, when the linear molecule is polyethylene glycol and the cyclic molecule is α-cyclodextrin molecule, the maximum inclusion amount is measured experimentally (see, Macromolecules 1993, 26, 5698-5703, whole contents of which is incorporated herein by reference).

### <<Capping group>>

The capping group of the polyrotaxane according to the present invention is not limited, as long as the group is located at both ends of a pseudopolyrotaxane, and the group has an action of preventing dissociation of a cyclic molecule (s) from a linear molecule. The capping group may be selected from the group consisting of dinitrophenyl groups; cyclodextrins; adamantane groups; trityl groups; fluoresceins; pyrenes; substituted benzenes (example of the substituent may include, but are not limited to, alkyl, alkyloxy, hydroxy, halogen, cyano, sulfonyl, carboxyl, amino, phenyl and the like. The substituent may be single orplural.) ; polycyclic aromatics whichmaybe substituted (examples of the substituent may include, but are not limited to, those described above. The substituent may be single or plural.); and steroids. Preferably, the capping group may be selected from the group consisting of dinitrophenyl groups; cyclodextrins; adamantane groups; trityl groups; fluoresceins; and pyrenes, more preferably adamantane groups; or trityl groups.

### <<An ability to respond to an external stimulus>>

The polyrotaxane according to the present invention has an ability to respond reversibly to an external stimulus.

The polyrotaxane according to the present invention can have an ability to respond reversibly to an external stimulus where the polyrotaxane is reversibly transformed from an uncrosslinked state to a crosslinked state or from a crosslinked state to an uncrosslinked state, depending on the presence or the absence of the external stimulus. The term "uncrosslinked state" used herein means a state where at least two molecules of the polyrotaxane are in an uncrosslinked state, and the term "crosslinked state" used herein means a state where at least two molecules of the polyrotaxane are crosslinked.

The external stimulus may include heat, pH, light, electric field, magnetic field and the like. The external stimulus may be heat.

Further, the external stimulus may be heat, and the transition temperature from an uncrosslinked state to a crosslinked state, or from a crosslinked state to an uncrosslinked state may be in the range of 5 to 90°C, preferably 10 to 80°C and more preferably 20 to 60°C.

When the polyrotaxane according to the present invention has an ability to respond reversibly to an external stimulus like this, in particular, the an ability to respond reversibly to heat, applications to materials such as cosmetics, DDS (control of sustained release of drugs caused by the change of temperature, protection of wound site by making use of volume contraction with the change of temperature), biosensors, actuators and the like are considered. Further, fields of applications as well are varied by controlling the transition temperatures. For instance, a polyrotaxane transforming from an uncrosslinked state to a crosslinked state at a body temperature may be applied to a material that may effectively control a retention time of a drug in eyes of an eye lotion. <Crosslinked Polyrotaxane via Physical Bond>

The polyrotaxane according to the present invention may have an ability to respond reversibly to an external stimulus, where the polyrotaxane is reversibly transformed from an uncrosslinked state to a crosslinked state or from a crosslinked state to an uncrosslinked state, depending on the presence or the absence of the external stimulus. Accordingly, the polyrotaxane according to the present invention may provide a crosslinked polyrotaxane via physical bond when at least two molecules of the polyrotaxane are crosslinked.

The "physical bond" used herein is a term used in comparison with the chemical bond, which is mainly made of a covalent bond. The typical examples of the "physical bond" may include van der Waals bond, hydrogen bond, hydrophobic interaction and the like. The physical bond has a feature capable of bonding molecules in a liquid.

The respective constituents of the crosslinked polyrotaxane formed via physical bond such as the cyclic molecule and linear molecule have the same definitions as mentioned above.

The crosslinked polyrotaxane formed from the polyrotaxane according to the present invention or a material having the crosslinked polyrotaxane has an ability to respond reversibly to an external stimulus, in particular, an ability to respond reversibly to heat; and thus, applications to fields same as those mentioned above are expected.

### <Crosslinked Polyrotaxane via Chemical Bond>

The polyrotaxane according to the present invention may provide a crosslinked polyrotaxane via chemical bond (hereinafter, simply referred to as "chemically crosslinked PR" in some cases) by crosslinking the polyrotaxane and other polymer via chemical bond. The present invention can provide a material having the chemically crosslinked PR as well. In the chemically crosslinked PR according to the present invention, the respective constituents derived from a polyrotaxane such as the cyclic molecule and linear molecule have the same definitions as mentioned above.

The chemically crosslinked PR is classified into two, depending on the other polymer: i) a case where the other polymer is a polyrotaxane and ii) a case where the other polymer is a general polymer other than a polyrotaxane (hereinafter, abbreviated as "general polymer"). Further, in the i) case where the other polymer is a polyrotaxane, there are two kinds of a) a case where the polyrotaxane is the polyrotaxane according to the present invention and b) a case where the polyrotaxane is a polyrotaxane other than the polyrotaxane according to the present invention.

The present invention is capable of providing both of these.

In particular, the polyrotaxane according to the present invention has high solubility; and thus, a choice of solvent is expanded. As a result, the polyrotaxane according to the present invention is soluble in solvents that can not be used in the existing polyrotaxane, that is, the compatibility with general polymers is improved. Accordingly, the present invention may provide chemically crosslinked PR with a general polymer that has not been used previously.

### <General Polymer>

In the chemically crosslinked PR according to the present invention, at least a part of the general polymers may be crosslinked with a polyrotaxane according to the present invention. In particular, at least a part of the general polymers may be crosslinked with a polyrotaxane according to the present invention to crosslink by bonding through a cyclic molecule of the polyrotaxane according to the present invention. When crosslinking is formed through a cyclic molecule, the cyclic molecule is able to move along a linear molecule, thereby various characteristics of the chemically crosslinked PR are imparted.

At least a part of the general polymers in the chemically crosslinked polyrotaxane according to the present invention may be physically and/or chemically crosslinked.

The weight ratio of the polyrotaxane to the general polymer ((polyrotaxane)/(general polymer)) may be 1/1000 or more, preferably 1/100 or more, more preferably 1/10.

The general polymer is not limited, and may have on a backbone chain or side chain at least one selected from the group consisting of a -OH group, a -NH₂ group, a -COOH group, an epoxy group, a vinyl group, a thiol group and a photo-crosslinkable group. The photo-crosslinkable group may include, but is not limited to, cinnamic acid, coumarin, chalcone, anthracene, styrylpyridine, styrylpyridinium salt, styrylquinolium salt and the like.

The general polymer may be a homopolymer or a copolymer. Two or more polymers may be present. In a case where two or more polymers are present, at least one polymer may be bound to a polyrotaxane through a cyclic molecule. In a case where the polymer of a material according to the present invention isacopolymer, it maybe composed of two, orthreeormoremonomers. In the case of a copolymer, the copolymer may be one of a block copolymer, an alternating copolymer, a random copolymer, a graft copolymer and the like.

Examples of the general polymer may include, but are not limited to, polyvinyl alcohol, polyvinylpyrrolidone, poly(meth)acrylic acid, cellulose-based resins (carboxymethylcellulose, hydroxyethylcellulose, hydroxypropylcellulose and the like), polyacrylamide, polyethylene oxide, polyethylene glycol, polypropylene glycol, polyvinyl acetal-based resins, polyvinyl methyl ether, polyamine, polyethyleneimine, casein, gelatin, starch and the like and/or copolymers thereof, polyolefin-based resins such as polyethylene, polypropylene, and copolymer resins with other olefinic monomers, polyester resins, polyvinyl chloride resins, polystyrene-based resins such as polystyrene, acrylonitrile-styrene copolymer resin and the like, acrylic resins such as polymethyl methacrylate, copolymer of (meth)acrylate, acrylonitrile-methyl acrylate copolymer resin and the like, polycarbonate resins, polyurethane resins, vinyl chloride-vinyl acetate copolymer resin, polyvinylbutyral resin and the like; and derivatives and modifications thereof, polyisobutylene, polytetrahydrofuran, polyaniline, acrylonitrile-butadiene-styrene copolymer (ABS resin), polyamides such as nylon and the like, polyimides, polydienes such as polyisoprene, polybutadiene and the like, polysiloxanes such as polydimethylsiloxane and the like, polysulfones, polyimines, polyacetic anhydrides, polyureas, polysulfides, polyphosphazenes, polyketones, polyphenylenes, polyhaloolefins, and derivatives thereof. The derivatives may contain the above-described group, i.e., at least one selected from the group consisting of a -OH group, a -NH₂ group, a -COOH group, an epoxy group, a vinyl group, a thiol group and a photo-crosslinkable group.

The chemically crosslinked PR according to the present invention, in particular ii)-a, that is the chemically crosslinked PR that uses the polyrotaxane according to the present invention as the other polymer that forms chemical crosslink is capable of having characteristics shown below. «Characteristics of Chemically Crosslinked PR-Transmittance, Extension Ratio and Young's Modulus»

The chemically crosslinked PR according to the present invention may have high transmittance. Specifically, the chemically crosslinked PR according to the present invention may have the transmittance of 90%/mm or more, preferably 95%/mm or more and more preferably 98%/mm or more in a wavelength from 400 to 800 nm.

Further, the chemically crosslinked PR according to the present invention may provide an extension ratio in the range of 100 to 1500%, preferably of 200% or more, more preferably of 300% or more, still more preferably 600% or more, most preferably of 1000% or more.

More, the chemically crosslinked PR according to the present invention may have the transmittance of 90%/mm or more, preferably 95%/mm or more, more preferably 98%/mm or more in a wavelength from 400 to 800 nm and an extension ratio of 300% or more, preferably 600% or more, more preferably of 1000% or more.

Further, the chemically crosslinked PR according to the present invention may provide not only high transmittance and high extension ratio but also high Young's modulus. For instance, the chemically crosslinked PR according to the present invention may provide the Young's modulus such high as 10 kPa or more.

Accordingly, the present invention may provide chemically crosslinked PR that is capable of simultaneously satisfying three of transmittance, extension ratio and Young's modulus.

### <<Characteristics of Chemically Crosslinked PR - External Stimulus Response ->>

The chemically crosslinked PR according to the present invention may reversibly vary in an optical property and/or swelling-contraction characteristics, depending on the presence or the absence of an external stimulus.

In particular, the optical property and/or swelling-contraction characteristics may reversibly vary when the external stimulus is heat and a temperature is from 5 to 90°C, preferably from 10 to 80°C and more preferably from 20 to 60°C.

Herein, the optical property may be transparence of the crosslinked polyrotaxane. The term "transparence" means that the transmittance of the crosslinked polyrotaxane is 90%/mm or more, preferably 95%/mm or more, more preferably 98%/mm or more in a wavelength from 400 to 800 nm.

The swelling-contraction characteristics may be a change of an amount of a contained solvent caused by absorption and/or release of the solvent by the crosslinked polyrotaxane. That is, the swelling-contraction characteristics may be defined by normalizing a volume change when the crosslinked polyrotaxane swells and contracts by an amount of solvent contained in the crosslinked polyrotaxane. In brief, the swelling-contraction characteristics of the crosslinked polyrotaxane can be normalized by a content of a solvent contained in the crosslinked body, that is, water content = (weight of hydrogel) / (weight of dry gel).

The above-described chemically crosslinked PR having an ability to respond to an external stimulus or a material comprising the chemically crosslinked PR can be applied to materials such as materials for cosmetics, that is, materials in which effective ingredient release can be effectively controlled at a body temperature.

Further, by controlling the transition temperature variously, the crosslinked body according to the present invention may be used in DDS (such as control of sustained release of drugs caused by temperature variation and protection of wound site by making use of volume contraction caused by the temperature variation), above-mentioned cosmetic materials, biosensors and actuators.

When a transition speed at the transition temperature is variously controlled, a sustained release speed in the DDS and an effective ingredient release speed in the cosmetic materials may be controlled. In the present invention, the transition temperature and/or transition speedmaybe controlled by variously combining substances constituting the chemically crosslinked PR such as a cyclic molecule, a linear molecule, a functional group represented by formula I and a functional group represented by formula II, and the like.

A polyrotaxane according to the present invention, a physically crosslinked body formed via physical bond of at least two molecules of the polyrotaxane and a chemically crosslinked PR as well as materials comprising them can be produced according to the methods shown below.

### <Producing Method of Polyrotaxane according to the present invention>

A polyrotaxane according to the present invention may be produced as shown below:

The method comprises:
1) a step of preparing a pseudopolyrotaxane;
2) a step of capping each of both ends of the resulting pseudopolyrotaxane with capping groups to prepare a polyrotaxane; and
3) a step of substituting a part of hydroxyl groups of a cyclic molecule with a functional group,
   wherein the step of substituting with the functional group is carried out
   A) before 1) the step of preparing a pseudopolyrotaxane and/or,
   B) after 2) the step of capping to prepare a polyrotaxane; thereby to obtain a polyrotaxane according to the present invention.

In this regard, the cyclic molecule may be a molecule having a hydroxyl group. Further, the other substances constituting a polyrotaxane, such as the linear molecule, the capping group and the like can be used in the same way as mentioned above.

For 1) the step of preparing a pseudopolyrotaxane, various existing methods may be used. For instance, WO2005/052026 and WO2005/08469 may be referenced.

For 2) the step of capping to prepare a polyrotaxane as well, in a manner similar to 1) the step of preparing a pseudopolyrotaxane, various existing methods may be used. For instance, WO2005/052026 and WO2005/08469 may be referenced.
3) The step of substituting is a step of substituting a part of hydroxyl groups of a cyclic molecule with the functional group. The step may be carried out A) before 1) the step of preparing a pseudopolyrotaxane and/or B) after 2) the step of capping to prepare a polyrotaxane, and preferably carried out after 2) the step of capping to prepare a polyrotaxane.

In the step of substituting, a step of introducing a functional group represented by formula I may be carried out
X) before a step of introducing any one of the functional groups represented by formula II;
Y) after a step of introducing any one of the functional groups represented by formula II; or
Z) simultaneously with the step of introducing any one of the functional groups represented by formula II, and preferably Y) after a step of introducing any one of the functional groups represented by formula II.

More specifically, 3) the step of substituting may be carried out under the conditions shown below. For instance when the 3) the step of substituting is carried out after the 2) step of preparing polyrotaxane and an acetyl group is used as a functional group represented by formula I, dimethylacetamide, acetic anhydride or triethylamine may be used as the solvent at a temperature of 25°C.

Further, when a functional group represented by formula II, in particular a functional group represented by formula II-2 such as a hydroxypropyl group is introduced after the functional group of the formula I is introduced, an aqueous solution of sodium hydroxide or oxidized propylene may be used as a solvent at a temperature of 25°C.

### <Producing Method of Physically Crosslinked Body>

A physically crosslinked body formed via physical bond of at least two molecules of the above-obtained polyrotaxane can be produced as follows:

A physically crosslinked body can be produced according to a method comprising:
4) a step of dissolving at least two molecules of the resulting polyrotaxane in a solvent; and
5) a step of imparting an external stimulus to the at least two molecules of the polyrotaxane in the solvent to physically crosslink the polyrotaxane via physical bond.

In the 4) step of dissolving, a solvent is not particularly restricted as far as it dissolves a polyrotaxane. The solvent may be a hydrophilic solvent such as water.

The 5) step of physically crosslinking is a step of imparting an external stimulus. As the external stimulus, those cited above may be cited, heat being particularly preferable.

### <Producing Method of Chemically Crosslinked PR>

A chemically crosslinked PR can be produced as shown below with the resulting polyrotaxane described above:

A chemically crosslinked PR is obtained by including a step of crosslinking 4) the resulting first polyrotaxane and a first polymer via chemical bond.

Herein, the step of chemically crosslinking depends on a polymer used, a polyrotaxane used (such as a functional group represented by formula I used, a functional group represented by formula II used) and the like. The step of chemically crosslinking may be carried out through G) a crosslinking reaction owing to an addition of a crosslinking agent or H) a photo-crosslinking reaction where a photo-reactive group contained in the first polyrotaxane is irradiated with light.

In a case where G) a crosslinking reaction is caused by addition of a crosslinking agent, examples of the crosslinking agent may include, but are not limited to, cyanuric chloride, trimesoyl chloride, terephthaloyl chloride, epichlorohydrin, dibromobenzene, glutaraldehyde, aliphatic polyfunctional isocyanate, aromatic polyfunctional isocyanate, tolylene diisocyanate, hexamethylene diisocyanate, divinyl sulfone, 1,1'-carbonyldiimidazole, alkoxysilanes and derivatives thereof.

In a case where H) photo-irradiation is used, the polyrotaxane comprises the function group represented by formula II-6, and the photo-reactive group of the function group is a group having an unsaturated bond, the photo-crosslinking reaction is initiated by means of photo-irradiation and a reaction initiator to generate bindings between the groups each having the unsaturated bond. In this case, a reaction initiator may be present in the reaction field. Alternatively, in a case where the photo-reactive group is a photosensitive group, the photo-crosslinking reaction is initiated by photo-irradiation to generate bindings between photosensitive groups without using a reaction initiator.

The reaction initiator may be at least one selected from the group consisting of quinones, aromatic ketones, benzoin, benzoin ethers, biimidazole compounds and derivatives thereof, N-phenylglycines, combinations of thioxanthones and alkylaminobenzoic acid, combinations of biimidazole compounds and derivatives thereof and Michler's ketones, acridines, and oxime esters. Specifically, the reaction initiator may be quinones such as 2-ethylanthraquinone, octaethylanthraquinone, 1,2-benzanthraquinone, 2,3-benzanthraquinone, 2-phenylanthraquinone, 2,3-diphenylanthraquinone, 1-chloroanthraquinone, 1,4-naphthoquinone, 9,10-phenanthraquinone, 2-methyl-1,4-naphthoquinone, 2,3-dimethylanthraquinone, 3-chloro-2-methylanthraquinone and the like; aromatic ketones such as benzophenone, Michler's ketone[4,4'-bis(dimethylamino)benzophenone], 4,4'-bis(diethylamino)benzophenone and the like; benzoin etherssuch asbenzoin,benzoinethylether,benzoinphenylether, methylbenzoin, ethylbenzoin and the like; biimidazole compounds and derivatives thereof such as benzyldimethyl ketal, benzyldiethyl ketal, triarylimidazolyl dimers and the like; N-phenylglycines such as N-phenylglycine, N-methyl-N-phenylglycine, N-ethyl-N-phenylglycine and the like; combinations of thioxanthones and alkylamine benzoic acid such as a combination of ethylthioxanthone and ethyl dimethylaminobenzoate, a combination of 2-chlorothioxanthone and ethyl dimethylaminobenzoate, a combination of isopropylthioxanthone and ethyl dimethylbenzoate, and the like; combinations of biimidazole compounds such as triarylimidazolyl dimers and derivatives thereof and Michler's ketone; acridines such as 9-phenylacridine and the like; and oxime esters such as 1-phenyl-1,2-propanedione-2-o-benzoineoxime, 1-phenyl-1,2-propanedione-2-(o-ethoxycarbonyl)oxime and the like. Preferably, the reaction initiator may be thioxanthones such as diethylthioxanthone, chlorothioxanthone and the like; dialkylaminobenzoate esters such as ethyl dimethylaminobenzoate and the like; benzophenone, 4,4'-bis(dimethylamino)benzophenone, 4,4'-bis(diethylamino)benzophenone; biimidazole compounds such as triarylimidazolyl dimers and derivatives thereof; 9-phenylacridine, N-phenylglycines; and combinations thereof. Furthermore, the biimidazole compounds such as triarylimidazolyl dimers and derivatives thereof may include, for example, 2-(o-chlorophenyl)-4,5-diphenylimidazolyl dimers, 2,2',5-tris-(o-chlorophenyl)-4-(3,4-dimethoxyphenyl)-4',5'-diphenylimidazolyl dimers, 2,4-bis-(o-chlorophenyl)-5-(3,4-dimethoxyphenyl)-diphenylim idazolyl dimers, 2,4,5-tris-(o-chlorophenyl)-diphenylimidazolyl dimers, 2-(o-chlorophenyl)-bis-4,5-(3,4-dimethoxyphenyl)-imidazolyl dimers, 2,2'-bis-(2-fluorophenyl)-4,4',5,5'-tetrakis-(3-methoxyphen yl)-imidazolyl dimers, 2,2'-bis-(2,3-difluoromethylphenyl)-4,4',5,5'-tetrakis-(3-m ethoxyphenyl)-imidazolyl dimers, 2,2'-bis-(2,4-difluorophenyl)-4,4',5,5'-tetrakis-(3-methoxy phenyl)-imidazolyl dimers, 2,2'-bis-(2,5-difluorophenyl)-4,4',5,5'-tetrakis-(3-methoxy phenyl)-imidazolyl dimers, 2,2'-bis-(2,6-difluorophenyl)-4,4',5,5'-tetrakis-(3-methoxy phenyl)-imidazolyl dimers, 2,2'-bis-(2,3,4-trifluorophenyl)-4,4',5,5'-tetrakis-(3-meth oxyphenyl)-imidazolyl dimers, 2,2'-bis-(2,3,5-trifluorophenyl)-4,4',5,5'-tetrakis-(3-meth oxyphenyl)-imidazolyl dimers, 2,2'-bis-(2,3,6-trifluorophenyl)-4,4',5,5'-tetrakis-(3-meth oxyphenyl)-imidazolyl dimers, 2,2'-bis-(2,4,5-trifluorophenyl)-4,4',5,5'-tetrakis-(3-meth oxyphenyl)-imidazolyl dimers, 2,2'-bis-(2,4,6-trifluorophenyl)-4,4', 5, 5' -tetrakis- (3-meth oxyphenyl)-imidazolyl dimers, 2,2'-bis-(2,3,4,5-tetrafluorophenyl)-4,4',5,5'-tetrakis-(3-methoxyphenyl)-imidazolyl dimers, 2,2'-bis-(2,3,4,6-tetrafluorophenyl)-4,4',5,5'-tetrakis-(3-methoxyphenyl)-imidazolyl dimers, 2,2'-bis-(2,3,4,5,6-pentafluorophenyl)-4,4',5,5'-tetrakis-( 3-methoxyphenyl)-imidazolyl dimers, and the like.

The present invention will be illustrated more specifically by way of following Examples, but is not limited thereby.

Example 1:

### <1-1. (Synthesis of Hydroxypropylated Polyrotaxane, substitution rate 48%>

Adamantane polyrotaxane (hereinafter, abbreviated as "APR") made of polyethylene glycol (average molecular weight: 35,000) as a linear molecule and an adamantane group as a capping group was prepared in a manner similar to a method described in WO2005/080469.

In a three-neck flask, α-cyclodextrin (hereinafter, abbreviated as "α-CD" in some cases) as a cyclic molecule and 200 g of APR were dissolved in 1 L of an aqueous solution of 1.5N NaOH, followed by dropping 400 g of propylene oxide therein over 4 hours (solution temperature: 30°C or lower). The solution was stirred for 20 hours with a vessel hermetically sealed, followed by neutralizing with 6N hydrochloric acid. The solution was dialyzed with a dialysis tube (fraction molecular weight: 12,000) for 48 hours in flowing tap water. The resulting solution was further dialyzed for 3 hours twice in purified water. The resulting solution was freeze dried and a yield of resulting hydroxypropylated APR (hereinafter, abbreviated as "HAPR" in some cases) was 240 g (α-CD incorporation amount: 0.27).

The rate of hydroxypropyl substitution was substantially 48% by ¹H-NMR. A weight-average molecular weight Mw was 120,000 and a molecular weight distribution Mw/Mn was 1.3 by GPC. As the result of a thermal analysis by TG/DTA, a decomposition temperature was 350°C (in air).
¹H-NMR, (DMSO-d₆, 400MHz), δ(ppm) 1.0 (s, 1.1H), 3.0-4. 0 (m, 3.6H), 4.0-6.0(m, 1H).

### <1-2. Synthesis of Acetylated body of HAPR; H = 48%; E2 = 8%>

3.0 g of HAPR (degree of hydroxypropylation: 48%) prepared in the above 1-1 was dissolved in 45 ml of dehydrated N,N'-dimethylacetoamide (DMA). Thereto, 0.6 ml of triethylamine and 0.28 ml of acetic anhydride (10% by mol relative to the number of hydroxyl groups of HAPR) were sequentially added, followed by reaction for 5 hr. Thereafter, the solution was dropped in 360 ml of hexane and a precipitate was dialyzed by a dialysis tube (fraction molecular weight: 12,000) for 24 hours in flowing tap water. The dialysis was further performed twice in purified water for 3 hours. By freeze-drying the precipitate, 3.0 g of a product (acetylated substance of HAPR. Hereinafter, "acetylated substance of HAPR" is generally abbreviated as "E2-HAPR" in some cases) was obtained. An weight-average molecular weight Mw thereof was 150,000 and a molecular weight distribution Mw/Mn was 1.2 by GPC. As the result of a thermal analysis by TG/DTA, a decomposition temperature was 346°C (in air). The degree of acetylation relative to a total number of hydroxyl groups, that is, the degree of acetylation with the number of hydroxyl groups derived from α-CD normalized to 100% by ¹H-NMR, was 8%. Hereinafter, in some cases, the degree of hydroxypropylation and degree of acetylation are abbreviated simply as "H" and "E2", respectively. Regarding E2-HAPR obtained in Example 1, H was 48% and E2 was 8%.
¹H-NMR, (DMSO-d₆, 400MHz), δ (ppm) 1.0 (ds, 1.0H), 2.0 (ds, 0.18H), 3.0-4.0(m, 3.2H), 4.0-6.0(m, 0.89H).

### Example 2:

### <Synthesis of E2-HAPR; H = 48%, E2 = 18%>

E2-HAPR was prepared in a manner similar to a method of Example 1, except that an amount of triethylamine and an amount of acetic anhydride in Example 1-2 were changed to 1.2 ml and 0.556 ml (20% by mol relative to the number of hydroxyl groups of HAPR), respectively. The resulting product was 3.0 g. A weight-average molecular weight Mw was 140,000 and a molecular weight distribution Mw/Mn was 1.3 by GPC. The degree of acetylation relative to a total number of hydroxyl groups by ¹H-NMR was 18% (H = 48% and E2 = 18%).
¹H-NMR, (DMSO-d₆, 400MHz), δ (ppm) 1.0 (ds, 1.0H), 2.0 (ds, 0.37H), 3.0-6.4(m, 4.3H).

### Example 3:

### <Synthesis of E2-HAPR; H = 48%, E2 = 35%>

E2-HAPR was prepared in a manner similar to a method of Example 1, except that an amount of triethylamine and an amount of acetic anhydride in Example 1-2 were changed to 2.4 ml and 1.1 ml (40% by mol relative to the number of hydroxyl groups of HAPR), respectively. The resulting product was 3.3 g. A weight-average molecular weight Mw was 140,000 and a molecular weight distribution Mw/Mn was 1.2 by GPC. The degree of acetylation relative to a total number of hydroxyl groups by ¹H-NMR was 35% (H = 48% and E2 = 35%).
¹H-NMR, (DMSO-d₆, 400MHz), δ (ppm) 1.0 (ds, 1.0H), 2.0 (ds, 0.73H), 3.0-6.4(m, 4.3H).

### Example 4:

### <Synthesis of E2-HAPR; H = 48%, E2 = 47%>

E2-HAPR was prepared in a manner similar to a method of Example 1, except that an amount of triethylamine and an amount of acetic anhydride in Example 1-2 were changed to 2.4 ml and 1.4 ml (50% by mol relative to the number of hydroxyl groups of HAPR), respectively. The resulting product was 3.1 g. A weight-average molecular weight Mw was 150, 000 and a molecular weight distribution Mw/Mn was 1.3 by GPC. The degree of acetylation relative to a total number of hydroxyl groups by ¹H-NMR was 47% (H = 48% and E2 = 47%).
¹H-NMR, (DMSO-d₆, 400MHz), δ (ppm) 1.0 (ds, 1.0H), 2.0 (ds, 0.97H), 3.0-6.4(m, 4.2H).

### Example 5:

### <Synthesis of E2-HAPR; H = 48%, E2 = 79%>

E2-HAPR was prepared in a manner similar to a method of Example 1, except that 0.6 ml of triethylamine in Example 1-2 was changed to 7.2 ml of pyridine and an amount of acetic anhydride in Example 1-2 was changed to and 1.1 ml. The resulting product was 3.8 g. A weight-average molecular weight Mw was 170,000 and a molecular weight distribution Mw/Mn was 1.3 by GPC. As the result of a thermal analysis by TG/DTA, a decomposition temperature was 343°C (in air). The degree of acetylation relative to a total number of hydroxyl groups was 79% (H = 48% and E2 = 79%).
¹H-NMR, (DMSO-d₆, 400MHz), δ(ppm) 1.0(s, 1.0H), 2.0(s, 1.7H), 3.0-6.4(m, 3.8H).

### Example 6:

### <6-1. Synthesis of HAPR; H = 9%>

HAPR was prepared in a manner similar to a method of Example 1-1, except that amounts of APR, NaOH aqueous solution and propyleneoxide were changed to 50 g, 250 g and 15 g, respectively. A weight-average molecular weight Mw was 110, 000 and a molecular weight distribution Mw/Mn was 1.3 by GPC. As the result of a thermal analysis by TG/DTA, a decomposition temperature was 300°C (in air). The degree of hydroxypropylation relative to a total number of hydroxyl groups by ¹H-NMR was about 9%.
¹H-NMR, (DMSO-d₆, 400MHz), δ (ppm) 1.0(s, 0.21H), 3.0-4.0(m, 2.9H), 4.0-6.0 (m, 1H).

### <6-2. Synthesis of E2-HAPR; H = 9%; E2 = 40%>

3.0 g of HAPR (H= 9%) prepared in the above 6-1 was dissolved in 45 ml of dehydrated DMA. Thereto, 2.9 ml of triethylamine and 2.0 ml of acetic anhydride were sequentially added, followed by reaction for 5 hr. Thereafter, the solution was dropped in 360 ml of hexane and a precipitate was dialyzed by a dialysis tube (fraction molecular weight: 12, 000) for 24 hours in flowing tap water. The dialysis was further performed twice in purified water for 3 hours. By freeze-drying the precipitate, 3.0 g of a product was obtained. An weight-average molecular weight Mw thereof was 130, 000 and a molecular weight distribution Mw/Mn was 1.3 by GPC. The degree of acetylation relative to a total number of hydroxyl groups by ¹H-NMR was 40% (H = 9%; E2 = 40%). ¹H-NMR, (DMSO-d₆, 400MHz), δ (ppm) 1.0(ds, 1.0H), 2.0(s, 4.6H), 3.0-6.4(m, 19.3H).

### Example 7:

### <7-1. Synthesis of HAPR; H = 25%>

HAPR was prepared in a manner similar to a method of Example 1-1, except that amounts of APR, 1.5 N NaOH aqueous solution and propylene oxide were changed to 50 g, 250 g and 45 g, respectively. A weight-average molecular weight Mw was 110,000 and a molecular weight distribution Mw/Mn was 1.3 by GPC. As the result of a thermal analysis by TG/DTA, a decomposition temperature was 328°C (in air). The degree of hydroxypropylation relative to a total number of hydroxyl groups by ¹H-NMR was about 25% (H = 25%).
¹H-NMR, (DMSO-d₆, 400MHz), δ (ppm) 1.0(s, 0.56H), 3.0-4.0(m, 3.3H), 4.0-6.0(m, 1H).

### <7-2. Synthesis of E2-HAPR; H = 25%; E2 = 40%>

3.0 g of HAPR (H = 25%) prepared in the above 7-1 was dissolved in 45 ml of dehydrated DMA. Thereto, 2.9 ml of triethylamine and 2.0 ml of acetic anhydride were sequentially added, followed by reaction for 5 hr. Then, the solution was dropped in 360 ml of hexane and a precipitate was dialyzed by a dialysis tube (fraction molecular weight: 12,000) for 24 hours in flowing tap water. The dialysis was further performed twice in purified water for 3 hours, followed by freeze-drying the precipitate, toprepareE2-HAPR. 3.0 g of a product was obtained. An weight-average molecular weight Mw thereof was 120,000 and a molecular weight distribution Mw/Mn was 1.3 by GPC. The degree of acetylation relative to a total number of hydroxyl groups by ¹H-NMR was 40% (E2 = 40%).
¹H-NMR, (DMSO-d₆, 400MHz), δ (ppm) 1.0 (ds, 1.0H), 2.0(ds, 1.6H), 3.0-6.4(m, 7.2H).

### Example 8:

### <8-1. Synthesis of HAPR; H = 63%>

HAPR was prepared in a manner similar to a method of Example 1-1, except that amounts of APR, NaOH aqueous solution and propylene oxide were changed to 50 g, 250 g and 150 g, respectively. A weight-average molecular weight Mw was 130, 000 and a molecular weight distribution Mw/Mn was 1.4 by GPC. The degree of hydroxypropylation relative to a total number of hydroxyl groups by ¹H-NMR was about 63% (H = 63%).
¹H-NMR, (DMSO-d₆, 400MHz), δ (ppm) 1.0 (s, 1.4H), 3.0-4.0(m, 4.1H), 4.0-6.0 (m, 1H).

### <8-2. Synthesis of E2-HAPR; H = 63%; E2 = 40%>

2.0 g of HAPR (H = 63%) prepared in the above item 8-1 was dissolved in 30 ml of dehydrated DMA. Thereto, 1.3 ml of pyridine and 1.3 ml of acetic anhydride were sequentially added, followed by reaction for 5 hr. Then, the solution was dropped in 240 ml of hexane and a precipitate was dialyzed by a dialysis tube (fraction molecular weight: 12, 000) for 24 hours in flowing tap water. The dialysis was further performed twice in purified water for 3 hours, followed by freeze-drying the precipitate, to prepare E2-HAPR. 2.1 g of a product was obtained. An weight-average molecular weight Mw thereof was 140,000 and a molecular weight distribution Mw/Mn was 1.3 by GPC. The degree of acetylation relative to a total number of hydroxyl groups by ¹H-NMR was 40%.
¹H-NMR, (DMSO-d₆, 400MHz), δ (ppm) 1.0 (ds, 1.0H), 2.0(s, 0.64H), 3.0-6.4 (m, 3.4H).

### Example 9:

### <Synthesis of E3-HAPR; H = 48%; E3 = 27%>

3.0 g of HAPR (H = 48%) prepared in the above item 1-1 in Example 1 was dissolved in 45 ml of dehydrated DMA. Thereto, 2.0 ml of triethylamine and 1.9 ml of propionic anhydride were sequentially added, followed by reaction for 5 hr. Then, the solution was dropped in 360 ml of hexane and a precipitate was dialyzed by a dialysis tube (fraction molecular weight: 12,000) for 24 hours in flowing tap water. The dialysis was further performed twice in purified water for 3 hours, followed by freeze-drying the precipitate, to obtain propionated HAPR (as described above, "propionated HAPR" is abbreviated as "E3-HAPR" in some cases). 2.9 g of a product was obtained. An weight-average molecular weight Mw thereof was 140,000 and a molecular weight distribution Mw/Mn was 1.2 by GPC. The degree of propionylation relative to a total number of hydroxyl groups by ¹H-NMR was 27% (H = 48%; E3 = 27%).
¹H-NMR, (DMSO-d₆, 400MHz), δ (ppm) 1.0(s, 1.0H), 2.0(s, 0.31H), 3.0-6.4 (m, 2.2H).

### Example 10:

### <10-1. Synthesis of 1,2-dihydroxypropylated Polyrotaxane>

APR was prepared in a manner similar to Example 1. In a three-neck flask, α-CD, polyethylene glycol and 50 g of APR were dissolved in 250 ml of an aqueous solution of 1.5N NaOH and 125 g of glycidol was dropped therein over 1 hours (a solution temperature was set at 30°C or less). The solution was stirred for 20 hours with the vessel hermetically sealed, followed by neutralizing with hydrochloric acid. The solution was dialyzed with a dialysis tube (fraction molecular weight: 12,000) for 48 hours in flowing tap water. The resulting solution was further dialyzed twice in purified water for 3 hours. The resulting solution was freeze dried, to obtain 1,2-dihydroxypropylated APR (hereinafter, simply abbreviated as "GAPR") (yield: 59 g). The substitution rate of 1,2-dihydroxypropyl (hereinafter, simply referred to as "G") to a total number of hydroxyl groups was substantially 42% by ¹H-NMR (G: 42%). A weight-average molecular weight Mw was 130,000 and a molecular weight distribution Mw/Mn was 1.3 by GPC. As the result of a thermal analysis by TG/DTA, a decomposition temperature was 331°C (in air).
¹H-NMR, (DMSO-d₆, 400MHz), δ (ppm) 3.0-4.0 (m, 3.3H), 4.0-6.4 (m, 1.0H).

### <Synthesis of E3-GAPR; G = 42%; E2 = 49%>

2.0 g of GAPR (G = 42%) prepared in the above item 10-1 was dissolved in 30 ml of dehydrated DMA. Thereto, 2.0 ml of triethylamine and 1.9 ml of acetic anhydride were sequentially added, followed by reaction for 5 hr. Then, the solution was dropped in 360 ml of hexane and a precipitate was dialyzed by a dialysis tube (fraction molecular weight: 12,000) for 24 hours in flowing tap water. The dialysis was further performed for 3 hours twice in purified water, followed by freeze-drying the precipitate, to obtain acetylated GAPR (as described above, "acetylated GAPR" is simply abbreviated as "E2-GAPR"). 2.5 g of a product was obtained. An weight-average molecular weight Mw thereof was 150, 000 and a molecular weight distribution Mw/Mn was 1.3 by GPC. The degree of acetylation relative to a total number of hydroxyl groups by ¹H-NMR was 49% (G = 42%; E2 = 49%). ¹H-NMR, (DMSO-d₆, 400MHz), δ (ppm) 2.0(s, 1.0H), 3.0-6.4 (m, 3.1H).

### Example 11:

### <Synthesis of E2 + Photoreactive Group-HAPR>

3.0 g of HAPR prepared in 1-1 of Example 1 (rate of hydroxypropylation: 48%) was dissolved in 45 ml of dehydrated DMA, followed by adding 0.06 ml of acryloyloxyethyl isocyanate (manufactured by SHOWA DENKO K. K.) and reacting for 3 hr, further followed by adding 2.2 ml of triethylamine and 1.40 ml of acetic anhydride and further reacting for 5 hr. Thereafter, the solution was dropped in 360 ml of hexane and the resulting precipitatewasdialyzedwithadialysistube (fraction molecular weight: 12,000) for 24 hours in flowing tap water. The precipitate was further dialyzed twice each for 2 hours in purified water. The precipitate was freeze-dried and thereby (E2 + photoreactive group-HAPR) obtained by further acetylating and acryloyloxyethyl-carbamating (photoreactive group) hydroxypropylated α-CD of HAPR were prepared. The resulting product was 3.2 g. The rate of acetylation to a total number of hydroxyl groups was 16% and the substitution rate of acryloyloxyethyl carbamate groups by ¹H-NMR was substantially 2%.

### Comparative Example 1:

HAPR having the substitution rate of 48% with hydroxypropyl groups was prepared in a manner similar to 1-1 of Example 1.

### Comparative Example 2:

GAPR having the substitution rate of 42% with 1, 2-dihydroxypropyl groups was prepared in a manner similar to 10-1 of Example 10.

### Comparative Example 3:

The APR was only acetylated in a manner similar to that of 6-2 of Example 6. That is, E2-APR was obtained by acetylating α-CD in a manner similar to 6-2 of Example 6 except that, in 6-2 of Example 6, 3.0 g of APR was used in place of 3.0 g of HAPR (H = 9%), 1.9 ml of acetic anhydride was used in place of 2.0 ml of acetic anhydride and 1.8 g of lithium chloride was used. When the rate of acetylation was measured in a manner similar to 6-2 of Example 6, E2 was 40%.

### Comparative Example 4:

According to a method described in Patent Document 2, OH groups of α-CD of APR were methylated. The rate of methylation was 95% (M = 95%). Hereinafter, the resulting methylated APR is abbreviated as "MAPR" in some cases.

### Comparative Example 5:

Of the HAPR (H = 48%) obtained in 1-1 of Example 1, OH groups were further methylated according to a method described in Patent Document 2. The rate of methylation was 98%. Hereinafter, the resulting methylated HAPR is abbreviated as "MHAPR" in some cases.

The polyrotaxanes obtained in Examples 1 to 11 and Comparative Examples 1 to 5 are summarized in Table 1.

**Table 1. Polyrotaxanes obtained in Examples 1-11 and Comparative Examples 1-5**

| | First Substitution | Second Substitution | Abbreviation |
|---|---|---|---|
| Ex. 1 | H = 48 % | E2 = 8 % | E2-HAPR (H=48,E2=8) |
| Ex. 2 | H = 48 % | E2 = 18 % | E2-HAPR (H=48, E2=18) |
| Ex. 3 | H = 48 % | E2 = 35 % | E2-HAPR (H=48, E2=35) |
| Ex. 4 | H = 48 % | E2 = 47 % | E2-HAPR (H=48, E2=47) |
| Ex. 5 | H = 48 % | E2 = 79 % | E2-HAPR (H=48, E2=79) |
| Ex. 6 | H = 9 % | E2 = 40 % | E2-HAPR (H=9, E2=40) |
| Ex. 7 | H = 25 % | E2 = 40 % | E2-HAPR (H=25, E2=40) |
| Ex. 8 | H = 63 % | E2 = 40 % | E2-HAPR (H=63, E2=40) |
| Ex. 9 | H = 48 % | E3 = 27 % | E3-HAPR (H=48, E3=27) |
| Ex. 10 | G = 42 % | E2 = 49 % | E2-GAPR (G=42, E2=49) |
| Ex. 11 | H = 48 % | E2 = 16 % | E2+photo-HAPR (H=48, E2=8) |
| | | Photo-crosslinking Group 2% | |
| Comp. Ex. 1 | H = 48 % | - | HAPR (H=48) |
| Comp. Ex. 2 | G = 42 % | - | GAPR (G=42) |
| Comp. Ex. 3 | E2 = 40 % | - | E2-APR (E2=40) |
| Comp. Ex. 4 | M = 95 % | - | MAPR (M=95) |
| Comp. Ex. 5 | H = 48 % | M = 98 % | MHAPR (H=48, M=98) |

### <A. Solubility of Various Polyrotaxanes in Various Solvents>

### <<A-1. Case where Substitution Rates of Hydroxypropyl groups in First Substitution are same>>

The solubility was compared between cases where a first substitution was hydroxypropylation and the substitution rates thereof were constant (H = 48%). Specifically, regarding Examples 1 to 5, 9 and 11, and Comparative Example 1, the solubility was compared between various solvents. The results are shown in Table 2. In Table 2, when 0.02 g of each of the polyrotaxanes was added as a solute in 1 ml of a solvent followed by stirring for 2 hr, a case where the polyrotaxane was dissolved is marked with o, a case where a residue was found is marked with Δ, and a case where the polyrotaxane was not dissolved is marked with ×.

Table 2 shows that as the acetylation rate becomes higher (a value of E2 becomes larger), the solubility of E2-HAPR in a halogen-based solvent such as dichloroethane or dichloromethane increases. Furthermore, it is found that, in Example 9 where the second substitution was propionylation (E3), the polyrotaxane was dissolved in all solvents described in Table 2 except for water.

The results show that the polyrotaxane according to the present invention exhibits improved solubility to various solvents. Further, as the result of improvement in the solubility, a solvent for dissolving the polyrotaxane is chosen more widely.

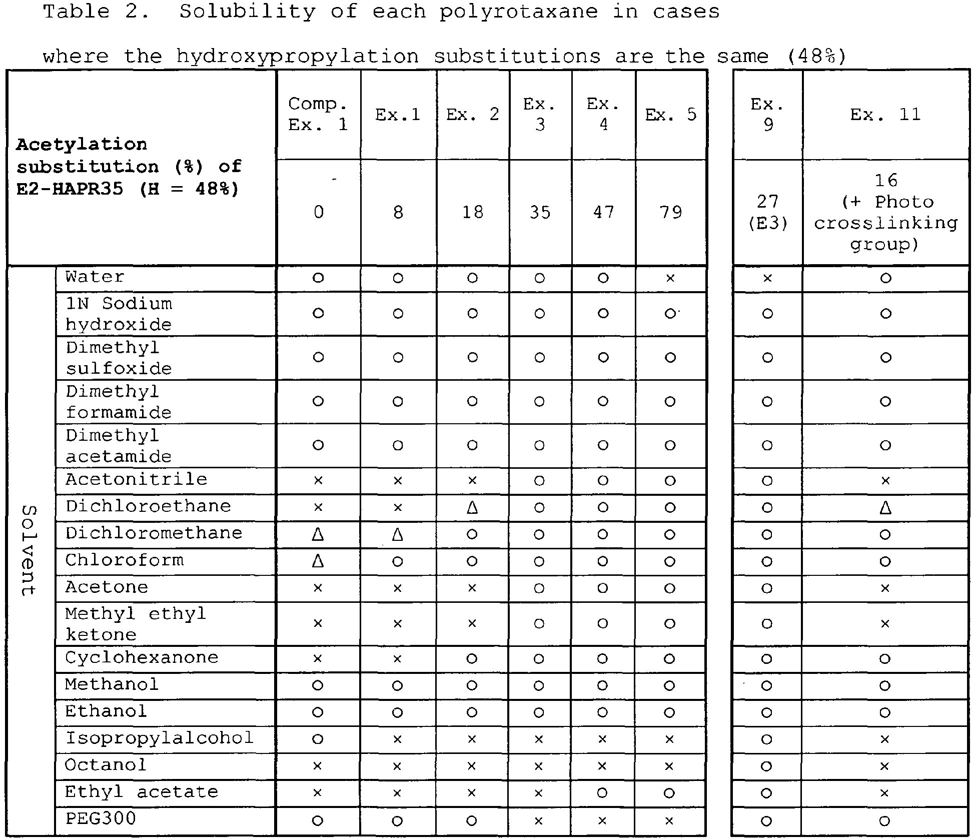

### «A-2. Case where Acetyl Substitution Rates in Second Substitution are same»

The solubility was compared between cases where a second substitution was acetylation and the substitution rates thereof were constant (E2 = 40%). Specifically, regarding Examples 6 to 8 and Comparative Example 3, the solubility was compared among various solvents. The substitution in comparative Example 3 was only with acetylation. The results are shown in Table 3. Marks "o", "Δ" and "×" in Table 3 have meanings same as that in A-1.

Table 3 shows that as the hydroxypropylation rate becomes higher (a value of H becomes larger), the solubility of E2-HAPR in water, halogen-based solvent (such as dichloroethane or dichloromethane), ketone-based solvents (such as acetone, methyl ethyl ketone, cyclohexanone) and low molecular weight PEG (molecular weight: 300) increases.

The results show that the polyrotaxane according to the present invention exhibits improved solubility in various solvents. Further, as the result of improvement in the solubility, a solvent for dissolving the polyrotaxane is chosen more widely.

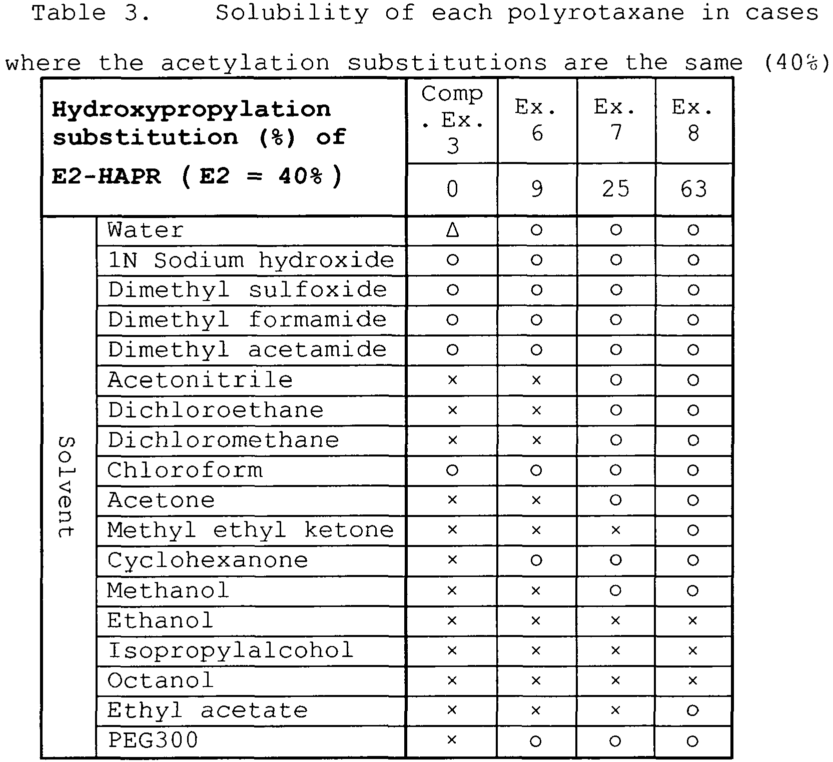

### <<A-3. Cases where First Substitution is

### 1,2-dihydroxypropylation>>

The solubility was compared between cases where the first substitution was 1,2-dihydroxypropylation and the substitution rates were constant (G = 42%). Specifically, regarding Example 10 and Comparative Example 2, the solubility was compared among various solvents. The substitution in comparative Example 2 was only with 1,2-dihydroxypropylation. The results are shown in Table 4. Marks "○", "Δ" and "×" in Table 4 have meanings same as that of A-1.

Table 4 shows that further acetylated polyrotaxane (Example 10) exhibits improved solubility to halogen-based solvents (dichloroethane, dichloromethane or chloroform) in comparison with the polyrotaxane that was not acetylated (Comparative Example 2).

The results show that the polyrotaxane according to the present invention exhibits improved solubility to various solvents. Further, as the result of improvement in the solubility, a solvent for dissolving the polyrotaxane is chosen more widely.

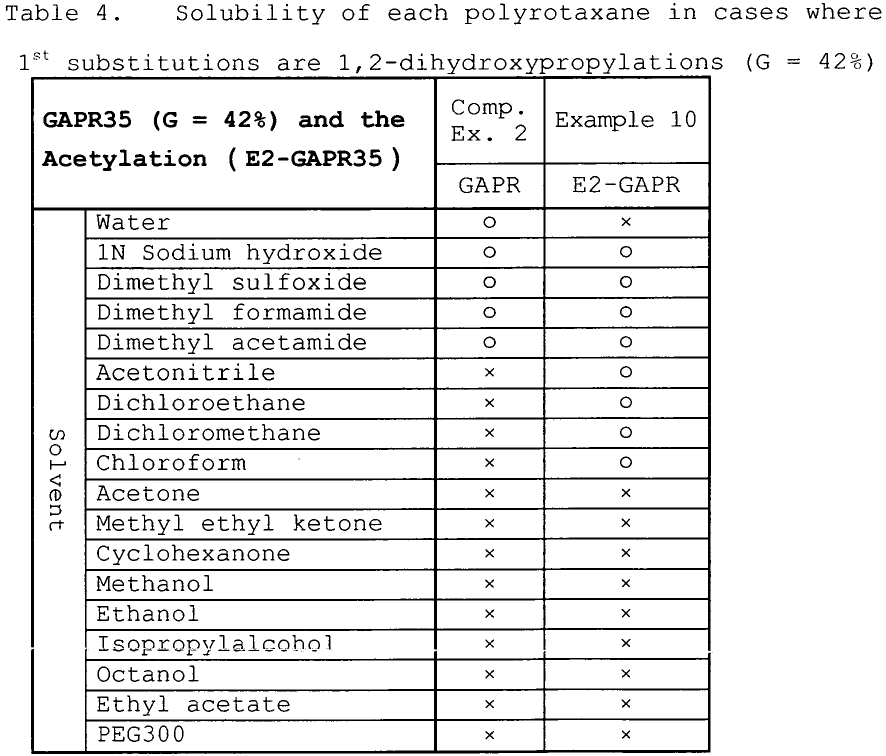

### Example 12:

In 0.460 ml of water, 40 mg of E2-HAPR (H = 48, E2 = 47) obtained in Example 4 was dissolved and the solution was charged in a capped glass tube having a diameter of 6.0 mm. A transition temperature from sol to gel was measured in a water bath provided with a thermometer. The minimum temperature where the gel did not fall when the glass tube was turned upside down was taken as a transition temperature. Thesol-gel transition temperature was 80°C.

### Example 13:

An experiment was carried out in a manner similar to Example 12, except that 50 mg of E2-HAPR (H = 48, E2 = 47) obtained in Example 4 was dissolved in 0.450 ml of water. The sol-gel transition temperature was 50°C.

### Example 14:

An experiment was carried out in a manner similar to Example 12, except that 75 mg of E2-HAPR (H = 48, E2 = 47) obtained in Example 4 was dissolved in 0.425 ml of water. The sol-gel transition temperature was 21°C.

### Example 15:

An experiment was carried out in a manner similar to Example 12, except that 75 mg of E2-HAPR (H = 48, E2 = 35) obtained in Example 3 was dissolved in 0.425 ml of water. The sol-gel transition temperature was 86°C.

### Example 16:

An experiment was carried out in a manner similar to Example 12, except that 100 mg of E2-HAPR (H = 48, E2 = 35) obtained in Example 3 was dissolved in 0.400 ml of water. The sol-gel transition temperature was 71°C.

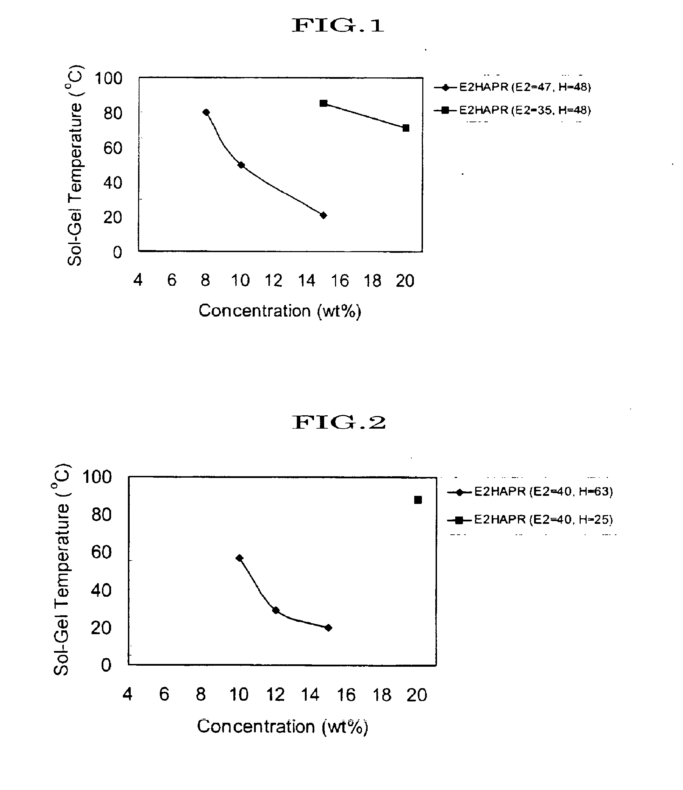

The sol-gel transition temperatures of Examples 12 to 16 are shown in Fig. 1. As shown in Fig. 1, when a concentration of polyrotaxane used is varied, the sol-gel transition temperature remarkably varies. Further, Fig. 1 shows that the sol-gel transition temperature varies as well by varying the kind of polyrotaxane used.

### Example 17:

An experiment was carried out in a manner similar to Example 12, except that 50 mg of E2-HAPR (H = 63, E2 = 40) obtained in Example 8 was dissolved in 0.450 ml of water. The sol-gel transition temperature was 57°C.

### Example 18:

An experiment was carried out in a manner similar to Example 12, except that 60 mg of E2-HAPR (H = 63, E2 = 40) obtained in Example 8 was dissolved in 0.440 ml of water. The sol-gel transition temperature was 29°C.

### Example 19:

An experiment was carried out in a manner similar to Example 12, except that 75 mg of E2-HAPR (H = 63, E2 = 40) obtained in Example 8 was dissolved in 0.425 ml of water. The sol-gel transition temperature was 20°C.

### Example 20:

An experiment was carried out in a manner similar to Example 12, except that 100 mg of E2-HAPR (H = 25, E2 = 40) obtained in Example 7 was dissolved in 0.400 ml of water. The sol-gel transition temperature was 88°C.

The sol-gel transition temperatures of Examples 17 to 20 are shown in Fig. 2. As shown in Fig. 2, when a concentration of polyrotaxane used is varied, the sol-gel transition temperature remarkably varies. Further, Fig. 2 shows that the sol-gel transition temperature varies as well by varying the kind of polyrotaxane used.

### Comparative Example 6:

An experiment was carried out in a manner similar to Example 12, except that 25 mg of MAPR (M = 95) obtained in Comparative Example 4 was dissolved in 0.475 ml of water. The sol-gel transition temperature was 28°C.

### Comparative Example 7:

An experiment was carried out in a manner similar to Example 12, except that 50 mg of MAPR (M = 95) obtained in Comparative Example 4 was dissolved in 0.450 ml of water. The sol-gel transition temperature was 20°C.

### Comparative Example 8:

An experiment was carried out in a manner similar to Example 12, except that 75 mg of MAPR (M = 95) obtained in Comparative Example 4 was dissolved in 0.425 ml of water. The sol-gel transition temperature was 16°C.

### Comparative Example 9:

An experiment was carried out in a manner similar to Example 12, except that 40 mg of MHAPR (H = 48, M = 98) obtained in Comparative Example 5 was dissolved in 0.460 ml of water. The sol-gel transition temperature was 51°C.

### Comparative Example 10:

An experiment was carried out in a manner similar to Example 12, except that 50 mg of MHAPR (H = 48, M = 98) obtained in Comparative Example 5 was dissolved in 0.450 ml of water. The sol-gel transition temperature was 44°C.

### Comparative Example 11:

An experiment was carried out in a manner similar to Example 12, except that 75 mg of MHAPR (H = 48, M = 98) obtained in Comparative Example 5 was dissolved in 0.425 ml of water. The sol-gel transition temperature was 39°C.

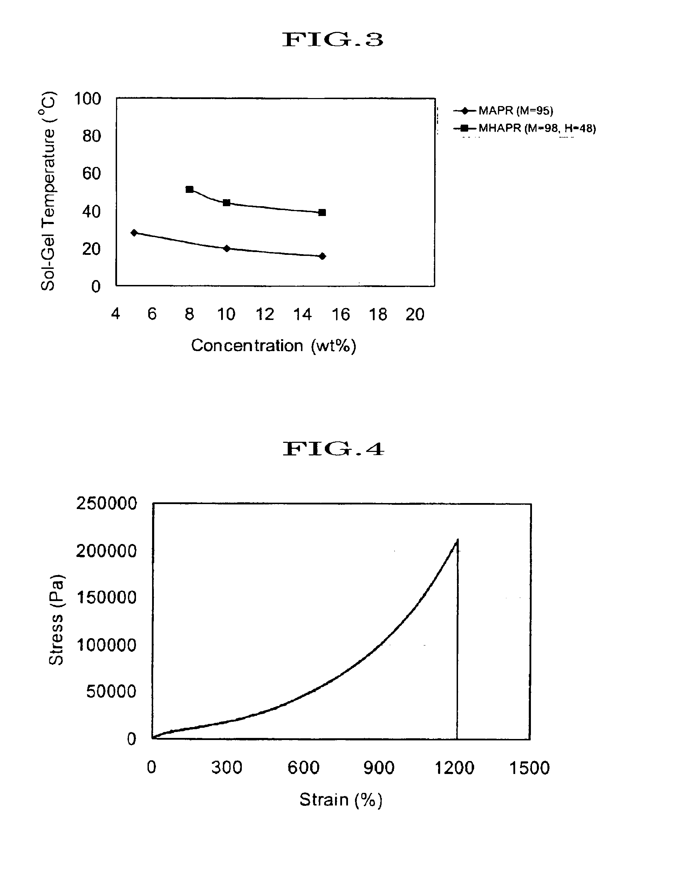

The sol-gel transition temperatures of Comparative Examples 6 to 11 are shown in Fig. 3. It is found that the concentration dependence of the sol-gel temperature is smaller in comparison with Fig. 1 or 2.

When Examples 12 to 20 and Comparative Examples 6 to 11 are compared, it is found that in the present invention, Examples 12 to 20, the sol-gel transition temperature varies largely depending on the concentration of polyrotaxane used. Further, Examples 12 to 20 show that the sol-gel transition temperatures largely vary depending on the kind of polyrotaxane used as well. The above things show that the polyrotaxanes according to the present invention can largely vary the sol-gel transition temperature, depending on the kind and/or concentration used. Accordingly, the present invention can provide a polyrotaxane or a crosslinked polyrotaxane of which sol-gel transition can be generated at a desired temperature.

### Example 21:

### <Chemically Crosslinked Body of E2-HAPR (H = 48, E2 = 47)>

100 mg of E2-HAPR obtained in Example 4 (H = 48, E2 = 47) was dissolved in 0.50 ml of dimethyl sulfoxide (DMSO). Thereto, 1.0 µl of hexamethylene diisocyanate was added, followed by reaction for 10 hours at 60°C, thereby to obtain crosslinked E2-HAPR. The crosslinked polyrotaxane was charged in water and subjected to water replacement, thereby to obtain transparent and elastic hydrogel.

The crosslinked E2-HAPR having a thickness of 0.5 mm was prepared and the transmittance thereof was measured in a visible region from 400 to 800 nm and found to be 99.0% of average transmittance.

Further, crosslinked E2-HAPR having a length of 20 mm, a width of 4 mm and a thickness of 0.5 mm was prepared and the viscoelasticity thereof was measured, thereby to obtain a stress-strain curve shown in Fig. 4. The Young's modulus was 18 kPa and an extension ratio was 1200%.

### Comparative Example 12:

### <Chemically Crosslinked Body of Unsubstituted Polyrotaxane APR>

APR, which was similar to Example 1, was prepared. 100 mg of the APR was dissolved in 0.50 ml of DMSO. Thereto, 1.6 µl of hexamethylene diisocyanate was added, followed by reaction at 60°C for 10 hr, thereby to obtain crosslinked APR. The crosslinked APR was immersed in water and subjected to water replacement, thereby to obtain a transparent and elastic hydrogel.

Crosslinked APR having a length of 20 mm, a width of 4 mm and a thickness of 0.5 mm was prepared and the viscoelasticity thereof was measured, thereby it was found that the Young's modulus is 456 kPa and an extension ratio was 790%. The obtained gel was not transparent (transmittance = 5%).

### Example 22:

### <Chemically Crosslinked Body of E2-HAPR (H = 25, E2 = 40)>

100 mg of E2-HAPR obtained in Example 7 (H = 25, E2 = 40) was dissolved in 0.50 ml of DMSO. Thereto, 1.6 µl of hexamethylene diisocyanate was added, followed by reaction for 10 hours at 60°C, thereby to obtain crosslinked E2-HAPR (H = 25, E2 = 40). The crosslinked E2-HAPR was added in water and subjected to water replacement, thereby to obtain transparent and elastic hydrogel.

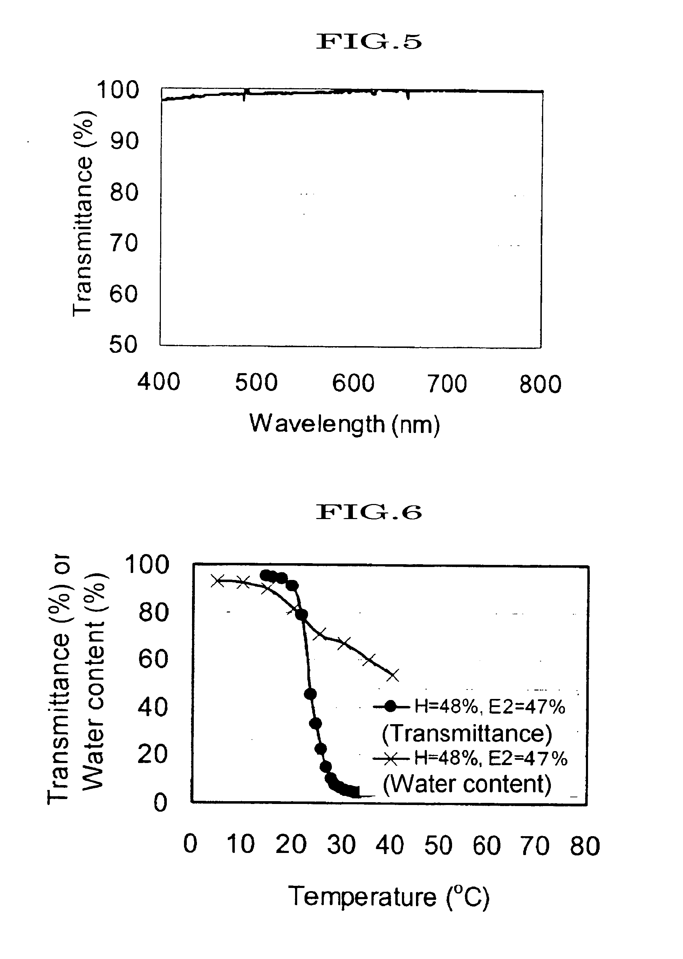

The crosslinked E2-HAPR having a thickness of 0. 5 mm was prepared and the transmittance thereof was measured in a visible region from 400 to 800 nm and found to be very high such as 99.8% of average transmittance (Fig. 5).

Further, crosslinked polyrotaxane having a length of 20 mm, a width of 4 mm and a thickness of 0.5 mm was prepared and when the viscoelasticity thereof was measured, the Young's modulus was found to be 24 kPa and an extension ratio to be 325%.

### Comparative Example 13:

### <Chemically Crosslinked Body of Hydroxypropylated Polyrotaxane HAPR (H = 48)>

100 mg of HAPR obtained in Comparative Example 1 (H = 48) was dissolved in 0.50 ml of DMSO. Thereto, 1.6 µl of hexamethylene diisocyanate was added, followed by reaction for 10 hours at 60°C, thereby to obtain crosslinked HAPR. The crosslinked HAPR was added in water and subjected to water replacement, and, thereby to obtain a transparent and elastic hydrogel.

The crosslinked HAPR having a thickness of 0.5 mm was prepared and the transmittance thereof was measured in a visible region from 400 to 800 nm and found to be 99% of average transmittance.

Further, crosslinked HAPR having a length of 20 mm, a width of 4 mm and a thickness of 0.5 mm was prepared and when the viscoelasticity thereof was measured, the Young's modulus was found to be 12 kPa and an extension ratio was found to be 90%.

### Example 23:

### <Chemically Crosslinked Body of E2-HAPR (H = 63, E2 = 40)>

100 mg of E2-HAPR obtained in Example 8 (H = 63, E2 = 40) was dissolved in 0.50 ml of DMSO. Thereto, 1.6 µl of hexamethylene diisocyanate was added, followed by reaction for 10 hours at 60°C, thereby to obtain crosslinked E2-HAPR (H = 63, E2 = 40). The crosslinked E2-HAPR was added in water and subjected to water replacement, thereby to obtain a transparent and elastic hydrogel.

The crosslinked E2-HAPR having a thickness of 0.5 mm was prepared and the transmittance thereof was measured in a visible region from 400 to 800 nm and found to be 97% of average transmittance.

Further, crosslinked polyrotaxane having a length of 20 mm, a width of 4 mm and a thickness of 0.5 mm was prepared and when the viscoelasticity thereof was measured, the Young's modulus was found to be 28 kPa and an extension ratio was found to be 450%.

**Table 5.**

| | Polyrotaxane | Average transmittance | Young' s modulus | Extension ratio |
|---|---|---|---|---|
| Ex. 21 | E2-HAPR (H=48, E2=47) | 99.0% | 18kPa | 1200% |
| Ex. 22 | E2-HAPR (H=25, E2=40) | 99.8% | 24kPa | 325% |
| Ex. 23 | E2-HAPR (H=63, E2=40) | 97% | 28kPa | 450% |
| Comp. Ex. 12 | APR | Opaque (5%) | 456kPa | 790% |
| Comp. Ex. 13 | HAPR (H=48) | 99% | 28kPa | 90% |

The results of Examples 21 to 23 and Comparative Examples 12 and 13 are summarized in Table 5.

Table 5 shows in comparison of Examples 21 to 23 with Comparative Examples 12 and 13 that Examples 21 to 23 can provide the crosslinked polyrotaxane having high Young' s modulus, high extension ratio and high transmittance simultaneously, that is, three parameters of high Young's modulus, high extension ratio and high transmittance can be achieved. On the other hand, Comparative Example 12 shows that although the Young's modulus and extension ratio are high, the transmittance is poor. Comparative Example 13 shows that although the transmittance and Young's modulus are high, an extension ratio is poor. Accordingly, it is found that the present invention can provide acrosslinkedpolyrotaxane that can simultaneously achieve three of high Young's modulus, high extension ratio and high transmittance.

### Example 24:

### <Preparation of Crosslinked E2-HAPR (H = 48, E2 = 47) in Methyl Ethyl Ketone>

100 mg of E2-HAPR obtained in Example 4 (H = 48, E2 = 47) was dissolved in 0.50 ml of methyl ethyl ketone. Thereto, 8 µl of hexamethylene diisocyanate was added, followed by reaction for 5 hours at 40°C. As the result, it was confirmed that a transparent and elastic crosslinked E2-HAPR (H = 48, E2 = 47) was obtained.

### Example 25:

### <Preparation of Crosslinked E2-HAPR (H = 48, E2 = 47) in Chloroform>

100 mg of E2-HAPR obtained in Example 4 (H = 48, E2 = 47) was dissolved in 0.50 ml of chloroform. Thereto, 8 µl of hexamethylene diisocyanate was added, followed by reaction for 24 hours at room temperature (25°C). As the result, it was confirmed that a transparent and elastic crosslinked E2-HAPR (H = 48, E2 = 47) was obtained.

Examples 24 and 25 show that the polyrotaxane according to the present invention can provide a crosslinked polyrotaxane which is crosslinked in a solvent different from a conventional solvent (such as water or DMSO), since the polyrotaxane according to the present invention has high solubility in various solvents.

### Example 26:

### <Preparation of Mixed, Crosslinked Body between E2-HAPR (H = 48, E2 = 47) and poly(4-vinylphenol) in Methyl Ethyl Ketone>

50 mg of E2-HAPR obtained in Example 4 (H = 48, E2 = 47) and 50 mg of poly(4-vinylphenol) were dissolved in 0.50 ml of methyl ethyl ketone. Thereto, 8 µl of hexamethylene diisocyanate was added, followed by reaction for 5 hours at 40°C. As the result, it was confirmed that a transparent and elastic mixed, crosslinked material of E2-HAPR (H = 48, E2 = 47) and poly(4-vinylphenol) was obtained.

Poly(4-vinylphenol) is difficult to dissolve in a conventionalpolyrotaxane-soluble solvent (water or DMSO); and thus, it had been difficult to prepare a crosslinked body of poly (4-vinylphenol) mixed with a polyrotaxane. However, since the polyrotaxane according to the present invention exhibits improved solubility in various solvents, polymers for preparing a mixed, crosslinked body are chosen widely. Specifically, the present Example could provide a mixed, crosslinked body with poly(4-vinylphenol).

### Example 27:

### <Evaluation of Optical property and Swelling Property of Crosslinked E2-HAPR (H = 48, E2 = 47)>

A crosslinked E2-HAPR (H = 48, E2 = 47) was prepared in a manner similar to Example 21. The crosslinked E2-HAPR (H = 48, E2 = 47) was added in water and subjected to undergo water replacement, thereby to obtain a hydrogel.

The transmittance at a wavelength in the visible light region was measured by varying a temperature of the resulting hydrogel. The results are shown in Fig. 6.

Fig. 6 shows that the transmittance remarkably responds to temperature variation. A hydrogel having a long side of 20 mm, a short side of 4 mm and a thickness of 0.5 mm at a temperature of 25°C swelled to a hydrogel having a long side of 32 mm, a short side of 7 mm and a thickness of 0.8 mm when the temperature was varied to 5°C. When the temperature was returned to 25°C, the hydrogel shrank to a shape having a long side of 20 mm, a short side of 4 mm and a thickness of 0.5 mm.

A content of a solvent contained in a crosslinked body (water content = weight of hydrogel/weight of dry gel) was investigated to normalize a volume change during swelling and contraction owing to a solvent of the crosslinked body caused by temperature variation. The results thereof are shown as well in Fig. 6.

The transmittance variation caused by temperature variation is remarkable as shown in Fig. 6; accordingly, a change between a transparent state and an opaque state of a material occurs rapidly. In the case of this Example, a transition temperature was controlled, for instance, between a body temperature (substantially 36°C) and room temperature (substantially 22°C); accordingly, a material having the following function was realized; when a human body comes into contact with a crosslinked body (since a temperature of the crosslinked body becomes a temperature substantially equal to a body temperature), the crosslinked body becomes opaque and when the human body disengages (since a temperature of the crosslinked body returns to room temperature) the crosslinked body returns to the transparent state.

Accordingly, a material having such functions is applied to materials such as cosmetic materials, that is, materials that are able to effectively control a release of an effective ingredient at a body temperature.

Further, by controlling the transition temperature variously, the crosslinked body according to the present invention may be used in DDS (such as control of sustained release of drugs caused by temperature variation and protection of wound site by making use of volume contraction caused by the temperature variation), above-mentioned cosmetics, biosensors and actuators.

### Example 28:

### <Preparation of Crosslinked E2-HAPR (H = 48, E2 = 35) Hydrogel>

A crosslinked E2-HAPR (H = 48, E2 = 35) was prepared in a manner similar to Example 21, except that E2-HAPR (H = 48, E2 = 35) obtained in Example 3 was used. The crosslinked E2-HAPR (H= 48, E2 = 35) was added in water to subject to water replacement, thereby to obtain a hydrogel.

### <Evaluation of Optical property and Swelling Property of Crosslinked E2-HAPR (H = 48, E2 = 35) Hydrogel>

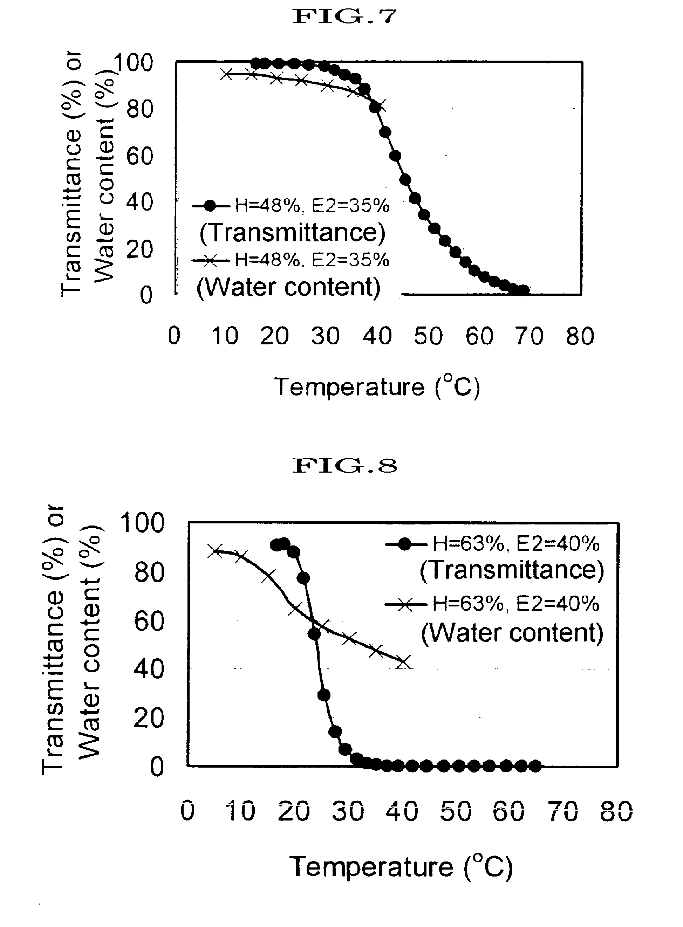

The optical property, that is, the transmittance variation in the visible light region accompanying a temperature variation of the resulting hydrogel was measured in a manner similar to Example 27. Results thereof are shown in Fig. 7. Fig. 7 shows that the transmittance remarkably responds to the temperature variation.

The temperature dependence of the water content contained in the gel (water content = weight of hydrogel/weight of dry gel) as well is shown in Fig. 7.

The crosslinked body according the present Example as well was found to undergo the reversible volume variation in a manner similar to Example 27. It was found that, although the volume variation according the present Example is milder than Example 27, the transmittance as well reversibly varies with the temperature in a manner similar to Example 27. The milder variation may exert an advantage in that a milder release of an effective ingredient is realized in materials such as the cosmetics as described above.

### Example 29:

### <Evaluation of Optical property and Swelling Property of Crosslinked E2-HAPR (H = 63, E2 = 40) Hydrogel>

A crosslinked E2-HAPR (H = 63, E2 = 40) was prepared in a manner similar to Example 23. The crosslinked E2-HAPR (H = 63, E2 = 40) was added in water and subjected to water replacement, thereby to obtain a hydrogel.

The transmittance variation in the visible light region was measured by varying a temperature of the resulting hydrogel. Results thereof are shown in Fig. 8. Fig. 8 shows that the transmittance remarkably responds to the temperature variation. The temperature dependence of the water content contained in the gel (water content = weight of hydrogel/weight of dry gel) as well is shown in Fig. 8.

The crosslinked body according the present Example as well was found to undergo the reversible volume variation in a manner similar to Example 27. It was found that the transmittance as well varies rapidly and reversibly with the temperature in a manner similar to Example 27.

### Example 30:

### <Evaluation of Optical property and Swelling Property of Crosslinked E2-HAPR (H = 25, E2 = 40) Hydrogel>

A crosslinked E2-HAPR (H = 25, E2 = 40) was prepared in a manner similar to Example 22. The crosslinked E2-HAPR (H = 25, E2 = 40) was added in water and subjected to water replacement, thereby to obtain a hydrogel.

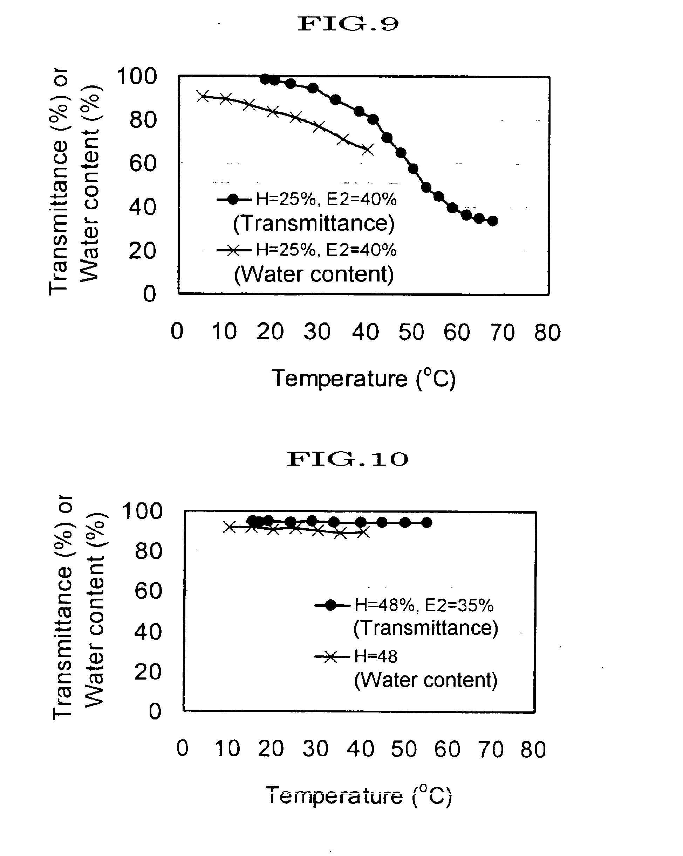

The transmittance variation in the visible light region was measured by varying a temperature of the resulting hydrogel. Results thereof are shown in Fig. 9. Fig. 9 shows that the transmittance remarkably responds to the temperature variation. The temperature dependence of the water content contained in the gel (water content = weight of hydrogel/weight of dry gel) as well is shown in Fig. 9.

The crosslinked body according the present Example as well was found to undergo the reversible volume variation in a manner similar to Example 28. It was found that the transmittance, although milder than Example 27, varies reversibly with the temperature in a manner similar to Example 28.

### Comparative Example 14:

### <Evaluation of Optical property and Swelling Property of Crosslinked HAPR Hydrogel>

A crosslinked HAPR (H = 48%) was prepared in a manner similar to Comparative Example 13. The crosslinked HAPR (H = 48%) was added in water and subjected to water replacement, thereby to obtain a hydrogel.

The transmittance variation in the visible light region was measured by varying a temperature of the resulting hydrogel. Results thereof are shown in Fig. 10. The temperature dependence of the water content contained in the gel (water content = weight of hydrogel/weight of dry gel) as well is shown in Fig. 10.

Fig. 10 shows that in the hydrogel of Comparative Example 14, the transmittance does hardly exhibit the temperature dependence. Further, it is also found that the water content variation (= volume change) in the gel does hardly exhibit the temperature dependence.

Examples 27 to 30 and Comparative Example 14 show that the crosslinkedbodies according to the present invention exhibit an ability to respond to an external stimulus. In particular, it is found that the crosslinked bodies according to the present invention exhibit an ability to respond to an external stimulus where, when the external stimulus is heat, the transmittance and/or volume of the crosslinked bodies vary, depending on the heat.

## Claims

1. A polyrotaxane comprising a pseudopolyrotaxane, which has a linear molecule and a cyclic molecule (s) in which the linear molecule is included in a cavity (cavities) of the cyclic molecule(s) in a skewered manner, and capping groups, each of which locates at each end of the pseudopolyrotaxane in order to prevent the dissociation of the cyclic molecule(s),
wherein the cyclic molecule(s) comprises a functional group represented by following formula I, and at least one functional group selected from the group consisting of following formulae II-1 to II-6, wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms,
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms, and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom; and
R₆ represents a photoreactive group) :

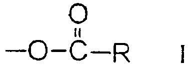

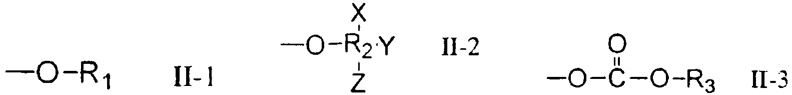

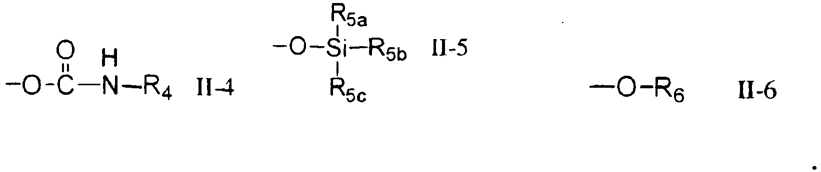

2. The polyrotaxane according to claim 1, wherein the cyclic molecule is a cyclic molecule having a hydroxy group(s), and a part of the hydroxy group (s) is substituted with the functional group represented by formula I and the at least one functional group selected from the group consisting of following formulae II-1 to II-6.

3. The polyrotaxane according to claim 1 or 2, wherein the polyrotaxane has an ability to respond reversibly to an external stimulus, which reversibly transforms the polyrotaxane from an uncrosslinked state to a crosslinked state, or from a crosslinked state to an uncrosslinked state, depending on the presence or the absence of the external stimulus.

4. The polyrotaxane according to claim 3, wherein the external stimulus is heat.

5. The polyrotaxane according to claim 3 or 4, wherein the external stimulus is heat, and a temperature range in which the crosslinked polyrotaxane transforms from an uncrosslinked state to a crosslinked state, or from a crosslinked state to an uncrosslinked state, is 5 to 90°C.

6. The polyrotaxane according to any one of claims 2 to 5, wherein the cyclic molecule having the hydroxy group(s) is selected from the group consisting of α-cyclodextrin, β-cyclodextrin and γ-cyclodextrin.

7. The polyrotaxane according to any one of claims 2 to 6, wherein the number of the functional group represented by formula I is 0.05 to 0.9, and the number of the functional group represented by formula II is 0.05 to 0.9, where the number of the hydroxy groups of the cyclic molecule is normalized to be 1.

8. The polyrotaxane according to any one of claims 1 to 7, wherein the linear molecule is selected from the group consisting of polyethylene glycol, polyisoprene, polyisobutylene, polybutadiene, polypropylene glycol, polytetrahydrofuran, polydimethylsiloxane, polyethylene and polypropylene.

9. The polyrotaxane according to any one of claims 1 to 8, wherein the linear molecule has a molecular weight of 10,000 or more.

10. The polyrotaxane according to any one of claims 1 to 9, wherein the capping group is selected from the group consisting of dinitrophenyl groups; cyclodextrins; adamantane groups; trityl groups; fluoresceins; pyrenes; substituted benzenes; polycyclic aromatics which may be substituted; and steroids.

11. The polyrotaxane according to any one of claims 1 to 10, wherein the cyclic molecule is derived from α-cyclodextrin, and the linear molecule is polyethylene glycol.

12. The polyrotaxane according to any one of claims 1 to 11, wherein the linear molecule has the cyclic molecule included in a skewered manner at an amount of 0.001 to 0.6 of a maximum inclusion amount, which is defined as an amount at which the cyclic molecules can be included at maximum when the linear molecule has the cyclic molecules included in a skewered manner, and the amount at maximum is normalized to be 1.

13. A material comprising the polyrotaxane according to any one of claims 1 to 12.

14. A method for producing a polyrotaxane comprising a pseudopolyrotaxane, which has a linear molecule and a cyclic molecule (s) in which the linear molecule is included in a cavity (cavities) of the cyclic molecule (s) in a skewered manner, and capping groups, each of which locates at each end of the pseudopolyrotaxane in order to prevent the dissociation of the cyclic molecule(s),
wherein a part of hydroxy groups of the cyclic molecule is substituted with a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms,
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms, and R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):
which comprises the steps of:
1) preparing the pseudopolyrotaxane;
2) capping both ends of the resulting pseudopolyrotaxane with the capping groups, to prepare the polyrotaxane; and
3) substituting a part of hydroxy groups of the cyclic molecule with the functional group;
wherein the step of substituting is conducted
A) before the step 1) of preparing the pseudopolyrotaxane,
and/or
B) after the step 2) of capping to prepare the polyrotaxane:

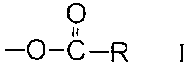

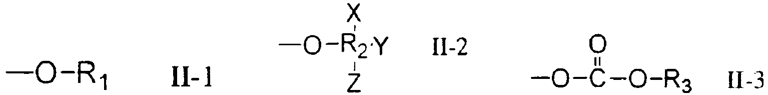

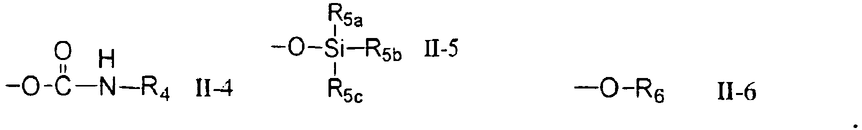

15. The method according to claim 14, wherein the step of substituting is conducted after the step 2) of capping to prepare the polyrotaxane.

16. The method according to claim 14 or 15, wherein in the step of substituting with the functional group, a step of introducing the functional group represented by formula I is carried out
X) before a step of introducing any one of functional groups represented by formula II;
Y) after the step of introducing any one of functional groups represented by formula II; or
Z) simultaneously with the step of introducing any one of functional groups represented by formula II.

17. The method according to any one of claims 14 to 16, wherein in the step of substituting with the functional group, a step of introducing the functional group represented by formula I is carried out Y) after a step of introducing any one of functional groups represented by formula II.

18. A crosslinked polyrotaxane comprising at least two molecules of polyrotaxane, wherein the at least two molecules of polyrotaxane are crosslinked via physical bond,
wherein the polyrotaxane comprises a pseudopolyrotaxane, which has a linear molecule and a cyclic molecule(s) in which the linear molecule is included in a cavity (cavities) of the cyclic molecule(s) in a skewered manner, and capping groups, each of which locates at each end of the pseudopolyrotaxane in order to prevent the dissociation of the cyclic molecule(s),
wherein the cyclic molecule(s) comprises a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):

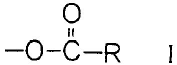

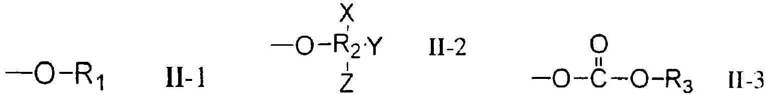

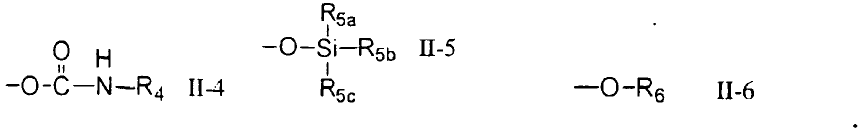

19. The crosslinked polyrotaxane according to claim 18, wherein the cyclic molecule is a cyclic molecule having a hydroxy group (s) , and a part of the hydroxy groups is substituted with the functional group represented by formula I and the at least one functional group selected from the group consisting of the formulae II-1 to II-6.

20. The crosslinked polyrotaxane according to claim 18 or 19, wherein the polyrotaxane has an ability to respond reversibly to an external stimulus, which reversibly transforms the polyrotaxane from an uncrosslinked state to a crosslinked state, or from a crosslinked state to an uncrosslinked state, depending on the presence or the absence of the external stimulus.

21. The crosslinked polyrotaxane according to claim 20, wherein the external stimulus is heat.

22. The crosslinked polyrotaxane according to claim 20 or 21, wherein the external stimulus is heat, and a temperature range in which the crosslinked polyrotaxane transforms from an uncrosslinked state to a crosslinked state, or from a crosslinked state to an uncrosslinked state, is 5 to 90°C.

23. The crosslinked polyrotaxane according to any one of claims 19 to 22, wherein the cyclic molecule having the hydroxy group (s) is selected from the group consisting of α-cyclodextrin, β-cyclodextrin and γ-cyclodextrin.

24. The crosslinked polyrotaxane according to any one of claims 19 to 23, wherein the number of the functional group represented by formula I is 0.05 to 0.9, and the number of the functional group represented by formula II is 0.05 to 0.9, where the number of the hydroxy groups of the cyclic molecule is normalized to be 1.

25. The crosslinked polyrotaxane according to any one of claims 18 to 24, wherein the linear molecule is selected from the group consisting of
polyethylene glycol, polyisoprene, polyisobutylene, polybutadiene, polypropylene glycol, polytetrahydrofuran, polydimethylsiloxane, polyethylene and polypropylene.

26. The crosslinked polyrotaxane according to any one of claims 18 to 25, wherein the linear molecule has a molecular weight of 10,000 or more.

27. The crosslinked polyrotaxane according to any one of claims 18 to 26, wherein the capping group is selected from the group consisting of dinitrophenyl groups; cyclodextrins; adamantane groups; trityl groups; fluoresceins; pyrenes; substituted benzenes; polycyclic aromatics which may be substituted; and steroids.

28. The crosslinked polyrotaxane according to any one of claims 18 to 27, wherein the cyclic molecule is derived from α-cyclodextrin, and the linear molecule is polyethylene glycol.

29. The crosslinked polyrotaxane according to any one of claims 18 to 28, wherein the linear molecule has the cyclic molecule included in a skewered manner at an amount of 0.001 to 0.6 of a maximum inclusion amount, which is defined as an amount at which the cyclic molecules can be included at maximum when the linear molecule has the cyclic molecules included in a skewered manner, and the amount at maximum is normalized to be 1.

30. A material comprising the crosslinked polyrotaxane according to any one of claims 18 to 29.

31. A method for producing a crosslinked polyrotaxane, which comprises at least two molecules of polyrotaxane, wherein the at least two molecules of polyrotaxane are crosslinked via physical bond,
wherein the polyrotaxane comprises a pseudopolyrotaxane, which has a linear molecule and a cyclic molecule(s) in which the linear molecule is included in a cavity (cavities) of the cyclic molecule(s) in a skewered manner, and capping groups, each of which locates at each end of the pseudopolyrotaxane in order to prevent the dissociation of the cyclic molecule(s),
wherein apart of hydroxy groups of the cyclic molecule (s) is substituted with a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₆ represents a photoreactive group):
the method comprising the steps of:
1) preparing the pseudopolyrotaxane;
2) capping both ends of the resulting pseudopolyrotaxane with a capping group to prepare a polyrotaxane; and
3) substituting a part of hydroxy groups of the cyclic molecule with the functional group represented by formula I, and at least one functional group selected from the group consisting of formulae II-1 to II-6;
thereby to obtain the polyrotaxane;
4) dissolving at least two molecules of the resulting polyrotaxane in a solvent; and
5) imparting an external stimulus to the at least two molecules of polyrotaxane in the solvent to physically crosslink the at least two molecules of polyrotaxane via physical bond,
wherein 3) the step of substituting is carried out
A) before 1) the step of preparing the pseudopolyrotaxane, and/or
B) after 2) the step of capping to prepare a polyrotaxane:

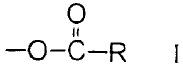

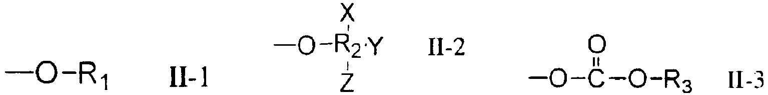

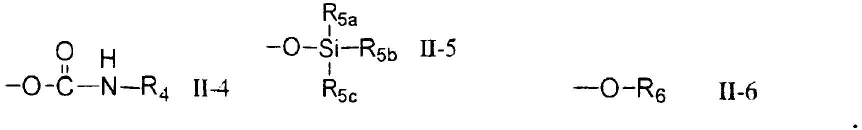

32. The method according to claim 31, wherein the step of substituting with the functional group is carried out after 2) the step of capping to prepare the polyrotaxane.

33. The method according to claim 31 or 32, wherein in the step of substituting with the functional group, a step of introducing the functional group represented by formula I is carried out
X) before a step of introducing any one of functional groups represented by formula II;
Y) after a step of introducing any one of functional groups represented by formula II; or
Z) simultaneously with the step of introducing any one of functional groups represented by formula II.

34. The method according to any one of claims 31 to 33, wherein in the step of substituting with the functional group, a step of introducing the_functional group represented by formula I is carried out Y) after a step of introducing any one of functional groups represented by formula II.

35. The method according to any one of claims 31 to 34, wherein the solvent is a hydrophilic solvent.

36. A crosslinked polyrotaxane comprising a first polymer and a first polyrotaxane, wherein all or a part of the first polymer and all or a part of the first polyrotaxane are crosslinked,
wherein the first polyrotaxane comprises a first pseudopolyrotaxane, which has a first linear molecule and a first cyclic molecule (s) in which the first linear molecule is included in a cavity (cavities) of the first cyclic molecule(s) in a skewered manner, and first capping groups, each of which locates at each end of the first pseudopolyrotaxane in order to prevent the dissociation of the first cyclic molecule(s),
wherein the first cyclic molecule(s) of the first polyrotaxane comprises a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, R₆ represents a photoreactive group) :

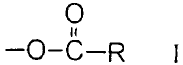

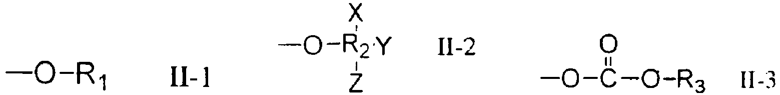

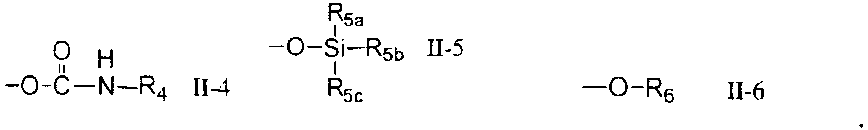

37. The crosslinked polyrotaxane according to claim 36, wherein the first polymer is a second polyrotaxane, wherein the second polyrotaxane comprises a second pseudopolyrotaxane, which has a second linear molecule and a second cyclic molecule (s) in which the second linear molecule is included in a cavity (cavities) of the second cyclic molecule(s) in a skewered manner, and second capping groups, each of which locates at each end of the second pseudopolyrotaxane in order to prevent the dissociation of the second cyclic molecule(s),
wherein the second cyclic molecule(s) of the second polyrotaxane comprises a functional group represented by following formula I', and at least one functional group selected from the group consisting of following formulae II'-1 to II'-6,
wherein R', R₁₁, R₁₃ and R₁₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R_{15a'} R_{15b} and R_{15c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₁₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, R₁₆ represents a photoreactive group) :

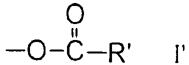

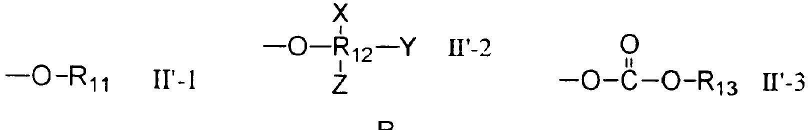

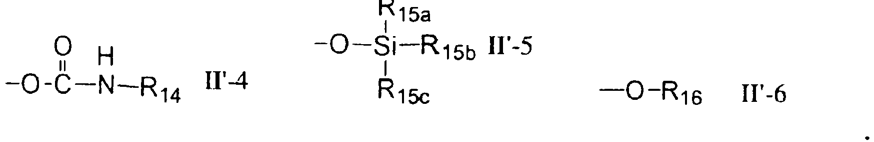

38. The crosslinked polyrotaxane according to claim 36 or 37, wherein the cyclic molecule is a cyclic molecule having a hydroxy group(s), and a part of the hydroxy group(s) is substituted with the functional group represented by formula I and the at least one functional group selected from the group consisting of the formulae II-1 to II-6.

39. The crosslinked polyrotaxane according to any one of claims 36 to 38, wherein the crosslinked polyrotaxane has the transmittance of 90%/mm or more in a wavelength from 400 to 800 nm.

40. The crosslinked polyrotaxane according to any one of claims 36 to 39, wherein the crosslinked polyrotaxane has an extension ratio in the range of 100 to 1500%.

41. The crosslinked polyrotaxane according to any one of claims 36 to 40, wherein the crosslinked polyrotaxane has the transmittance of 90%/mm or more in a wavelength from 400 to 800 nm, and an extension ratio of 300% or more.

42. The crosslinked polyrotaxane according to any one of claims 36 to 41, wherein the crosslinked polyrotaxane reversibly varies in an optical property and/or swelling-contraction characteristics, depending on the presence or the absence of the external stimulus.

43. The crosslinked polyrotaxane according to claim 42, wherein the external stimulus is heat, and the crosslinked polyrotaxane reversibly varies in the optical property and/or the swelling-contraction characteristics in a temperature range of from 5 to 90°C.

44. The crosslinked polyrotaxane according to claim 42 or 43, wherein the optical property is transparence of the crosslinked polyrotaxane.

45. The crosslinked polyrotaxane according to claim 42 or 43, wherein the swelling-contraction characteristics is a change of an amount of a contained solvent caused by absorption and/or release of the solvent by the crosslinked polyrotaxane.

46. The crosslinked polyrotaxane according to any one of claims 38 to 45, wherein the first and/or second cyclic molecule having the hydroxy group (s) is selected from the group consisting of α-cyclodextrin, β-cyclodextrin and γ-cyclodextrin.

47. The crosslinked polyrotaxane according to any one of claims 36 to 46, wherein the number of the functional group represented by formula I is 0.05 to 0.9, and the number of the functional group represented by formula II is 0.05 to 0.9, where the number of the hydroxy groups of the first and/or second cyclic molecule is normalized to be 1.

48. The crosslinked polyrotaxane according to any one of claims 36 to 47, wherein the first and/or second linear molecule is selected from the group consisting of polyethylene glycol, polyisoprene, polyisobutylene, polybutadiene, polypropylene glycol, polytetrahydrofuran, polydimethylsiloxane, polyethylene and polypropylene.

49. The crosslinked polyrotaxane according to any one of claims 36 to 48, wherein the first and/or second linear molecule has a molecular weight of 10,000 or more.

50. The crosslinked polyrotaxane according to any one of claims 36 to 49, wherein the first and/or second capping group is selected from the group consisting of dinitrophenyl groups; cyclodextrins; adamantane groups; trityl groups; fluoresceins; pyrenes; substituted benzenes; polycyclic aromatics which may be substituted; and steroids.

51. The crosslinked polyrotaxane according to any one of claims 36 to 50, wherein the first and/or second cyclic molecule is derived from α-cyclodextrin, and the first and/or second linear molecule is polyethylene glycol.

52. The crosslinked polyrotaxane according to any one of claims 36 to 51, wherein the first and/or second linear molecule has the first and/or second cyclic molecule included in a skewered manner at an amount of 0.001 to 0.6 of a maximum inclusion amount, which is defined as an amount at which the first and/or second cyclic molecules can be included at maximum when the first and/or second linear molecule has the first and/or second cyclic molecules included in a skewered manner, and the amount at maximum is normalized to be 1.

53. A material comprising the crosslinked polyrotaxane according to any one of claims 36 to 52.

54. A method for producing a crosslinked polyrotaxane comprising a first polymer and a first polyrotaxane, wherein all or a part of the first polymer and all or a part of the first polyrotaxane are crosslinked,
wherein the first polyrotaxane comprises a first pseudopolyrotaxane, which has a first linear molecule and a first cyclic molecule (s) in which the first linear molecule is included in a cavity (cavities) of the first cyclic molecule(s) in a skewered manner, and first capping groups, each of which locates at each end of the first pseudopolyrotaxane in order to prevent the dissociation of the first cyclic molecule(s),
wherein a part of hydroxy groups of the first cyclic molecule is substituted with a functional group represented by following formula I, and at least one functional group selected from the group consisting of following formulae II-1 to II-6,
wherein R, R₁, R₃ and R₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₅ₐ, R_{5b} and R_{5c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, R₆ represents a photoreactive group) :
the method comprising the steps of:
1) preparing the first pseudopolyrotaxane;
2) capping both ends of the resulting first pseudopolyrotaxane with a first capping group to prepare a first polyrotaxane; and
3) substituting a part of hydroxy groups of the first cyclic molecule with the functional group represented by formula I, and at least one functional group selected from the group consisting of formulae II-1 to II-6;
thereby to obtain the first polyrotaxane; and
4) chemically crosslinking the resulting first polyrotaxane and a first polymer;
wherein 3) the step of substituting is carried out
A) before 1) the step of preparing the first pseudopolyrotaxane, and/or
B) after 2) the step of capping to prepare a first polyrotaxane; and
4) the step of chemically crosslinking is carried out by
G) a crosslinking reaction through addition of a crosslinking agent, or
H) a photo-crosslinking reaction where a photo-reactive group contained in the first polyrotaxane is irradiated with light:

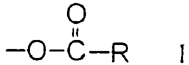

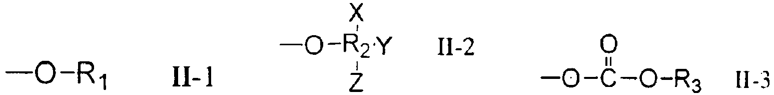

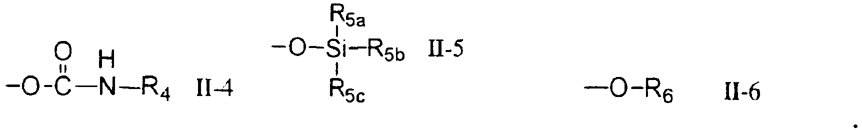

55. The method according to claims 54, wherein the first polymer is a secondpolyrotaxane, wherein the secondpolyrotaxane comprises a secondpseudopolyrotaxane, which has a second linear molecule and a second cyclic molecule(s) in which the second linear molecule is included in a cavity (cavities) of the second cyclic molecule(s) in a skewered manner, and second capping groups, each of which locates at each end of the second pseudopolyrotaxane in order to prevent the dissociation of the second cyclic molecule(s),
wherein a part of hydroxy groups of the second cyclic molecule is substituted with a functional group represented by following formula I', and at least one functional group selected from the group consisting of following formulae II'-1 to II'-6,
wherein R', R₁₁, R₁₃ and R₁₄ each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms;
R₁₅ₐ, R_{15b} and R_{15c} each independently represents a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms or a cyclic alkyl thioether group having 2 to 12 carbon atoms; and
R₁₂ represents a substitution group obtained by removing three hydrogen atoms from a group selected from the group consisting of a linear or branched alkyl group having 1 to 12 carbon atoms, a linear or branched alkyl group having 2 to 12 carbon atoms containing at least one ether group, a cyclic alkyl group having 3 to 12 carbon atoms, a cyclic alkyl ether group having 2 to 12 carbon atoms and a cyclic alkyl thioether group having 2 to 12 carbon atoms, wherein at least one of X to Z is a group selected from the group consisting of a hydroxyl group, a NH₃ group and a SH group, the remaining X to Z are a hydrogen atom, and
R₁₆ represents a photoreactive group); and
the second polyrotaxane is obtained by
1') preparing the second pseudopolyrotaxane;
2') capping both ends of the resulting second pseudopolyrotaxane with a second capping group to prepare a second polyrotaxane; and
3') substituting a part of hydroxy groups of the second cyclic molecule with the functional group represented by formula I', and at least one functional group selected from the group consisting of formulae II'-1 to II'-6:

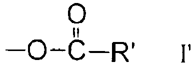

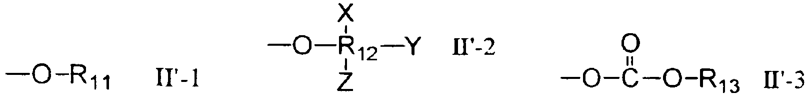

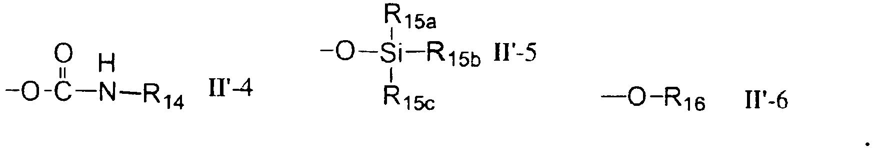

56. The method according to claim 54 or 55, wherein 4) the step of chemically crosslinking is carried out by G) the crosslinking reaction through addition of a crosslinking agent, and the crosslinking agent is selected from the group consisting of cyanuric chloride, trimesoyl chloride, terephthaloyl chloride, epichlorohydrin, dibromobenzene, glutaraldehyde, aliphatic polyfunctional isocyanate, aromatic polyfunctional isocyanate, tolylene diisocyanate, hexamethylenediisocyanate, divinyl sulfone, 1,1'-carbonyldiimidazole, alkoxysilanes and derivatives thereof, and photo-crosslinking reaction initiators.

57. The method according to any one of claims 54 to 56, wherein the step of substituting with the functional group is carried out after 2) and/or 2') the step of capping to prepare the first and/or second polyrotaxane.

58. The method according to any one of claims 54 to 57, wherein in the step of substituting with the functional group, a step of introducing the functional group represented by formula I or I' is carried out
X) before a step of introducing any one of functional groups represented by formula II or II';
Y) after a step of introducing any one of functional groups represented by formula II or II'; or
Z) simultaneously with the step of introducing any one of functional groups represented by formula II or II'.

59. The method according to any one of claims 55 to 58, wherein in the step of substituting with the functional group, a step of introducing the functional group represented by formula I or I' is carried out Y) after a step of introducing any one of functional groups represented by formula II or II'.

## Patentansprüche

1. Polyrotaxan, umfassend ein Pseudopolyrotaxan, das ein lineares Molekül und ein cyclisches Molekül (cyclische Moleküle) aufweist, worin das lineare Molekül in der Kavität (den Kavitäten) des darauf aufgereihten cyclischen Moleküls (Moleküle) eingeschlossen ist, und Endgruppen, die jeweils an den Enden des Pseudopolyrotaxans angeordnet sind, um die Dissoziation des cyclischen Moleküls (Moleküle) zu verhindern,
wobei das cyclische Molekül (Moleküle) eine funktionelle Gruppe der folgenden Formel I und zumindest eine funktionelle Gruppe ausgewählt unter den folgenden Formeln II-1 bis II-6, einschließt, worin R, R₁, R₃ und R₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₅ₐ, R_{5b} und R_{5c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und
R₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₆ für eine fotoreaktive Gruppe steht

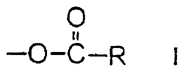

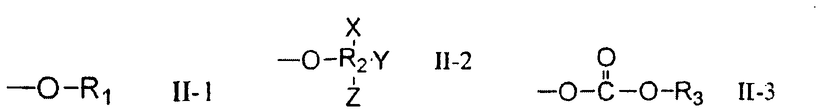

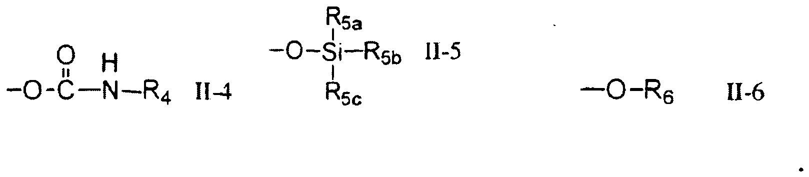

2. Polyrotaxan nach Anspruch 1, wobei das cyclische Molekül ein cyclisches Molekül ist, das eine Hydroxylgruppe(n) aufweist, und ein Teil der Hydroxylgruppe(n) durch die funktionelle Gruppe der Formel I und durch zumindest eine der unter den folgenden Formeln II-1 bis II-6 ausgewählten funktionellen Gruppen, substituiert ist.

3. Polyrotaxan nach Anspruch 1 oder 2, wobei das Polyrotaxan in der Lage ist, reversibel auf einen externen Stimulus zu reagieren, der das Polyrotaxan in Abhängigkeit von der Gegenwart oder vom Fehlen des externen Stimulus reversibel von einem nicht vernetzten Zustand in einen vernetzten Zustand überführt, oder von einem vernetzten Zustand in einen nicht vernetzten Zustand überführt.

4. Polyrotaxan nach Anspruch 3, wobei der externe Stimulus Wärme ist.

5. Polyrotaxan nach Anspruch 3 oder 4, wobei der externe Stimulus Wärme ist, und wobei ein Temperaturbereich in dem das vernetzte Polyrotaxan von einem nicht vernetzten Zustand in einen vernetzten Zustand übergeht, oder von einem vernetzten Zustand in einen nicht vernetzten Zustand übergeht, zwischen 5 und 90 °C liegt.

6. Polyrotaxan nach einem der Ansprüche 2 bis 5, wobei das cyclische Molekül, das die Hydroxylgruppe(n) aufweist, unter α-Cyclodextrin, β-Cyclodextrin und γ-Cyclodextrin ausgewählt ist.

7. Polyrotaxan nach einem der Ansprüche 2 bis 6, wobei die Zahl der funktionellen Gruppen der Formel I 0,05 bis 0,9 beträgt, und die Zahl der funktionellen Gruppen der Formel II 0,05 bis 0,9 beträgt, wobei die Zahl der Hydroxylgruppen des cyclischen Moleküls auf 1 normiert ist.

8. Polyrotaxan nach einem der Ansprüche 1 bis 7, wobei das lineare Molekül ausgewählt ist unter Polyethylenglycol, Polyisopren, Polyisobutylen, Polybutadien, Polypropylenglycol, Polytetrahydrofuran, Polydimethylsiloxan, Polyethylen und Polypropylen.

9. Polyrotaxan nach einem der Ansprüche 1 bis 8, wobei das lineare Molekül ein Molekulargewicht von 10000 oder mehr aufweist.

10. Polyrotaxan nach einem der Ansprüche 1 bis 9, wobei die Endgruppe ausgewählt ist unter Dinitrophenylgruppen; Cyclodextrinen; Adamantangruppen; Tritylgruppen; Fluoresceinen; Pyrenen; substituierten Benzolen; polycyclischen Aromaten die substituiert sein können; und Steroiden.

11. Polyrotaxan nach einem der Ansprüche 1 bis 10, wobei das cyclische Molekül von
α-Cyclodextrin abgeleitet ist, und das lineare Molekül Polyethylenglycol ist.

12. Polyrotaxan nach einem der Ansprüche 1 bis 11, wobei das lineare Molekül das cyclische Molekül in einer Menge von 0,001 bis 0,6 der größtmöglichen Einschlussmenge aufgereiht enthält, die als die Menge definiert ist, in der das cyclische Molekül maximal aufgenommen werden kann, wenn das lineare Molekül die cyclischen Moleküle aufgereiht aufnimmt, und die größtmögliche Menge auf 1 normiert ist.

13. Material, dass das Polyrotaxan nach einem der Ansprüche 1 bis 12 umfasst.

14. Verfahren zur Herstellung eines Polyrotaxans, umfassend ein Pseudopolyrotaxan, das ein lineares Molekül und ein cyclisches Molekül (cyclische Moleküle) aufweist, worin das lineare Molekül in der Kavität (den Kavitäten) des darauf aufgereihten cyclischen Moleküls (Moleküle) eingeschlossen ist, und Endgruppen, die jeweils an den Enden des Pseudopolyrotaxans angeordnet sind, um die Dissoziation des cyclischen Moleküls (Moleküle) zu verhindern,
wobei ein Teil der Hydroxylgruppen des cyclischen Moleküls durch eine funktionelle Gruppe der folgenden Formel I, und durch zumindest eine funktionelle Gruppe, die ausgewählt ist unter den folgenden Formeln II-1 bis II-6, substituiert ist,
worin R, R₁ R₃ und R₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₅ₐ, R_{5b} und R_{5c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und R₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₆ für eine fotoreaktive Gruppe steht:
umfassend die Schritte:
1) Herstellung des Pseudopolyrotaxans;
2) Anbringen der Endgruppen an beiden Enden des erhaltenen Pseudopolyrotaxans zur Herstellung des Polyrotaxans; und
3) Substitution eines Teils der Hydroxylgruppen des cyclischen Moleküls mit der funktionellen Gruppe;
wobei der Schritt der Substitution
A) vor dem Schritt 1 ) der Herstellung des Pseudopolyrotaxans, und/oder
B) nach dem Schritt 2 ) der Anbringung der Endgruppen zur Herstellung des
Polyrotaxans
durchgeführt wird:

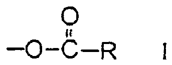

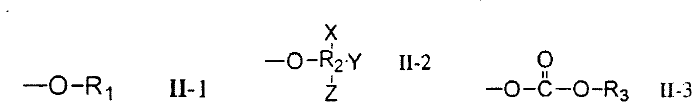

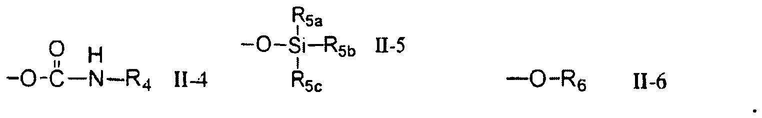

15. Verfahren nach Anspruch 14, wobei der Schritt der Substitution nach dem Schritt 2) der Anbringung der Endgruppen zur Herstellung des Polyrotaxans, durchgeführt wird.

16. Verfahren nach Anspruch 14 oder 15, wobei im Schritt der Substitution mit der funktionellen Gruppe ein Schritt zur Einführung der funktionellen Gruppen der Formel I
X) vor dem Schritt der Einführung einer der funktionellen Gruppe der Formel II;
Y) nach dem Schritt der Einführung einer der funktionellen Gruppen der Formel II; oder
Z) gleichzeitig mit dem Schritt der Einführung einer der funktionellen Gruppen der Formel II;
durchgeführt wird.

17. Verfahren nach einem der Ansprüche 14 bis 16, wobei im Schritt der Substitution mit der funktionellen Gruppe ein Schritt zur Einführung der funktionellen Gruppen der Formel 1 Y) nach dem Schritt der Einführung einer der funktionellen Gruppen der Formel II, durchgeführt wird.

18. Vernetztes Polyrotaxan, umfassend zumindest zwei Moleküle Polyrotaxan, wobei die zumindest zwei Moleküle Polyrotaxan durch eine physikalische Bindung vernetzt sind,
wobei das Polyrotaxan ein Pseudopolyrotaxan, das ein lineares Molekül und ein cyclisches Molekül (cyclische Moleküle) aufweist, worin das lineare Molekül in der Kavität (den Kavitäten) des darauf aufgereihten cyclischen Moleküls (Moleküle) eingeschlossen ist, und Endgruppen umfasst, die jeweils an den Enden des Pseudopolyrotaxans angeordnet sind, um die Dissoziation des cyclischen Moleküls (Moleküle) zu verhindern,
und das cyclische Molekül (Moleküle) eine funktionelle Gruppe der folgenden Formel I und zumindest eine funktionelle Gruppe ausgewählt unter den folgenden Formeln II-1 bis II-6, einschließt,
worin R, R₁, R₃ und R₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₅ₐ, R_{5b} und R_{5c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und
R₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₆ für eine fotoreaktive Gruppe steht:

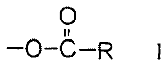

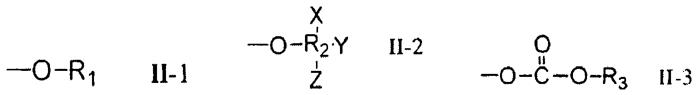

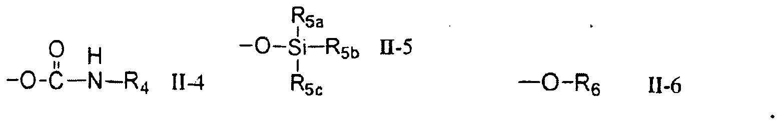

19. Vernetztes Polyrotaxan nach Anspruch 18, wobei das cyclische Molekül ein cyclisches Molekül ist, das eine Hydroxylgruppe(n) aufweist, und ein Teil der Hydroxylgruppen durch die funktionelle Gruppe der Formel I und durch zumindest eine der unter den folgenden Formeln II-1 bis II-6 ausgewählten funktionellen Gruppen, substituiert ist.

20. Vernetztes Polyrotaxan nach Anspruch 18 oder 19, wobei das Polyrotaxan in der Lage ist, reversibel auf einen externen Stimulus zu reagieren, der das Polyrotaxan in Abhängigkeit von der Gegenwart oder vom Fehlen des externen Stimulus reversibel von einem nicht vernetzten Zustand in einen vernetzten Zustand überführt, oder von einem vernetzten Zustand in einen nicht vernetzten Zustand überführt.

21. Vernetztes Polyrotaxan nach Anspruch 20, wobei der externe Stimulus Wärme ist.

22. Vernetztes Polyrotaxan nach Anspruch 20 oder 21, wobei der externe Stimulus Wärme ist, und wobei ein Temperaturbereich in dem das vernetzte Polyrotaxan von einem nicht vernetzten Zustand in einen vernetzten Zustand übergeht, oder von einem vernetzten Zustand in einen nicht vernetzten Zustand übergeht, zwischen 5 und 90 °C liegt.

23. Vernetztes Polyrotaxan nach einem der Ansprüche 19 bis 22, wobei das cyclische Molekül, das die Hydroxylgruppe(n) aufweist, unter α-Cyclodextrin, β-Cyclodextrin und γ-Cyclodextrin ausgewählt ist.

24. Vernetztes Polyrotaxan nach einem der Ansprüche 19 bis 23, wobei die Zahl der funktionellen Gruppen der Formel I 0,05 bis 0,9 beträgt, und die Zahl der funktionellen Gruppen der Formel II 0,05 bis 0,9 beträgt, wobei die Zahl der Hydroxylgruppen des cyclischen Moleküls auf 1 normiert ist.

25. Vernetztes Polyrotaxan nach einem der Ansprüche 18 bis 24, wobei das lineare Molekül ausgewählt ist unter Polyethylenglycol, Polyisopren, Polyisobutylen, Polybutadien, Polypropylenglycol, Polytetrahydrofuran, Polydimethylsiloxan, Polyethylen und Polypropylen.

26. Vernetztes Polyrotaxan nach einem der Ansprüche 18 bis 25, wobei das lineare Molekül ein Molekulargewicht von 10000 oder mehr aufweist.

27. Vernetztes Polyrotaxan nach einem der Ansprüche 18 bis 26, wobei die Endgruppe ausgewählt ist unter Dinitrophenylgruppen; Cyclodextrinen; Adamantangruppen; Tritylgruppen; Fluoresceinen; Pyrenen; substituierten Benzolen; polycyclischen Aromaten die substituiert sein können; und Steroiden.

28. Vernetztes Polyrotaxan nach einem der Ansprüche 18 bis 27, wobei das cyclische Molekül von α-Cyclodextrin abgeleitet ist, und das lineare Molekül Polyethylenglycol ist.

29. Vernetztes Polyrotaxan nach einem der Ansprüche 18 bis 28, wobei das lineare Molekül das cyclische Molekül in einer Menge von 0,001 bis 0,6 der größtmöglichen Einschlussmenge aufgereiht enthält, die als die Menge definiert ist, in der das cyclische Molekül maximal aufgenommen werden kann, wenn das lineare Molekül die cyclischen Moleküle aufgereiht aufnimmt, und die größtmögliche Menge auf 1 normiert ist.

30. Material, dass das vernetzte Polyrotaxan nach einem der Ansprüche 18 bis 29 umfasst.

31. Verfahren zur Herstellung eines vernetzten Polyrotaxans, das zumindest zwei Moleküle Polyrotaxan umfasst, wobei die zumindest zwei Moleküle Polyrotaxan durch physikalische Bindung vernetzt sind,
wobei das Polyrotaxan ein Pseudopolyrotaxan, das ein lineares Molekül und ein cyclisches Molekül (cyclische Moleküle) aufweist, worin das lineare Molekül in der Kavität (den Kavitäten) des darauf aufgereihten cyclischen Moleküls (Moleküle) eingeschlossen ist, und Endgruppen umfasst, die jeweils an den Enden des Pseudopolyrotaxans angeordnet sind, um die Dissoziation des cyclischen Moleküls (Moleküle) zu verhindern,
wobei ein Teil der Hydroxylgruppen des cyclischen Moleküls (Moleküle) durch eine funktionelle Gruppe der folgenden Formel I, und durch zumindest eine funktionelle Gruppe, die ausgewählt ist unter den folgenden Formeln 11-1 bis II-6, substituiert ist,
worin R, R₁, R₃ und R₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₅ₐ, R_{5b} und R_{5c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und R₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₆ für eine fotoreaktive Gruppe steht:
wobei das Verfahren die Schritte:
1) Herstellung des Pseudopolyrotaxans;
2) Anbringen der Endgruppe an beiden Enden des erhaltenen Pseudopolyrotaxans zur Herstellung eines Polyrotaxans; und
3) Substitution eines Teils der Hydroxylgruppen des cyclischen Moleküls durch eine funktionelle Gruppe der folgenden Formel I, und durch zumindest eine funktionelle Gruppe, die ausgewählt ist unter den folgenden Formeln II-1 bis II-6;
um dabei das Polyrotaxan zu erhalten;
4) Auflösung von zumindest zwei Molekülen des erhaltenen Polyrotaxans in einem Lösungsmittel; und
5) Vermittlung eines externen Stimulus an die zumindest zwei Moleküle Polyrotaxan im Lösemittel, um die zumindest zwei Moleküle Polyrotaxan physikalisch durch eine physikalische Bindung zu vernetzen,
wobei 3) der Schritt der Substitution
A) vor 1) dem Schritt der Herstellung des Pseudopolyrotaxans, und/oder
B) nach 2) dem Schritt der Anbringung der Endgruppen zur Herstellung des Polyrotaxans durchgeführt wird:

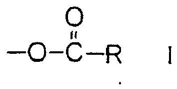

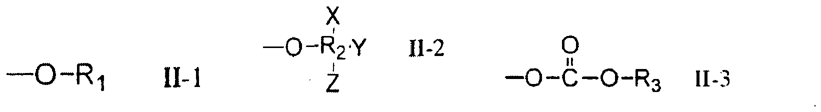

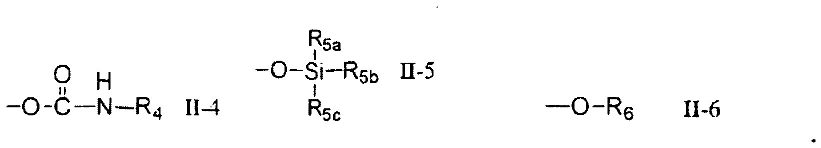

32. Verfahren nach Anspruch 31, wobei der Schritt der Substitution mit der funktionellen Gruppe nach 2), dem Schritt der Anbringung der Endgruppen zur Herstellung des Polyrotaxans, durchgeführt wird.

33. Verfahren nach Anspruch 31 oder 32, wobei im Schritt der Substitution mit der funktionellen Gruppe ein Schritt zur Einführung der funktionellen Gruppe der Formel I
X) vor dem Schritt der Einführung einer der funktionellen Gruppen der Formel II;
Y) nach dem Schritt der Einführung einer der funktionellen Gruppen der Formel II; oder
Z) gleichzeitig mit dem Schritt der Einführung einer der funktionellen Gruppen der
Formel II;
durchgeführt wird.

34. Verfahren nach einem der Ansprüche 31 bis 33, wobei im Schritt der Substitution mit der funktionellen Gruppe ein Schritt zur Einführung der funktionellen Gruppen der Formel I Y) nach dem Schritt der Einführung einer der funktionellen Gruppen der Formel II, durchgeführt wird.

35. Verfahren nach einem der Ansprüche 31 bis 34, wobei das Lösungsmittel ein hydrophiles Lösungsmittel ist.

36. Vernetztes Polyrotaxan umfassend ein erstes Polymer und ein erstes Polyrotaxan, wobei das erste Polymer ganz oder teilweise vernetzt und das erste Polyrotaxan ganz oder teilweise vernetzt sind,
wobei das erste Polyrotaxan ein erstes Pseudopolyrotaxan, das ein erstes lineares Molekül und ein erstes cyclisches Molekül (cyclische Moleküle) aufweist, worin das erste lineare Molekül in der Kavität (den Kavitäten) des darauf aufgereihten ersten cyclischen Moleküls (cyclischen Moleküle) eingeschlossen ist, und erste Endgruppen umfasst, die jeweils an den Enden des ersten Pseudopolyrotaxans angeordnet sind, um die Dissoziation des ersten cyclischen Moleküls (Moleküle) zu verhindern,
und das erste cyclische Molekül (Moleküle) des ersten Polyrotaxans eine funktionelle Gruppe der folgenden Formel I und zumindest eine funktionelle Gruppe ausgewählt unter den folgenden Formeln II-1 bis II-6, einschließt,
worin R, R₁, R₃ und R₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₅ₐ, R_{5b} und R_{5c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und
R₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₆ für eine fotoreaktive Gruppe steht:

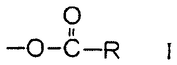

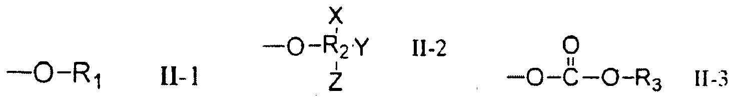

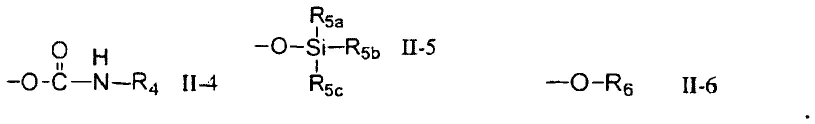

37. Vernetztes Polyrotaxan nach Anspruch 36, wobei das erste Polymer ein zweites Polyrotaxan ist und das zweite Polyrotaxan ein zweites Pseudopolyrotaxan, das ein zweites lineares Molekül und ein zweites cyclisches Molekül (Moleküle) aufweist, worin ein zweites lineares Molekül in der Kavität (Kavitäten) des darauf aufgereihten zweiten cyclischen Moleküls (Moleküle) eingeschlossen ist, und zweite Endgruppen umfasst, die jeweils an den Enden des zweiten Pseudopolyrotaxans angeordnet sind, um die Dissoziation des zweiten cyclischen Moleküls (Moleküle) zu verhindern,
und das zweite cyclische Molekül (Moleküle) des zweiten Polyrotaxans eine funktionelle Gruppe der folgenden Formel und zumindest eine funktionelle Gruppe ausgewählt unter den folgenden Formeln II'-1 bis II`-6, einschließt,
worin R', R₁₁, R₁₃ und R₁₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₁₅ₐ, R_{15b}, und R_{15c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und
R₁₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₁₆ für eine fotoreaktive Gruppe steht:

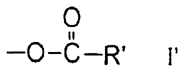

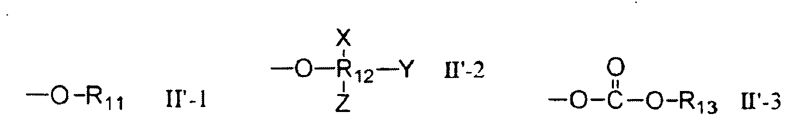

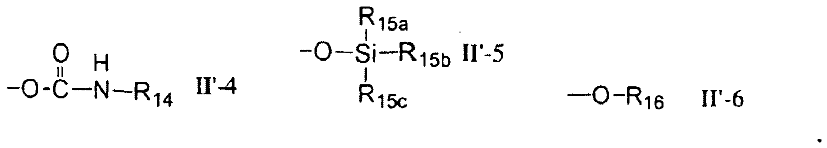

38. Vernetztes Polyrotaxan nach Anspruch 36 oder 37, wobei das cyclische Molekül ein cyclisches Molekül ist, das eine Hydroxylgruppe(n) aufweist, und ein Teil der Hydroxylgruppe(n) durch die funktionelle Gruppe der Formel I und durch zumindest eine der unter den folgenden Formeln II-1 bis II-6 ausgewählten funktionellen Gruppen, substituiert ist.

39. Vernetztes Polyrotaxans nach einem Ansprüche 36 bis 38, wobei das vernetzte Polyrotaxan eine Lichtdurchlässigkeit von 90%/mm oder mehr bei einer Wellenlänge von 400 bis 800 nm aufweist.

40. Vernetztes Polyrotaxan nach einem der Ansprüche 36 bis 39, wobei das vernetzte Polyrotaxan ein Streckverhältnis im Bereich von 100 bis 1500 % aufweist.

41. Vernetztes Polyrotaxan nach einem der Ansprüche 36 bis 40, wobei das vernetzte Polyrotaxan eine Lichtdurchlässigkeit von 90%/mm oder mehr bei einer Wellenlänge von 400 bis 800 nm und ein Streckverhältnis von 300 % oder mehr aufweist.

42. Vernetztes Polyrotaxan nach einem der Ansprüche 36 bis 41, wobei das vernetzte Polyrotaxan in Abhängigkeit von der Gegenwart oder vom Fehlen des externen Stimulus hinsichtlich einer optischen Eigenschaft und/oder hinsichtlich des Schwellungs-Kontraktionsverhaltens reversibel variiert.

43. Vernetztes Polyrotaxan nach Anspruch 42, wobei der externe Stimulus Wärme ist, und das vernetzte Polyrotaxan hinsichtlich der optischen Eigenschaft und/oder hinsichtlich des Schwellungs-Kontraktionsverhalten in einem Temperaturbereich von 5 bis 90°C reversibel variiert.

44. Vernetztes Polyrotaxan nach Anspruch 42 oder 43, wobei die optische Eigenschaft die Transparenz des vernetzten Polyrotaxan ist.

45. Vernetztes Polyrotaxan nach Anspruch 42 oder 43, wobei das Schwellungs-Kontraktionsverhalten eine Veränderung der Menge eines eingeschlossenen Lösungsmittels ist, die verursacht wird durch Absorption und/oder Freisetzung des Lösungsmittels durch das vernetzte Polyrotaxan.

46. Vernetztes Polyrotaxan nach einem Ansprüche 38 bis 45, wobei das erste und/oder zweite cyclische Molekül, das die Hydroxylgruppe(n) aufweist, unter α-Cyclodextrin, β-Cyclodextrin und γ-Cyclodextrin ausgewählt ist.

47. Vernetztes Polyrotaxan nach einem der Ansprüche 36 bis 46, wobei die Zahl der funktionellen Gruppen der Formel 0,05 bis 0,9 beträgt, und die Zahl der funktionellen Gruppen der Formel II 0,05 bis 0,9 beträgt, wobei die Zahl der Hydroxylgruppen des ersten und/oder zweiten cyclischen Moleküls auf 1 normiert ist.

48. Vernetztes Polyrotaxan nach einem der Ansprüche 36 bis 47, wobei das erste und/oder zweite lineare Molekül ausgewählt ist unter Polyethylenglycol, Polyisopren, Polyisobutylen, Polybutadien, Polypropylenglycol, Polytetrahydrofuran, Polydimethylsiloxan, Polyethylen und Polypropylen.

49. Vernetztes Polyrotaxan nach einem der Ansprüche 36 bis 48, wobei das erste und/oder zweite lineare Molekül ein Molekulargewicht von 10000 oder mehr aufweist.

50. Vernetztes Polyrotaxan nach einem der Ansprüche 36 bis 49, wobei die erste und/oder zweite Endgruppe ausgewählt ist unter Dinitrophenylgruppen; Cyclodextrinen; Adamantangruppen; Tritylgruppen; Fluoresceinen; Pyrenen; substituierten Benzolen; polycyclischen Aromaten die substituiert sein können; und Steroiden.

51. Vernetztes Polyrotaxan nach einem der Ansprüche 36 bis 50, wobei das erste und/oder zweite cyclische Molekül von α-Cyclodextrin abgelietet ist, und das erste und/oder zweite lineare Molekül Polyethylenglycol ist.

52. Vernetztes Polyrotaxan nach einem der Ansprüche 36 bis 51, wobei das erste und/oder zweite lineare Molekül das erste und/oder zweite cyclische Molekül in einer Menge von 0,001 bis 0,6 der größtmöglichen Einschlussmenge aufgereiht enthält, die als die Menge definiert ist, in der das erste und/oder zweite cyclische Molekül maximal aufgenommen werden kann, wenn das erste und/oder zweite lineare Molekül das erste und/oder zweite cyclische Molekül aufgereiht aufnimmt, und die größtmögliche Menge auf 1 normiert ist.

53. Material umfassend das vernetzte Polyrotaxan nach einem der Ansprüche 36 bis 52.

54. Verfahren zur Herstellung eines vernetzten Polyrotaxans, das ein erstes Polymer und ein erstes Polyrotaxan einschließt, wobei das erste Polymer ganz oder teilweise und das erste Polyrotaxan ganz oder teilweise vernetzt sind,
wobei das erste Polyrotaxan ein erstes Pseudopolyrotaxan, das ein erstes lineares Molekül und ein erstes cyclisches Molekül (cyclische Moleküle) aufweist, worin das erste lineare Molekül in der Kavität (den Kavitäten) des darauf aufgereihten ersten cyclischen Moleküls (cyclischen Moleküle) eingeschlossen ist, und erste Endgruppen umfasst, die jeweils an den Enden des ersten Pseudopolyrotaxans angeordnet sind, um die Dissoziation des ersten cyclischen Moleküls (Moleküle) zu verhindern,
wobei ein Teil der Hydroxylgruppen des ersten cyclischen Moleküls mit einer funktionellen Gruppe der folgenden Formel I, und zumindest einer funktionellen Gruppen ausgewählt unter den folgenden Formel II-1 bis II-6, substituiert ist,
worin R, R₁, R₃ und R₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₅ₐ, R_{5b} und R_{5c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und
R₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₆ für eine fotoreaktive Gruppe steht:
wobei das Verfahren die Schritte:
1) Herstellung des ersten Pseudopolyrotaxans;
2) Anbringen einer ersten Endgruppe an beiden Enden des erhaltenen Pseudopolyrotaxans zur Herstellung eines Polyrotaxans; und
3) Substitution eines Teils der Hydroxylgruppen des ersten cyclischen Moleküls durch die funktionelle Gruppe der Formel I, und durch zumindest eine funktionelle Gruppe, die ausgewählt ist unter den folgenden Formeln II-1 bis II-6;
um dabei das erste Polyrotaxan zu erhalten; und
4) chemische Vernetzung des erhaltenen ersten Polyrotaxans und eines ersten Polymers umfasst;
wobei 3) der Schritt der Substitution
A) vor 1) dem Schritt der Herstellung des ersten Pseudopolyrotaxans, und/oder
B) nach 2) dem Schritt der Anbringung der Endgruppen zur Herstellung eines ersten Polyrotaxans durchgeführt wird; und der Schritt der chemischen Vernetzung durch
G) eine Vernetzungsreaktion durch Zugabe eines Vernetzungsreagenzes, oder durch
H) eine Fotovernetzungsreaktion, bei der eine im ersten Polyrotaxan enthaltenen fotoreaktive Gruppe mit Licht bestrahlt wird,
durchgeführt wird:

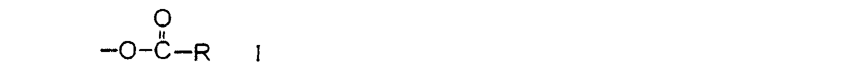

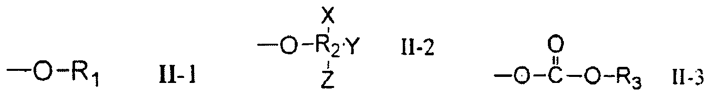

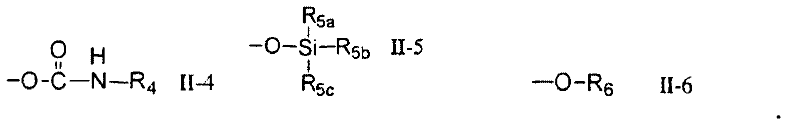

55. Verfahren nach Anspruch 54, wobei das erste Polymer ein zweites Polyrotaxan ist und das zweite Polyrotaxan ein zweites Pseudopolyrotaxan, das ein zweites lineares Molekül und ein zweites cyclisches Molekül (Moleküle) aufweist, worin ein zweites lineares Molekül in der Kavität (Kavitäten) des darauf aufgereihten zweiten cyclischen Moleküls (Moleküle) eingeschlossen ist, und zweite Endgruppen umfasst, die jeweils an den Enden des zweiten Pseudopolyrotaxans angeordnet sind, um die Dissoziation des zweiten cyclischen Moleküls (Moleküle) zu verhindern,
und ein Teil der Hydroxylgruppen des zweiten cyclischen Moleküls durch die funktionelle Gruppe der Formel l'und durch zumindest eine der unter den folgenden Formeln II'-1 bis II'-6 ausgewählten funktionellen Gruppen, substituiert ist,
worin R', R₁₁, R₁₃ und R₁₄ jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für einen cyclische Alkylthioethergruppe mit 2 bis 12 Kohlenstoffatomen, stehen,
R₁₅ₐ, R_{15b}, und R_{15c} jeweils unabhängig voneinander für eine lineare oder verzweigte Alkylgruppe mit 1 bis 12 Kohlenstoffatomen, eine lineare oder verzweigte Alkylgruppe mit 2 bis 12 Kohlenstoffatomen die zumindest eine Ethergruppe aufweist, eine cyclische Alkylgruppe mit 3 bis 12 Kohlenstoffatomen, eine cyclische Alkylethergruppe mit 2 bis 12 Kohlenstoffatomen, oder für eine cyclische Alkylthioethergruppe mit 2 bis 12 Kohlenstoffatomen stehen, und
R₁₂ für eine Substitutionsgruppe steht, die durch Entfernen von drei Wasserstoffatomen von einer Gruppe ausgewählt unter einer linearen oder verzweigten Alkylgruppe mit 1 bis 12 Kohlenstoffatomen, einer linearen oder verzweigten Alkylgruppe mit 2 bis 12 Kohlenstoffatomen die zumindest eine Ethergruppe aufweist, einer cyclischen Alkylgruppe mit 3 bis 12 Kohlenstoffatomen, einer cyclischen Alkylethergruppe mit 2 bis 12 Kohlenstoffatomen und einer cyclischen Alkylthioethergruppe mit 2 bis 12 Kohlenstoffatomen erhalten wird, wobei zumindest eine der Gruppen X bis Z für eine Gruppe steht, die unter einer Hydroxylgruppe, einer NH₃-Gruppe und einer SH-Gruppe ausgewählt ist, und wobei die übrigen Gruppen X bis Z für Wasserstoffatome stehen;
und worin R₁₆ für eine fotoreaktive Gruppe steht); und
wobei das zweite Polyrotaxan durch
1') Herstellung des zweiten Pseudopolyrotaxans;
2') Anbringen der Endgruppen an beiden Enden des erhaltenen zweiten Pseudopolyrotaxans mit einer zweiten Endgruppe zur Herstellung eines zweiten Polyrotaxans; und
3') Substitution eines Teils der Hydroxylgruppen des zweiten cyclischen Moleküls mit der funktionellen Gruppe der Formel I', und mit zumindest einer funktionellen Gruppe ausgewählt unter den Formeln II'-1 bis II`-6 erhalten wird:

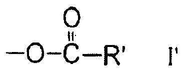

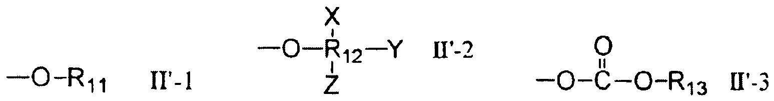

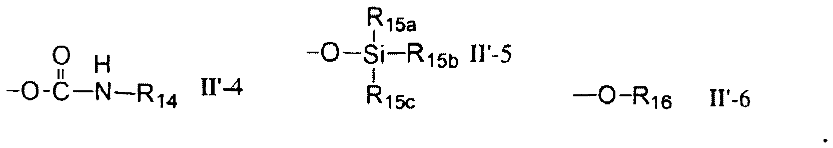

56. Verfahren nach Ansprüchen 54 oder 55, wobei 4) der Schritt der chemischen Vernetzung über G) die Vernetzungsreaktion durch Zugabe eines Vernetzungsreagenzes durchgeführt wird, und das Vernetzungsreagenz unter Cyanurchlorid, Trimesoylchlorid, Terephthaloylchlorid; Epichlorhydrin; Dibrombenzol, Glutaraldehyd, aliphatischem polyfunktionellem Isocyanat, aromatischem polyfunktionellem Isocyanat, Toluoldiisocyanat, Hexamethylendiisocyanat, Diphenylsulfon, 1,1'-Carbonyldiimidazol, Alkoxysilanen, und deren Derivaten, und Initiatoren von Fotovernetzungsreaktionen ausgewählt ist.

57. Verfahren nach einem der Ansprüche 54 bis 56, wobei der Schritt der Substitution mit der funktionellen Gruppe nach 2) und/oder 2') dem Schritt der Anbringung der Endgruppen zur Herstellung des ersten und/oder zweiten Polyrotaxans, durchgeführt wird.

58. Verfahren nach einem der Ansprüche 54 bis 57, wobei im Schritt der Substitution mit der funktionellen Gruppe ein Schritt zur Einführung der funktionellen Gruppe der Formel I oder I'
X) vor einem Schritt zur Einführung einer der funktionellen Gruppen der Formeln II oder II';
Y) nach einem Schritt zur Einführung einer der funktionellen Gruppen der Formeln II oder II'; oder
Z) gleichzeitig mit dem Schritt der Einführung einer der funktionellen Gruppen der Formeln II oder II';
durchgeführt wird.

59. Verfahren nach einem der Ansprüche 55 bis 58, wobei im Schritt der Substitution mit der funktionellen Gruppe ein Schritt zur Einführung der funktionellen Gruppe der Formel I oder I' Y) nach einem Schritt zur Einführung einer der funktionellen Gruppen der Formeln II oder II' durchgeführt wird.

## Revendications

1. Polyrotaxane comprenant un pseudo-polyrotaxane, qui a une molécule linéaire et une ou plusieurs molécules cycliques, dans lequel la molécule linéaire est incluse dans une ou plusieurs cavités de la ou des molécules cycliques d'une manière embrochée, et des groupes de coiffage, chacun se situant à chaque extrémité du pseudo-polyrotaxane afin d'empêcher la dissociation de la ou des molécules cycliques,
dans lequel la ou les molécules cycliques comprennent un groupe fonctionnel représenté par la formule I suivante, et au moins un groupe fonctionnel choisi dans l'ensemble constitué par les formules II-1 à II-6 suivantes, où chacun de R, R₁, R₃ et R₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone,
chacun de R₅ₐ, R_{5b} et R_{5c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone, et
R₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène ; et
R₆ représente un groupe photoréactif :

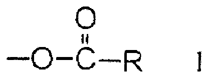

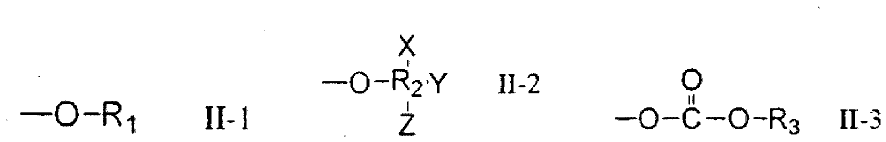

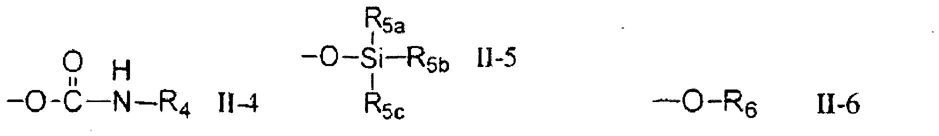

2. Polyrotaxane selon la revendication 1, dans lequel la molécule cyclique est une molécule cyclique ayant un ou plusieurs groupes hydroxy, et une partie du ou des groupes hydroxy est substituée par le groupe fonctionnel représenté par la formule I et l'au moins un groupe fonctionnel choisi dans l'ensemble constitué par les formules II-1 à II-6 suivantes.

3. Polyrotaxane selon la revendication 1 ou 2, lequel polyrotaxane est capable de répondre de manière réversible à un stimulus externe, qui transforme de manière réversible le polyrotaxane d'un état non réticulé à un état réticulé, ou d'un état réticulé à un état non réticulé, en fonction de la présence ou de l'absence du stimulus externe.

4. Polyrotaxane selon la revendication 3, dans lequel le stimulus externe est la chaleur.

5. Polyrotaxane selon la revendication 3 ou 4, dans lequel le stimulus externe est la chaleur, et la plage de température dans laquelle le polyrotaxane réticulé se transforme d'un état non réticulé à un état réticulé, ou d'un état réticulé à un état non réticulé, va de 5 à 90°C.

6. Polyrotaxane selon l'une quelconque des revendications 2 à 5, dans lequel la molécule cyclique ayant le ou les groupes hydroxy est choisie dans l'ensemble constitué par l'α-cyclodextrine, la β-cyclodextrine et la γ-cyclodextrine.

7. Polyrotaxane selon l'une quelconque des revendications 2 à 6, dans lequel le nombre de groupes fonctionnels représentés par la formule I est de 0,05 à 0,9, et le nombre de groupes fonctionnels représentés par la formule II est de 0,05 à 0,9, quand le nombre de groupes hydroxy de la molécule cyclique est normalisé à 1.

8. Polyrotaxane selon l'une quelconque des revendications 1 à 7, dans lequel la molécule linéaire est choisie dans l'ensemble constitué par le polyéthylèneglycol, le polyisoprène, le polyisobutylène, le polybutadiène, le polypropylèneglycol, le polytétrahydrofurane, le polydiméthylsiloxane, le polyéthylène et le polypropylène.

9. Polyrotaxane selon l'une quelconque des revendications 1 à 8, dans lequel la molécule linéaire a une masse moléculaire de 10 000 ou plus.

10. Polyrotaxane selon l'une quelconque des revendications 1 à 9, dans lequel le groupe de coiffage est choisi dans l'ensemble constitué par les groupes dinitrophényle ; les cyclodextrines ; les groupes adamantane ; les groupes trityle ; les fluorescéines ; les pyrènes ; les benzènes substitués ; les aromatiques polycycliques qui peuvent être substitués ; et les stéroïdes.

11. Polyrotaxane selon l'une quelconque des revendications 1 à 10, dans lequel la molécule cyclique est dérivée d'α-cyclodextrine, et la molécule linéaire est le polyéthylèneglycol.

12. Polyrotaxane selon l'une quelconque des revendications 1 à 11, dans lequel la molécule linéaire a la molécule cyclique incluse d'une manière embrochée en une quantité de 0,001 à 0,6 fois la quantité d'inclusion maximale, laquelle est définie par la quantité maximale à laquelle les molécules cycliques peuvent être incluses quand la molécule linéaire a les molécules cycliques incluses d'une manière embrochée, et la quantité maximale est normalisée à 1.

13. Matériau comprenant le polyrotaxane selon l'une quelconque des revendications 1 à 12.

14. Procédé pour produire un polyrotaxane comprenant un pseudo-polyrotaxane, qui a une molécule linéaire et une ou plusieurs molécules cycliques, dans lequel la molécule linéaire est incluse dans une ou plusieurs cavités de la ou des molécules cycliques d'une manière embrochée, et des groupes de coiffage, chacun se situant à chaque extrémité du pseudo-polyrotaxane afin d'empêcher la dissociation de la ou des molécules cycliques,
dans lequel une partie des groupes hydroxy de la molécule cyclique est substituée par un groupe fonctionnel représenté par la formule I suivante, et au moins un groupe fonctionnel choisi dans l'ensemble constitué par les formules II-1 à II-6 suivantes,
où chacun de R, R₁, R₃ et R₄ représente indépendamment un groupe alkyle linéaire ou ramifié ayant de 1 à 12 atomes de carbone, un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone,
chacun de R₅ₐ, R_{5b} et R_{5c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone, et
R₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène ; et
R₆ représente un groupe photoréactif :
qui comprend les étapes consistant à :
1) préparer le pseudo-polyrotaxane ;
2) coiffer les deux extrémités du pseudo-polyrotaxane résultant avec les groupes de coiffage, pour préparer le polyrotaxane ; et
3) substituer une partie des groupes hydroxy de la molécule cyclique par le groupe fonctionnel ;
dans lequel l'étape de substitution est effectuée
A) avant l'étape 1) de préparation du pseudo-polyrotaxane, et/ou
B) après l'étape 2) de coiffage pour préparer le polyrotaxane :

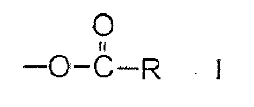

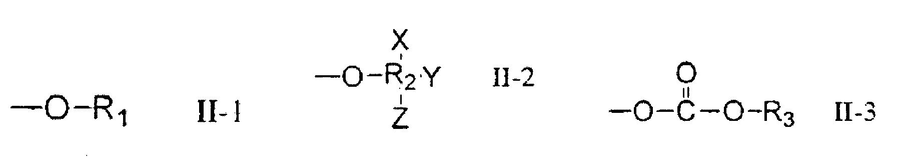

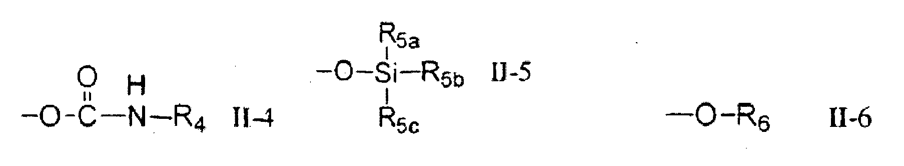

15. Procédé selon la revendication 14, dans lequel l'étape de substitution est effectuée après l'étape 2) de coiffage pour préparer le polyrotaxane.

16. Procédé selon la revendication 14 ou 15, dans lequel, dans l'étape de substitution par le groupe fonctionnel, une étape d'introduction du groupe fonctionnel représenté par la formule I est effectuée
X) avant une étape d'introduction de l'un quelconque des groupes fonctionnels représentés par la formule II ;
Y) après l'étape d'introduction de l'un quelconque des groupes fonctionnels représentés par la formule II ; ou
Z) en même temps que l'étape d'introduction de l'un quelconque des groupes fonctionnels représentés par la formule II.

17. Procédé selon l'une quelconque des revendications 14 à 16, dans lequel, dans l'étape de substitution par le groupe fonctionnel, une étape d'introduction du groupe fonctionnel représenté par la formule I est effectuée Y) après une étape d'introduction de l'un quelconque des groupes fonctionnels représentés par la formule II.

18. Polyrotaxane réticulé comprenant au moins deux molécules de polyrotaxane, dans lequel les au moins deux molécules de polyrotaxane sont réticulées via une liaison physique,
dans lequel le polyrotaxane comprend un pseudo-polyrotaxane, qui a une molécule linéaire et une ou plusieurs molécules cycliques, dans lequel la molécule linéaire est incluse dans une ou plusieurs cavités de la ou des molécules cycliques d'une manière embrochée, et des groupes de coiffage, chacun se situant à chaque extrémité du pseudo-polyrotaxane afin d'empêcher la dissociation de la ou des molécules cycliques,
dans lequel la ou les molécules cycliques comprennent un groupe fonctionnel représenté par la formule I suivante, et au moins un groupe fonctionnel choisi dans l'ensemble constitué par les formules II-1 à II-6 suivantes,
où chacun de R, R₁, R₃ et R₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ;
chacun de R₅ₐ, R_{5b} et R_{5c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ; et
R₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène, et
R₆ représente un groupe photoréactif :

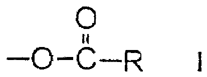

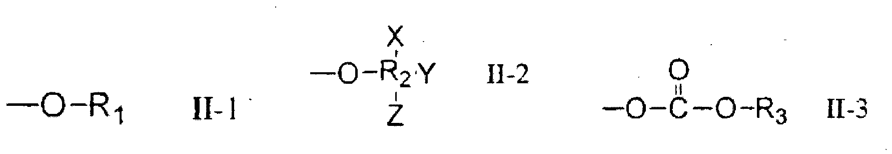

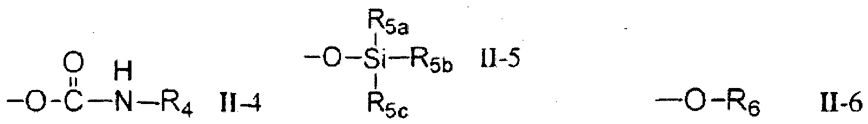

19. Polyrotaxane réticulé selon la revendication 18, dans lequel la molécule cyclique est une molécule cyclique ayant un ou plusieurs groupes hydroxy, et une partie des groupes hydroxy est substituée par le groupe fonctionnel représenté par la formule I et l'au moins un groupe fonctionnel choisi dans l'ensemble constitué par les formules II-1 à II-6.

20. Polyrotaxane réticulé selon la revendication 18 ou 19, dans lequel ledit polyrotaxane est capable de répondre de manière réversible à un stimulus externe, qui transforme de manière réversible le polyrotaxane d'un état non réticulé à un état réticulé, ou d'un état réticulé à un état non réticulé, en fonction de la présence ou de l'absence du stimulus externe.

21. Polyrotaxane réticulé selon la revendication 20, dans lequel le stimulus externe est la chaleur.

22. Polyrotaxane réticulé selon la revendication 20 ou 21, dans lequel le stimulus externe est la chaleur, et la plage de température dans laquelle le polyrotaxane réticulé se transforme d'un état non réticulé à un état réticulé, ou d'un état réticulé à un état non réticulé, va de 5 à 90°C.

23. Polyrotaxane réticulé selon l'une quelconque des revendications 19 à 22, dans lequel la molécule cyclique ayant le ou les groupes hydroxy est choisie dans l'ensemble constitué par l'α-cyclodextrine, la β-cyclodextrine et la γ-cyclodextrine.

24. Polyrotaxane réticulé selon l'une quelconque des revendications 19 à 23, dans lequel le nombre de groupes fonctionnels représentés par la formule I est de 0,05 à 0,9, et le nombre de groupes fonctionnels représentés par la formule II est de 0,05 à 0,9, quand le nombre de groupes hydroxy de la molécule cyclique est normalisé à 1.

25. Polyrotaxane réticulé selon l'une quelconque des revendications 18 à 24, dans lequel la molécule linéaire est choisie dans l'ensemble constitué par le polyéthylèneglycol, le polyisoprène, le polyisobutylène, le polybutadiène, le polypropylèneglycol, le polytétrahydrofurane, le polydiméthylsiloxane, le polyéthylène et le polypropylène.

26. Polyrotaxane réticulé selon l'une quelconque des revendications 18 à 25, dans lequel la molécule linéaire a une masse moléculaire de 10 000 ou plus.

27. Polyrotaxane réticulé selon l'une quelconque des revendications 18 à 26, dans lequel le groupe de coiffage est choisi dans l'ensemble constitué par les groupes dinitrophényle ; les cyclodextrines ; les groupes adamantane ; les groupes trityle ; les fluorescéines ; les pyrènes ; les benzènes substitués ; les aromatiques polycycliques qui peuvent être substitués ; et les stéroïdes.

28. Polyrotaxane réticulé selon l'une quelconque des revendications 18 à 27, dans lequel la molécule cyclique est dérivée d'α-cyclodextrine, et la molécule linéaire est le polyéthylèneglycol.

29. Polyrotaxane réticulé selon l'une quelconque des revendications 18 à 28, dans lequel la molécule linéaire a la molécule cyclique incluse d'une manière embrochée en une quantité de 0,001 à 0,6 fois la quantité d'inclusion maximale, laquelle est définie par la quantité maximale à laquelle les molécules cycliques peuvent être incluses quand la molécule linéaire a les molécules cycliques incluses d'une manière embrochée, et la quantité maximale est normalisée à 1.

30. Matériau comprenant le polyrotaxane réticulé selon l'une quelconque des revendications 18 à 29.

31. Procédé pour produire un polyrotaxane réticulé qui comprend au moins deux molécules de polyrotaxane, dans lequel les au moins deux molécules de polyrotaxane sont réticulées via une liaison physique,
dans lequel le polyrotaxane comprend un pseudo-polyrotaxane, qui a une molécule linéaire et une ou plusieurs molécules cycliques, dans lequel la molécule linéaire est incluse dans une ou plusieurs cavités de la ou des molécules cycliques d'une manière embrochée, et des groupes de coiffage, chacun se situant à chaque extrémité du pseudo-polyrotaxane afin d'empêcher la dissociation de la ou des molécules cycliques,
dans lequel une partie des groupes hydroxy de la ou des molécules cycliques est substituée par un groupe fonctionnel représenté par la formule I suivante, et au moins un groupe fonctionnel choisi dans l'ensemble constitué par les formules II-1 à II-6 suivantes,
où chacun de R, R₁, R₃ et R₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ;
chacun de R₅ₐ, R_{5b} et R_{5c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ; et
R₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène ; et
R₆ représente un groupe photoréactif :
lequel procédé comprend les étapes consistant à :
1) préparer le pseudo-polyrotaxane ;
2) coiffer les deux extrémités du pseudo-polyrotaxane résultant avec les groupes de coiffage, pour préparer un polyrotaxane ;
et
3) substituer une partie des groupes hydroxy de la molécule cyclique par le groupe fonctionnel représenté par la formule I, et au moins un groupe fonctionnel choisi dans l'ensemble constitué par les formules II-1 à II-6 ;
de façon à obtenir ainsi le polyrotaxane ;
4) dissoudre au moins deux molécules du polyrotaxane résultant dans un solvant ; et
5) conférer un stimulus externe aux au moins deux molécules de polyrotaxane dans le solvant pour réticuler physiquement les au moins deux molécules de polyrotaxane via une liaison physique,
dans lequel l'étape de substitution 3) est effectuée
A) avant l'étape 1) de préparation du pseudo-polyrotaxane, et/ou
B) après l'étape 2) de coiffage pour préparer le polyrotaxane :

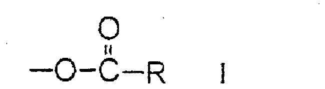

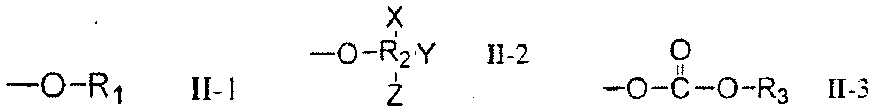

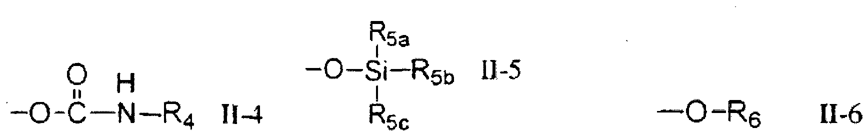

32. Procédé selon la revendication 31, dans lequel l'étape de substitution par le groupe fonctionnel est effectuée après l'étape 2) de coiffage pour préparer le polyrotaxane.

33. Procédé selon la revendication 31 ou 32, dans lequel, dans l'étape de substitution par le groupe fonctionnel, une étape d'introduction du groupe fonctionnel représenté par la formule I est effectuée
X) avant une étape d'introduction de l'un quelconque des groupes fonctionnels représentés par la formule II ;
Y) après l'étape d'introduction de l'un quelconque des groupes fonctionnels représentés par la formule II ; ou
Z) en même temps que l'étape d'introduction de l'un quelconque des groupes fonctionnels représentés par la formule II.

34. Procédé selon l'une quelconque des revendications 31 à 33, dans lequel, dans l'étape de substitution par le groupe fonctionnel, une étape d'introduction du groupe fonctionnel représenté par la formule I est effectuée Y) après une étape d'introduction de l'un quelconque des groupes fonctionnels représentés par la formule II.

35. Procédé selon l'une quelconque des revendications 31 à 34, dans lequel le solvant est un solvant hydrophile.

36. Polyrotaxane réticulé comprenant un premier polymère et un premier polyrotaxane, dans lequel tout ou partie du premier polymère et tout ou partie du premier polyrotaxane sont réticulés,
dans lequel le premier polyrotaxane comprend un premier pseudo-polyrotaxane, qui a une première molécule linéaire et une ou plusieurs premières molécules cycliques, dans lequel la première molécule linéaire est incluse dans une ou plusieurs cavités de la ou des premières molécules cycliques d'une manière embrochée, et des premiers groupes de coiffage, chacun se situant à chaque extrémité du premier pseudo-polyrotaxane afin d'empêcher la dissociation de la ou des premières molécules cycliques,
dans lequel la ou les premières molécules cycliques comprennent un groupe fonctionnel représenté par la formule I suivante, et au moins un groupe fonctionnel choisi dans l'ensemble constitué par les formules II-1 à II-6 suivantes,
où chacun de R, R₁, R₃ et R₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ;
chacun de R₅ₐ, R_{5b} et R_{5c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ; et
R₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène, et
R₆ représente un groupe photoréactif :

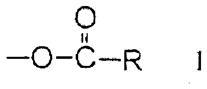

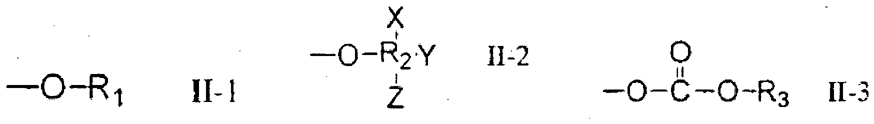

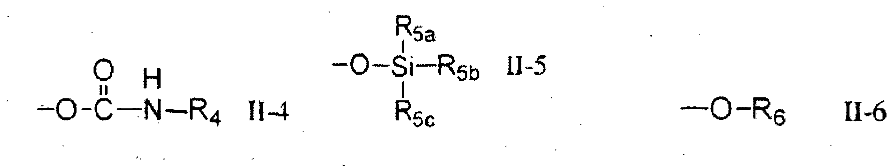

37. Polyrotaxane réticulé selon la revendication 36, dans lequel le premier polymère est un deuxième polyrotaxane, dans lequel le deuxième polyrotaxane comprend un deuxième pseudo-polyrotaxane, qui a une deuxième molécule linéaire et une ou plusieurs deuxièmes molécules cycliques, dans lequel la deuxième molécule linéaire est incluse dans une ou plusieurs cavités de la ou des deuxièmes molécules cycliques d'une manière embrochée, et des deuxièmes groupes de coiffage, chacun se situant à chaque extrémité du deuxième pseudo-polyrotaxane afin d'empêcher la dissociation de la ou des deuxièmes molécules cycliques,
dans lequel la ou les deuxièmes molécules cycliques du deuxième polyrotaxane comprennent un groupe fonctionnel représenté par la formule I' suivante, et au moins un groupe fonctionnel choisi dans l'ensemble constitué par les formules II'-1 à II'-6 suivantes,
où chacun de R', R₁₁, R₁₃ et R₁₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ;
chacun de R₁₅ₐ, R_{15b} et R_{15c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ; et
R₁₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène, et
R₁₆ représente un groupe photoréactif :

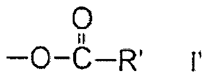

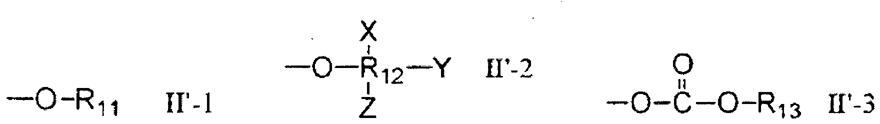

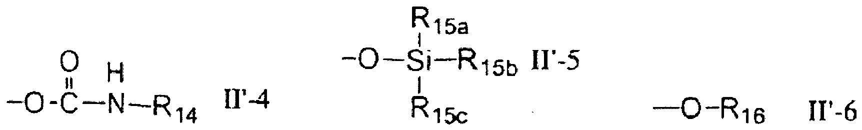

38. Polyrotaxane réticulé selon la revendication 36 ou 37, dans lequel la molécule cyclique est une molécule cyclique ayant un ou plusieurs groupes hydroxy, et une partie du ou des groupes hydroxy est substituée par le groupe fonctionnel représenté par la formule I et l'au moins un groupe fonctionnel choisi dans l'ensemble constitué par les formules II-1 à II-6.

39. Polyrotaxane réticulé selon l'une quelconque des revendications 36 à 38, lequel polyrotaxane réticulé a un coefficient de transmission de 90 %/mm ou plus pour une longueur d'onde de 400 à 800 nm.

40. Polyrotaxane réticulé selon l'une quelconque des revendications 36 à 39, lequel polyrotaxane réticulé a un rapport d'extension situé dans la plage allant de 100 à 1500 %.

41. Polyrotaxane réticulé selon l'une quelconque des revendications 36 à 40, lequel polyrotaxane réticulé a un coefficient de
transmission de 90 %/mm ou plus pour une longueur d'onde de 400 à 800 nm et un rapport d'extension de 300 % ou plus.

42. Polyrotaxane réticulé selon l'une quelconque des revendications 36 à 41, lequel polyrotaxane réticulé a des caractéristiques de propriété optique et/ou de gonflement-contraction variables de façon réversible, en fonction de la présence ou de l'absence du stimulus externe.

43. Polyrotaxane réticulé selon la revendication 42, dans lequel le stimulus externe est la chaleur, et le polyrotaxane réticulé a des caractéristiques de propriété optique et/ou de gonflement-contraction variables de façon réversible dans la plage de température allant de 5 à 90°C.

44. Polyrotaxane réticulé selon la revendication 42 ou 43, dans lequel la propriété optique est la transparence du polyrotaxane réticulé.

45. Polyrotaxane réticulé selon la revendication 42 ou 43, dans lequel la caractéristique de gonflement-contraction est un changement de la quantité contenue d'un solvant, dû à l'absorption et/ou à la libération du solvant par le polyrotaxane réticulé.

46. Polyrotaxane réticulé selon l'une quelconque des revendications 38 à 45, dans lequel les première et/ou deuxième molécules cycliques ayant le ou les groupes hydroxy sont choisies dans l'ensemble constitué par l'α-cyclodextrine, la β-cyclodextrine et la γ-cyclodextrine.

47. Polyrotaxane réticulé selon l'une quelconque des revendications 36 à 46, dans lequel le nombre de groupes fonctionnels représentés par la formule I est de 0,05 à 0,9, et le nombre de groupes fonctionnels représentés par la formule II est de 0,05 à 0,9, quand le nombre de groupes hydroxy des première et/ou deuxième molécules cycliques est normalisé à 1.

48. Polyrotaxane réticulé selon l'une quelconque des revendications 36 à 47, dans lequel les première et/ou deuxième molécules linéaires sont choisies dans l'ensemble constitué par le polyéthylèneglycol, le polyisoprène, le polyisobutylène, le polybutadiène, le polypropylèneglycol, le polytétrahydrofurane, le polydiméthylsiloxane, le polyéthylène et le polypropylène.

49. Polyrotaxane réticulé selon l'une quelconque des revendications 36 à 48, dans lequel les première et/ou deuxième molécules linéaires ont une masse moléculaire de 10 000 ou plus.

50. Polyrotaxane réticulé selon l'une quelconque des revendications 36 à 49, dans lequel les premier et/ou deuxième groupes de coiffage sont choisis dans l'ensemble constitué par les groupes dinitrophényle ; les cyclodextrines ; les groupes adamantane ; les groupes trityle ; les fluorescéines ; les pyrènes ; les benzènes substitués ; les aromatiques polycycliques qui peuvent être substitués ; et les stéroïdes.

51. Polyrotaxane réticulé selon l'une quelconque des revendications 36 à 50, dans lequel les première et/ou deuxième molécules cycliques sont dérivées d'α-cyclodextrine, et les première et/ou deuxième molécules linéaires sont le polyéthylèneglycol.

52. Polyrotaxane réticulé selon l'une quelconque des revendications 36 à 51, dans lequel les première et/ou deuxième molécules linéaires ont les première et/ou deuxième molécules cycliques incluses d'une manière embrochée en une quantité de 0,001 à 0,6 fois la quantité d'inclusion maximale, laquelle est définie par la quantité maximale à laquelle les première et/ou deuxième molécules cycliques peuvent être incluses quand les première et/ou deuxième molécules linéaires ont les première et/ou deuxième molécules
cycliques incluses d'une manière embrochée, et la quantité maximale est normalisée à 1.

53. Matériau comprenant le polyrotaxane réticulé selon l'une quelconque des revendications 36 à 52.

54. Procédé pour produire un polyrotaxane réticulé comprenant un premier polymère et un premier polyrotaxane, dans lequel tout ou partie du premier polymère et tout ou partie du premier polyrotaxane sont réticulés,
dans lequel le premier polyrotaxane comprend un premier pseudo-polyrotaxane, qui a une première molécule linéaire et une ou plusieurs premières molécules cycliques, dans lequel la première molécule linéaire est incluse dans une ou plusieurs cavités de la ou des premières molécules cycliques d'une manière embrochée, et des premiers groupes de coiffage, chacun se situant à chaque extrémité du premier pseudo-polyrotaxane afin d'empêcher la dissociation de la ou des premières molécules cycliques,
dans lequel une partie des groupes hydroxy de la première molécule cyclique est remplacée par un groupe fonctionnel représenté par la formule I suivante, et au moins un groupe fonctionnel choisi dans l'ensemble constitué par les formules II-1 à II-6 suivantes,
où chacun de R, R₁, R₃ et R₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ;
chacun de R₅ₐ, R_{5b} et R_{5c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ; et
R₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène, et
R₆ représente un groupe photoréactif ;
lequel procédé comprend les étapes consistant à :
1) préparer le premier pseudo-polyrotaxane ;
2) coiffer les deux extrémités du premier pseudo-polyrotaxane résultant avec un premier groupe de coiffage, pour préparer un premier polyrotaxane ; et
3) substituer une partie des groupes hydroxy de la première molécule cyclique par le groupe fonctionnel représenté par la formule I, et au moins un groupe fonctionnel choisi dans l'ensemble constitué par les formules II-1 à II-6 ;
de façon à obtenir ainsi le premier polyrotaxane ; et
4) réticuler chimiquement le premier polyrotaxane résultant et un premier polymère ;
dans lequel l'étape de substitution 3) est effectuée
A) avant l'étape 1) de préparation du premier pseudo-polyrotaxane, et/ou
B) après l'étape 2) de coiffage pour préparer le premier polyrotaxane ; et
l'étape de réticulation chimique 4) est effectuée par
G) une réaction de réticulation par addition d'un agent de réticulation, ou
H) une réaction de photo-réticulation où un groupe photoréactif contenu dans le premier polyrotaxane est irradié avec une lumière :

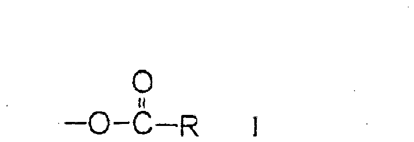

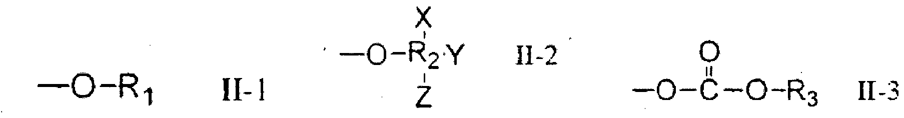

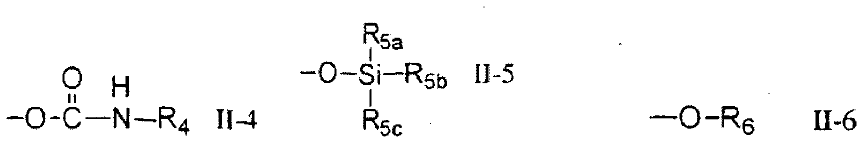

55. Procédé selon la revendication 54, dans lequel le premier polymère est un deuxième polyrotaxane, dans lequel le deuxième polyrotaxane comprend un deuxième pseudo-polyrotaxane, qui a une deuxième molécule linéaire et une ou plusieurs deuxièmes molécules cycliques, dans lequel la deuxième molécule linéaire est incluse dans une ou plusieurs cavités de la ou des deuxièmes molécules cycliques d'une manière embrochée, et des deuxièmes groupes de coiffage, chacun se situant à chaque extrémité du deuxième pseudo-polyrotaxane afin d'empêcher la dissociation de la ou des deuxièmes molécules cycliques,
dans lequel une partie des groupes hydroxy de la deuxième molécule cyclique est substituée par un groupe fonctionnel représenté par la formule I' suivante, et au moins un groupe fonctionnel choisi dans l'ensemble constitué par les formules II'-1 à II'-6 suivantes,
où chacun de R', R₁₁, R₁₃ et R₁₄ représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ;
chacun de R₁₅ₐ, R_{15b} et R_{15c} représente indépendamment un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone ou un groupe alkylthioéther cyclique ayant de 2 à 12 atomes de carbone ; et
R₁₂ représente un groupe de substitution obtenu par élimination de trois atomes d'hydrogène sur un groupe choisi dans l'ensemble constitué par un groupe alkyle linéaire ou ramifié ayant de 1 à 12 atomes de carbone, un groupe alkyle linéaire ou ramifié ayant de 2 à 12 atomes de carbone et contenant au moins un groupe éther, un groupe alkyle cyclique ayant de 3 à 12 atomes de carbone, un groupe alkyléther cyclique ayant de 2 à 12 atomes de carbone et un groupe alkylthioéther cyclique ayant de 2 à 12 atomes de carbone,
au moins l'un de X à Z est un groupe choisi dans l'ensemble constitué par un groupe hydroxyle, un groupe NH₃ et un groupe SH, les X à Z restants étant un atome d'hydrogène, et
R₁₆ représente un groupe photoréactif ; et
le deuxième polyrotaxane est obtenu par
1') préparation du deuxième pseudo-polyrotaxane ;
2') coiffage des deux extrémités du deuxième pseudo-polyrotaxane résultant avec un deuxième groupe de coiffage pour préparer un deuxième polyrotaxane ; et
3') substitution d'une partie des groupes hydroxy de la deuxième molécule cyclique par le groupe fonctionnel représenté par la formule I', et au moins un groupe fonctionnel choisi dans l'ensemble constitué par les formules II'-1 à II'-6 :

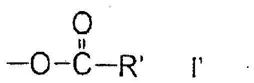

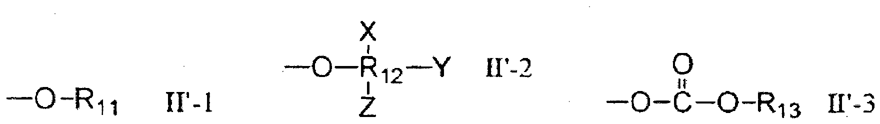

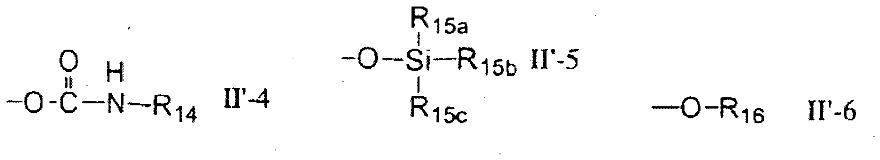

56. Procédé selon la revendication 54 ou 55, dans lequel l'étape de réticulation chimique 4) est effectuée par la réaction de réticulation G) par addition d'un agent de réticulation, et l'agent de réticulation est choisi dans l'ensemble constitué par le chlorure cyanurique, le chlorure de trimésoyle, le chlorure de téréphtaloyle, l'épichlorhydrine, le dibromobenzène, le glutaraldéhyde, un isocyanate polyfonctionnel aliphatique, un isocyanate polyfonctionnel aromatique, le diisocyanate de tolylène, le diisocyanate d'hexaméthylène, la divinylsulfone, le 1,1'-carbonyldiimidazole, les alcoxysilanes et leurs dérivés, et les amorceurs de réaction de photo-réticulation.

57. Procédé selon l'une quelconque des revendications 54 à 56, dans lequel l'étape de substitution par le groupe fonctionnel est effectuée après les étapes de coiffage 2) et/ou 2') pour préparer les premier et/ou deuxième polyrotaxanes.

58. Procédé selon l'une quelconque des revendications 54 à 57, dans lequel, dans l'étape de substitution par le groupe fonctionnel, une étape d'introduction du groupe fonctionnel représenté par la formule I ou I' est effectuée
X) avant une étape d'introduction de l'un quelconque des groupes fonctionnels représentés par la formule II ou II' ;
Y) après l'étape d'introduction de l'un quelconque des groupes fonctionnels représentés par la formule II ou II' ; ou
Z) en même temps que l'étape d'introduction de l'un quelconque des groupes fonctionnels représentés par la formule II ou II'.

59. Procédé selon l'une quelconque des revendications 55 à 58, dans lequel, dans l'étape de substitution par le groupe fonctionnel, une étape d'introduction du groupe fonctionnel représenté par la formule I ou I' est effectuée Y) après une étape d'introduction de l'un quelconque des groupes fonctionnels représentés par la formule II ou II'.
